(19) Europäisches Patentamt
European Patent Office
Office européen des brevets

(11) EP 1 514 942 A1

(12) **EUROPEAN PATENT APPLICATION**
published in accordance with Art. 158(3) EPC

(43) Date of publication:
**16.03.2005 Bulletin 2005/11**

(21) Application number: **03725810.0**

(22) Date of filing: **16.05.2003**

(51) Int Cl.⁷: **C12Q 1/04**, C07K 14/47, C12N 9/02, A61K 31/53, A61K 31/5377, A61K 31/55, A61K 45/00, A61P 3/10, A61P 5/50, A61P 43/00, C07D 251/70, C07D 403/14, C07D 405/14, C07D 487/14, G01N 33/15, G01N 33/50

(86) International application number:
**PCT/JP2003/006137**

(87) International publication number:
**WO 2003/097862 (27.11.2003 Gazette 2003/48)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **17.05.2002 JP 2002143599**

(71) Applicant: **KYOWA HAKKO KOGYO CO., LTD.**
**Chiyoda-ku, Tokyo 100-8185 (JP)**

(72) Inventors:
- **731IGURO, Hiroki, c/o Tokyo Research Lab. Machida-shi, Tokyo 194-8533 (JP)**
- **OSHIMA, Yuko, c/o Tokyo Research Lab. Machida-shi, Tokyo 194-8533 (JP)**
- **SUGIMOTO, Seiji, c/o Head Office, Kyowa Hakko Chiyoda-ku, Tokyo 100-8185 (JP)**
- **MATSUBARA, Masahiro, c/o Pharmaceutical Res. Inst. Nagaizumicho, Suntogun, Shizuoka 411-873 (JP)**
- **UENO, Kimihisa, c/o Pharmaceutical Res. Inst. Nagaizumicho, Suntogun, Shizuoka 411-873 (JP)**
- **MORI, Kiyotoshi, c/o Pharmaceutical Res. Inst. Nagaizumicho, Suntogun, Shizuoka 411-873 (JP)**
- **NAKANISHI, Satoshi, c/o Head Office, Kyowa Hakko Tokyo 100-8185 (JP)**
- **YANO, Hiroshi, c/o Pharmaceutical Res. Inst. Nagaizumicho, Suntogun, Shizuoka 411-873 (JP)**

(74) Representative: **VOSSIUS & PARTNER**
**Siebertstrasse 4**
**81675 München (DE)**

# (54) METHOD OF SEARCHING SUBSTANE HAVING ANTIDIABETIC ACTIVITY

(57) The present invention provides a screening method of a substance which inhibits binding of a condensed purine derivative to a pancreatic β cell or a treated product of the cell, a substance which inhibits binding of a condensed purine derivative to a protein capable of the condensed purine derivative, and a substance which inhibits the expression or enzymatic activity of a protein capable of a condensed purine derivative, which comprises using the condensed purine derivative and a pancreatic β cell or a treated product of the cell or a protein capable of binding to the condensed purine derivative.

FIG. 1

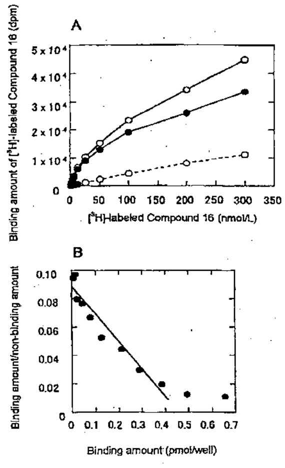


EP 1 514 942 A1

SEQUENCE LISTING

<110> KYOWA HAKKO KOGYO, CO., LTD.

<120> Method for screening of antidiabetic compounds

<130> 11486WO1

<140>
<141>

<150> JP 2002-143599
<151> 2002-5-17

<160> 9

<170> PatentIn Ver. 2.1

<210> 1
<211> 260
<212> PRT
<213> Homo sapiens

<400> 1
```
Met Ala Ser Ser Gly Met Thr Arg Arg Asp Pro Leu Ala Asn Lys Val
  1               5                  10                  15

Ala Leu Val Thr Ala Ser Thr Asp Gly Ile Gly Phe Ala Ile Ala Arg
             20                  25                  30

Arg Leu Ala Gln Asp Gly Ala His Val Val Val Ser Ser Arg Lys Gln
         35                  40                  45

Gln Asn Val Asp Gln Ala Val Ala Thr Leu Gln Gly Glu Gly Leu Ser
     50                  55                  60

Val Thr Gly Thr Val Cys His Val Gly Lys Ala Glu Asp Arg Glu Arg
```

65 70 75 80

Leu Val Ala Thr Ala Val Lys Leu His Gly Gly Ile Asp Ile Leu Val
85 90 95

Ser Asn Ala Ala Val Asn Pro Phe Phe Gly Ser Ile Met Asp Val Thr
100 105 110

Glu Glu Val Trp Asp Lys Thr Leu Asp Ile Asn Val Lys Ala Pro Ala
115 120 125

Leu Met Thr Lys Ala Val Val Pro Glu Met Glu Lys Arg Gly Gly Gly
130 135 140

Ser Val Val Ile Val Ser Ser Ile Ala Ala Phe Ser Pro Ser Pro Gly
145 150 155 160

Phe Ser Pro Tyr Asn Val Ser Lys Thr Ala Leu Leu Gly Leu Thr Lys
165 170 175

Thr Leu Ala Ile Glu Leu Ala Pro Arg Asn Ile Arg Val Asn Cys Leu
180 185 190

Ala Pro Gly Leu Ile Lys Thr Ser Phe Ser Arg Met Leu Trp Met Asp
195 200 205

Lys Glu Lys Glu Glu Ser Met Lys Glu Thr Leu Arg Ile Arg Arg Leu
210 215 220

Gly Glu Pro Glu Asp Cys Ala Gly Ile Val Ser Phe Leu Cys Ser Glu
225 230 235 240

Asp Ala Ser Tyr Ile Thr Gly Glu Thr Val Val Val Gly Gly Gly Thr
245 250 255

Pro Ser Arg Leu

260

<210> 2
<211> 260
<212> PRT
<213> Mus musculus

<400> 2
Met Ala Ser Ser Gly Leu Thr Arg Arg Asn Pro Leu Ser Asn Lys Val
1 5 10 15

Ala Leu Val Thr Ala Ser Thr Asp Gly Ile Gly Phe Ala Ile Ala Arg
20 25 30

Arg Leu Ala Glu Asp Gly Ala His Val Val Val Ser Ser Arg Lys Gln
35 40 45

Gln Asn Val Asp Arg Ala Val Ala Thr Leu Gln Gly Glu Gly Leu Ser
50 55 60

Val Thr Gly Ile Val Cys His Val Gly Lys Ala Glu Asp Arg Glu Lys
65 70 75 80

Leu Ile Thr Thr Ala Leu Lys Arg His Gln Gly Ile Asp Ile Leu Val
85 90 95

Ser Asn Ala Ala Val Asn Pro Phe Phe Gly Asn Leu Met Asp Val Thr
100 105 110

Glu Glu Val Trp Asp Lys Val Leu Ser Ile Asn Val Thr Ala Thr Ala
115 120 125

Met Met Ile Lys Ala Val Val Pro Glu Met Glu Lys Arg Gly Gly Gly
130 135 140

Ser Val Val Ile Val Gly Ser Val Ala Gly Phe Thr Arg Phe Pro Ser
145 150 155 160

Leu Gly Pro Tyr Asn Val Ser Lys Thr Ala Leu Leu Gly Leu Thr Lys
165 170 175

Asn Phe Ala Ala Glu Leu Ala Pro Lys Asn Ile Arg Val Asn Cys Leu
180 185 190

Ala Pro Gly Leu Ile Lys Thr Arg Phe Ser Ser Val Leu Trp Glu Glu
195 200 205

Lys Ala Arg Glu Asp Phe Ile Lys Glu Ala Met Gln Ile Arg Arg Leu
210 215 220

Gly Lys Pro Glu Asp Cys Ala Gly Ile Val Ser Phe Leu Cys Ser Glu
225 230 235 240

Asp Ala Ser Tyr Ile Asn Gly Glu Thr Val Val Val Gly Gly Gly Thr
245 250 255

Pro Ser Arg Leu
260

<210> 3
<211> 260
<212> PRT
<213> Oryctolagus cuniculus

<400> 3
Met Ala Ser Ser Gly Val Thr Arg Arg Asp Pro Leu Ala Asn Lys Val
1 5 10 15

Ala Ile Val Thr Ala Ser Thr Asp Gly Ile Gly Leu Ala Ile Ala Arg
20 25 30

Arg Leu Ala Gln Asp Gly Ala His Val Val Ile Ser Ser Arg Lys Gln
35 40 45

Gln Asn Val Asp Arg Ala Val Ala Ala Leu Gln Ala Glu Gly Leu Ser
50 55 60

Val Thr Gly Thr Val Cys His Val Gly Lys Ala Glu Asp Arg Glu Arg
65 70 75 80

Leu Val Ala Thr Ala Leu Asn Leu His Gly Gly Ile Asp Ile Leu Val
85 90 95

Ser Asn Ala Ala Val Asn Pro Phe Phe Gly Lys Leu Met Asp Val Thr
100 105 110

Glu Glu Val Trp Asp Lys Ile Leu Asp Ile Asn Val Lys Ala Met Ala
115 120 125

Leu Met Thr Lys Ala Val Val Pro Glu Met Glu Lys Arg Gly Gly Gly
130 135 140

Ser Val Val Ile Val Ala Ser Ile Ala Ala Phe Asn Pro Phe Ser Gly
145 150 155 160

Leu Gly Pro Tyr Asn Val Ser Lys Thr Ala Leu Val Gly Leu Thr Lys
165 170 175

Asn Leu Ala Leu Glu Leu Ala Ala Gln Asn Ile Arg Val Asn Cys Leu
180 185 190

Ala Pro Gly Leu Ile Lys Thr Ser Phe Ser Lys Ala Leu Trp Glu Asp
195 200 205

Lys Ala Gln Glu Glu Asn Ile Ile Gln Lys Leu Arg Ile Arg Arg Leu
210 215 220

Gly Lys Pro Glu Glu Cys Ala Gly Ile Val Ser Phe Leu Cys Ser Glu
225 230 235 240

Asp Ala Ser Tyr Ile Thr Gly Glu Thr Val Val Val Ala Gly Gly Ala
245 250 255

Pro Ser Arg Leu
260

<210> 4
<211> 780
<212> DNA
<213> Homo sapiens

<400> 4
atg gcc agc tcc ggg atg acc cgc cgg gac ccg ctc gca aat aag gtg 48
Met Ala Ser Ser Gly Met Thr Arg Arg Asp Pro Leu Ala Asn Lys Val
1 5 10 15

gcc ctg gta acg gcc tcc acc gac ggg atc ggc ttc gcc atc gcc cgg 96
Ala Leu Val Thr Ala Ser Thr Asp Gly Ile Gly Phe Ala Ile Ala Arg
20 25 30

cgt ttg gcc cag gac ggg gcc cat gtg gtc gtc agc agc cgg aag cag 144
Arg Leu Ala Gln Asp Gly Ala His Val Val Val Ser Ser Arg Lys Gln
35 40 45

cag aat gtg gac cag gcg gtg gcc acg ctg cag ggg gag ggg ctg agc 192
Gln Asn Val Asp Gln Ala Val Ala Thr Leu Gln Gly Glu Gly Leu Ser
50 55 60

gtg acg ggc acc gtg tgc cat gtg ggg aag gcg gag gac cgg gag cgg 240
Val Thr Gly Thr Val Cys His Val Gly Lys Ala Glu Asp Arg Glu Arg
65 70 75 80

ctg gtg gcc acg gct gtg aag ctt cat gga ggt atc gat atc cta gtc 288
Leu Val Ala Thr Ala Val Lys Leu His Gly Gly Ile Asp Ile Leu Val
85 90 95

tcc aat gct gct gtc aac cct ttc ttt gga agc ata atg gat gtc act 336
Ser Asn Ala Ala Val Asn Pro Phe Phe Gly Ser Ile Met Asp Val Thr
100 105 110

gag gag gtg tgg gac aag act ctg gac att aat gtg aag gcc cca gcc 384
Glu Glu Val Trp Asp Lys Thr Leu Asp Ile Asn Val Lys Ala Pro Ala
115 120 125

ctg atg aca aag gca gtg gtg cca gaa atg gag aaa cga gga ggc ggc 432
Leu Met Thr Lys Ala Val Val Pro Glu Met Glu Lys Arg Gly Gly Gly
130 135 140

tca gtg gtg atc gtg tct tcc ata gca gcc ttc agt cca tct cct ggc 480
Ser Val Val Ile Val Ser Ser Ile Ala Ala Phe Ser Pro Ser Pro Gly
145 150 155 160

ttc agt cct tac aat gtc agt aaa aca gcc ttg ctg ggc ctg acc aag 528
Phe Ser Pro Tyr Asn Val Ser Lys Thr Ala Leu Leu Gly Leu Thr Lys
165 170 175

acc ctg gcc ata gag ctg gcc cca agg aac att agg gtg aac tgc cta 576
Thr Leu Ala Ile Glu Leu Ala Pro Arg Asn Ile Arg Val Asn Cys Leu
180 185 190

gca cct gga ctt atc aag act agc ttc agc agg atg ctc tgg atg gac 624
Ala Pro Gly Leu Ile Lys Thr Ser Phe Ser Arg Met Leu Trp Met Asp
195 200 205

aag gaa aaa gag gaa agc atg aaa gaa acc ctg cgg ata aga agg tta 672
Lys Glu Lys Glu Glu Ser Met Lys Glu Thr Leu Arg Ile Arg Arg Leu
210 215 220

ggc gag cca gag gat tgt gct ggc atc gtg tct ttc ctg tgc tct gaa 720
Gly Glu Pro Glu Asp Cys Ala Gly Ile Val Ser Phe Leu Cys Ser Glu
225 230 235 240

gat gcc agc tac atc act ggg gaa aca gtg gtg gtg ggt gga gga acc 768
Asp Ala Ser Tyr Ile Thr Gly Glu Thr Val Val Val Gly Gly Gly Thr
245 250 255

ccg tcc cgc ctc
Pro Ser Arg Leu
260

<210> 5
<211> 780
<212> DNA
<213> Mus musculus

<400> 5
atg gcc agt tcc ggg ttg act cgt cga aac cct ctc tcc aat aag gtg 48
Met Ala Ser Ser Gly Leu Thr Arg Arg Asn Pro Leu Ser Asn Lys Val
1 5 10 15

gcc ctg gtc aca gcc tcc acc gac ggg atc ggc ttt gcc atc gcc cgg 96
Ala Leu Val Thr Ala Ser Thr Asp Gly Ile Gly Phe Ala Ile Ala Arg
20 25 30

cgc ctg gct gaa gat ggg gcc cac gtg gtc gtc agc agc cgc aaa cag 144
Arg Leu Ala Glu Asp Gly Ala His Val Val Val Ser Ser Arg Lys Gln
35 40 45

cag aat gtg gac cgt gca gtg gcc aca cta cag gga gag ggc ctg agt 192
Gln Asn Val Asp Arg Ala Val Ala Thr Leu Gln Gly Glu Gly Leu Ser
50 55 60

```
gtg act ggc atc gtg tgc cac gtg ggg aag gca gag gat cga gaa aag    240
Val Thr Gly Ile Val Cys His Val Gly Lys Ala Glu Asp Arg Glu Lys
 65                  70                  75                  80

ctg ata acc acg gct ctg aag cgt cac cag ggg att gat atc ctg gtc    288
Leu Ile Thr Thr Ala Leu Lys Arg His Gln Gly Ile Asp Ile Leu Val
                 85                  90                  95

tcc aat gct gct gtc aac cct ttc ttt gga aat cta atg gat gtc aca    336
Ser Asn Ala Ala Val Asn Pro Phe Phe Gly Asn Leu Met Asp Val Thr
            100                 105                 110

gag gag gtg tgg gac aag gtt ttg agc att aat gtg aca gct aca gcc    384
Glu Glu Val Trp Asp Lys Val Leu Ser Ile Asn Val Thr Ala Thr Ala
        115                 120                 125

atg atg att aaa gcg gtg gtg cca gag atg gaa aag cga gga ggc ggc    432
Met Met Ile Lys Ala Val Val Pro Glu Met Glu Lys Arg Gly Gly Gly
    130                 135                 140

tca gtg gtg att gtg ggt tct gta gca ggc ttc act cgg ttc cct tct    480
Ser Val Val Ile Val Gly Ser Val Ala Gly Phe Thr Arg Phe Pro Ser
145                 150                 155                 160

ctg ggt cct tac aat gtt agc aaa aca gct ttg ctg ggt ctt act aag    528
Leu Gly Pro Tyr Asn Val Ser Lys Thr Ala Leu Leu Gly Leu Thr Lys
                165                 170                 175

aac ttt gca gcg gag ttg gcc ccg aag aac att cga gtg aac tgc tta    576
Asn Phe Ala Ala Glu Leu Ala Pro Lys Asn Ile Arg Val Asn Cys Leu
            180                 185                 190

gca cct gga ctc atc aag act cga ttc agc agt gtg ttg tgg gag gag    624
Ala Pro Gly Leu Ile Lys Thr Arg Phe Ser Ser Val Leu Trp Glu Glu
        195                 200                 205
```

aaa gca aga gag gac ttc ata aaa gaa gcc atg caa atc aga agg cta 672
Lys Ala Arg Glu Asp Phe Ile Lys Glu Ala Met Gln Ile Arg Arg Leu
210 215 220

ggc aag cca gag gat tgt gct ggc ata gtt tcc ttc tta tgc tct gaa 720
Gly Lys Pro Glu Asp Cys Ala Gly Ile Val Ser Phe Leu Cys Ser Glu
225 230 235 240

gac gcc agt tac atc aat gga gag acc gta gta gtg ggg gga gga acc 768
Asp Ala Ser Tyr Ile Asn Gly Glu Thr Val Val Val Gly Gly Gly Thr
245 250 255

cct tct cgc ctc 783
Pro Ser Arg Leu
260

<210> 6
<211> 780
<212> DNA
<213> Oryctolagus cuniculus

<400> 6
atg gcc agc tct ggg gtg acc cgc cgg gac ccg ctc gcg aat aag gtg 48
Met Ala Ser Ser Gly Val Thr Arg Arg Asp Pro Leu Ala Asn Lys Val
1 5 10 15

gcc atc gta acg gcc tcc acc gac ggg atc ggc ttg gcc atc gcc cgg 96
Ala Ile Val Thr Ala Ser Thr Asp Gly Ile Gly Leu Ala Ile Ala Arg
20 25 30

cgt ctg gcc cag gat ggg gcc cac gtg gtc atc agc agc cgg aag cag 144
Arg Leu Ala Gln Asp Gly Ala His Val Val Ile Ser Ser Arg Lys Gln
35 40 45

cag aac gtg gac cgg gcg gtg gct gca ctg cag gcc gag ggg ctg agt 192

Gln Asn Val Asp Arg Ala Val Ala Ala Leu Gln Ala Glu Gly Leu Ser
50 55 60

gtg acg ggc acc gtg tgc cac gtg ggg aag gcc gag gac cgg gaa cgg 240
Val Thr Gly Thr Val Cys His Val Gly Lys Ala Glu Asp Arg Glu Arg
65 70 75 80

ctg gtg gcc acg gcc ctg aac ctc cac gga ggc att gat atc ctg gtc 288
Leu Val Ala Thr Ala Leu Asn Leu His Gly Gly Ile Asp Ile Leu Val
85 90 95

tcc aat gct gca gtc aac ccc ttc ttt gga aag cta atg gat gtc acc 336
Ser Asn Ala Ala Val Asn Pro Phe Phe Gly Lys Leu Met Asp Val Thr
100 105 110

gag gag gtg tgg gac aag att ctg gac att aac gtg aag gcc atg gcc 384
Glu Glu Val Trp Asp Lys Ile Leu Asp Ile Asn Val Lys Ala Met Ala
115 120 125

ctg atg aca aag gcg gtg gtg cca gag atg gag aaa cga gga ggc ggc 432
Leu Met Thr Lys Ala Val Val Pro Glu Met Glu Lys Arg Gly Gly Gly
130 135 140

tcc gtg gtg atc gtg gcc tcc ata gca gcc ttc aat cca ttc agt ggc 480
Ser Val Val Ile Val Ala Ser Ile Ala Ala Phe Asn Pro Phe Ser Gly
145 150 155 160

ctg ggc cct tat aat gtt agt aaa aca gcc ttg gtg ggc ctt acc aag 528
Leu Gly Pro Tyr Asn Val Ser Lys Thr Ala Leu Val Gly Leu Thr Lys
165 170 175

aac ctg gcc ctg gag ctg gct gcc cag aac atc cgg gtg aac tgc ctg 576
Asn Leu Ala Leu Glu Leu Ala Ala Gln Asn Ile Arg Val Asn Cys Leu
180 185 190

gca ccg gga ctc atc aag acc agc ttc agt aag gcg ttg tgg gag gac 624

Ala Pro Gly Leu Ile Lys Thr Ser Phe Ser Lys Ala Leu Trp Glu Asp
195 200 205

aag gca caa gag gaa aac atc ata caa aag ttg agg ata aga agg tta 672
Lys Ala Gln Glu Glu Asn Ile Ile Gln Lys Leu Arg Ile Arg Arg Leu
210 215 220

ggc aaa cca gag gaa tgt gcg ggc atc gtg tct ttc ctg tgc tcc gaa 720
Gly Lys Pro Glu Glu Cys Ala Gly Ile Val Ser Phe Leu Cys Ser Glu
225 230 235 240

gac gcc agc tac atc act ggg gag aca gtg gtg gtg gct gga gga gca 768
Asp Ala Ser Tyr Ile Thr Gly Glu Thr Val Val Val Ala Gly Gly Ala
245 250 255

cca tcc cgc ctc
Pro Ser Arg Leu
260

<210> 7
<211> 32
<212> DNA
<213> Artificial Sequence

<400> 7
ggaattccat atggccagtt ccgggttgac tc 32

<210> 8
<211> 36
<212> DNA
<213> Artificial Sequence

<400> 8
cgggatccgt cgactcagag gcgagaaggg gttcct 36

<210> 9
<211> 20
<212> PRT
<213> Mus musculus

<400> 9
Gly Ser His Met Ala Ser Ser Gly Leu Thr Arg Arg Asn Pro Leu Ser
1 5 10 15

Asn Lys Val Ala
20

## Description

TECHNICAL FIELD

The present invention relates to a screening method of a therapeutic agent of diabetes mellitus and an insulin secretion-promoting agent.

BACKGROUND ART

Diabetes mellitus is an abnormality of metabolism mainly including glucose metabolism based on the insufficient secretion of insulin or the reduction of the sensitivity of the target cell thereof and has a big characteristic feature of inducing hyperglycemia. When hyperglycemia is sustained for a long time, serious complications in various organs and nerve such as retinitis, nephropathy and nerve disorders occur due to the main cause of vascular disorders. For the therapeutic treatment of diabetes mellitus, thus, it is very important to control and maintain the blood glucose value at the normal value. Research works have traditionally been done about the measures therefor.

Among diabetes mellitus types, the disease type (type II diabetes mellitus) not necessarily requiring insulin therapy for vital control because the onset is very slow is therapeutically treated by controlling blood glucose value via combinations of therapeutic exercise, dietary therapy and medicaments. As such drugs, one of oral hypoglycemic agents, namely sulfonylurea promoting insulin secretion is widely used in clinical practice. In recent years, additionally, agents for promoting insulin secretion, namely non-sulfonylurea $K^+$ ATP channel blockers such as nateglinide and ripaglinide have been commercially available.

Sulfonylurea is a therapeutic agent of type II diabetes mellitus and works to lower blood glucose value by promoting insulin secretion from pancreatic β cell. Many sulfonylurea types differing in the intensity of hypoglycemic activity or the time period of the sustainment of the activity have been known [*Joslin's Diabetes Mellitus,* 13$^{th}$ Edition, 29, 505-525 (1994)].

However, side effects and complications common to sulfonylurea types or some diabetes mellitus types for which sulfonylurea is ineffective have been recognized [*Joslin's Diabetes Mellitus*, 13$^{th}$ Edition, 29, 505-525 (1994)].

As a major complication in the use of sulfonylurea, hypoglycemia is listed [*Bio. Med. J,* 296, 949-950 (1988); *Joslin's Diabetes Mellitus,* 13$^{th}$ Edition, 29, 505-525 (1994)].

Because currently applicable sulfonylurea and non-sulfonylurea $K^+$ ATP channel blockers such as nateglinide and ripaglinide all promote insulin secretion independent on blood glucose value, serious hypoglycemia occurs when they are administered at erroneous doses. Besides, these disadvantageously cannot control blood glucose value sufficiently. Therefore, these are not essentially satisfactory. A report tells that symptoms of hypoglycemia are observed in 20.2% of patients administered with sulfonylurea within the last 6 months [*Acta. Med. Scand. Suppl.,* 605, 7-48 (1977); *Joslin's Diabetes Mellitus,* 13$^{th}$ Edition, 29, 505-525 (1994)]. About 6% of the patients cause a hypoglycemic episode every month.

Based on such reasons, the development of a therapeutic agent of diabetes mellitus without any occurrence of complications such as hypoglycemia, unlike sulfonylurea and non-sulfonylurea $K^+$ ATP channel blockers, has been desired.

Some report tells that condensed purine derivatives or condensed cyclic pyrazine derivatives or the like having structures different from those of the non-sulfonylurea $K^+$ ATP channel blockers may be a potential hypoglycemic agent capable of promoting insulin secretion, depending on the blood glucose value (WO00/01388, WO01/47931, Japanese Published Unexamined Patent Application No. 154139/00). However, not any method for highly efficiently screening for a hypoglycemic agent capable of promoting insulin secretion depending on the blood glucose value has been found yet.

It has never been known about condensed purine derivatives specifically binding to pancreatic β cell or products from the treatment of the cell or about the relation between the binding activity and the hypoglycemic activity of condensed purine derivatives.

Proteins having amino acid sequences represented by SEQ ID NOs:1 to 3 (hereinafter referred to as "p31") are all known as enzymes having NADPH-dependent redox enzyme activities of retinol and short-chain alcohols [*Biochimica et Biophysica Acta,* 1338, 47 (1997)]. However, no relation between the enzymes and diabetes mellitus has been reported yet.

It has been known that triazine derivatives have kinase-inhibiting activity and can be used for therapeutic treatment of diabetes mellitus (WO01/47921). However, no specific description about the therapeutic activity of diabetes mellitus has been elucidated yet. Additionally, it has never been known that triazine derivatives have insulin secretion activity.

DISCLOSURE OF THE INVENTION

An object of the present invention is to provide a screening method of a therapeutic agent of diabetes mellitus and an insulin secretion-promoting agent without any occurrence of complications such as hypoglycemia.
The present inventors made investigations, and as a result, they found the correlation between the insulin secretion-promoting activity of condensed purine derivatives and the binding activity of the condensed purine derivatives to proteins capable of binding to the condensed purine derivatives. Thus, the present invention has been achieved.
In other words, the present invention relates to the following aspects (1) to (24).

(1) A screening method of a substance having anti-diabetic activity, which comprises:

[1] bringing a compound selected from compounds represented by the following formula (I):

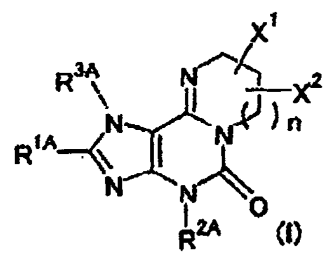

(I)

(wherein $R^{1A}$ represents hydrogen, lower alkyl, substituted or unsubstituted aryl, or a substituted or unsubstituted aromatic heterocyclic group; $R^{2A}$ represents hydrogen, lower alkyl, substituted or unsubstituted aralkyl, substituted or unsubstituted aryl or a substituted or unsubstituted aromatic heterocyclic group; $R^{3A}$ represents hydrogen, lower alkyl, or substituted or unsubstituted aralkyl; $X^1$ and $X^2$ each independently represents hydrogen, lower alkyl substituted or unsubstituted aralkyl, or substituted or unsubstituted. aryl; and n is an integer of 0 to 3) or compounds represented by the following formula (II):

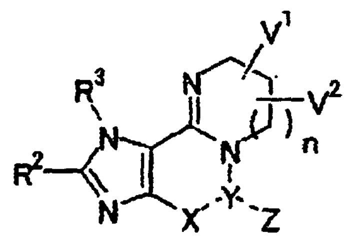

(II)

{wherein X--Y--Z represents $R^1$N C=O (wherein $R^1$ represents hydrogen, substituted or unsubstituted lower alkyl, substituted or unsubstituted aralkyl, substituted or unsubstituted aryl or a substituted or unsubstituted aromatic heterocyclic group) or N=C-W [wherein W represents halogen, a substituted or unsubstituted aromatic heterocyclic group, a substituted or unsubstituted alicyclic heterocyclic group, $-NR^4R^5$ (wherein $R^4$ and $R^5$ are the same or different and each represents hydrogen, substituted or unsubstituted lower alkyl, substituted or unsubstituted aryl, or substituted or unsubstituted aralkyl, or $R^4$ and $R^5$ together with adjacent nitrogen represent a heterocyclic group), $-OR^6$ (wherein $R^6$ represents substituted or unsubstituted lower alkyl, substituted or unsubstituted aryl, or substituted or unsubstituted aralkyl), $-SR^{6a}$ (wherein $R^{6a}$ has the same meaning as $R^6$), substituted or unsubstituted lower alkyl. or cyano]; $R^2$ represents hydrogen, substituted or unsubstituted lower alkyl, substituted or unsubstituted aralkyl substituted or unsubstituted aryl, a substituted or unsubstituted aromatic heterocyclic group, a substituted or unsubstituted alicyclic heterocyclic group, halogen, lower alkylthio, $-NR^7R^8$ (wherein $R^7$ and $R^8$ have the same meanings as $R^4$ and $R^5$ as described above, respectively), $-CO_2H$, lower alkoxycarbonyl, -COHal (wherein Hal represents halogen), $-CONR^9R^{10}$ (wherein $R^9$ and $R^{10}$ have the same meanings as $R^4$ and $R^5$ as described above, respectively), or -CHO; $R^3$ represents hydrogen, lower alkyl, substituted or unsubstituted aralkyl, or lower alkoxyalkyl; n represents an integer of 0 to 3; $V^1$ represents hydrogen, substituted or unsubstituted lower alkyl, substituted or unsubstituted aralkyl, substituted

or unsubstituted aryl, or a substituted or unsubstituted aromatic heterocyclic group; and $V^2$ represents substituted lower alkyl or a substituted or unsubstituted aromatic heterocyclic group, and when $V^1$ represents hydrogen, lower alkyl, substituted or unsubstituted aralkyl or substituted or unsubstituted aryl and (a) X--Y--Z represents $R^{1a}$N-C=O (wherein $R^{1a}$ represents the same groups defined for $R^1$ except the substituted lower alkyl), and $R^2$ represents substituted lower alkyl, substituted or unsubstituted aralkyl, a substituted or unsubstituted alicyclic heterocyclic group, halogen, lower alkylthio, -$NR^7R^8$ (wherein $R^7$ and $R^8$ have the same meanings as described above, respectively), -$CO_2$H, lower alkoxycarbonyl, -COHal (wherein Hal has the same meaning as described above), -$CONR^9R^{10}$ (wherein $R^9$ and $R^{10}$ independently have the same meanings as described above), or -CHO, (b) X--Y--Z represents $R^1$N-C=O (wherein $R^1$ has the same meaning as described above) and $R^3$ represents lower alkoxyalkyl, (c) X--Y--Z represents $R^{1b}$N-C=O (wherein $R^{1b}$ represents substituted lower alkyl), (d) X--Y--Z represents N=C-W (wherein W has the same meaning as described above), and $R^2$ represents substituted or unsubstituted lower alkyl, substituted or unsubstituted aralkyl, substituted or unsubstituted aryl a substituted or unsubstituted aromatic heterocyclic group, a substituted or unsubstituted alicyclic heterocyclic group, halogen, lower alkylthio, -$NR^7R^8$ (wherein $R^7$ and $R^8$ have the same meanings as described above, respectively), -$CO_2$H, lower alkoxycarbonyl, -COHal (wherein Hal has the same meaning as described above), -$CONR^9R^{10}$ (wherein $R^9$ and $R^{10}$ have the same meanings as described above, respectively), or -CHO, or (e) X--Y--Z represents N=C-W (wherein W has the same meaning as described above), and $R^3$ represents lower alkyl, substituted or unsubstituted aralkyl or lower alkoxyalkyl, $V^2$ may represent lower alkyl, substituted or unsubstituted aralkyl, or substituted or unsubstituted aryl] (hereinafter referred to as "condensed purine derivative") into contact with a pancreatic β cell or a treated product of the cell in the presence of a tested substance to assay the binding amount of the condensed purine derivative to the pancreatic β cell or the treated product of the cell,
[2] bringing the condensed purine derivative into contact with a pancreatic β cell or a treated product of the cell in the absence of the tested substance to assay the binding amount of the condensed purine derivative to the pancreatic β cell or the treated product of the cell,
[3] comparing the assayed amount in the presence of the tested substance with that in the absence of the tested substance, and
[4] selecting a substance which inhibits the binding of the condensed purine derivative to the pancreatic β cell or the treated product of the cell from the tested substances.

(2) A screening method of a substance having anti-diabetic activity, which comprises:

[1] bringing the condensed purine derivative described in the above (1) into contact with a protein capable of binding to the condensed purine derivative in the presence of a tested substance to assay the binding amount of the condensed purine derivative to the protein,
[2] bringing the condensed purine derivative into contact with the protein in the absence of the tested substance to assay the binding amount of the condensed purine derivative to the protein,
[3] comparing the assayed amount in the presence of the tested substance with that in the absence of the tested substance, and
[4] selecting a substance which inhibits the binding of the condensed purine derivative to the protein from the tested substances.

(3) A screening method of a substance which is capable of binding to a protein capable of binding to the condensed purine derivative described in the above (1), which comprises:

[1] bringing the condensed purine derivative described in the above (1) into contact with a pancreatic β cell or a treated product of the cell in the presence of a tested substance to assay the binding amount of the condensed purine derivative to the pancreatic β cell or the treated product of the cell,
[2] bringing the condensed purine derivative into contact with a pancreatic β cell or a treated product of the cell in the absence of the tested substance to assay the binding amount of the condensed purine derivative to pancreatic β cell or the treated product of the cell,
[3] comparing the assayed amount in the presence of the tested substance with that in the absence of the tested substance,
[4] selecting a substance which inhibits the binding of the condensed purine derivative to the pancreatic β cell or the treated product of the cell from the tested substances, and
[5] selecting a substance binding to a protein capable, of binding to the condensed purine derivative from the substances obtained in the step [4].

(4) A screening method of a substance which is capable of binding to a protein capable of binding to the condensed purine derivative described in the above (1), which comprises:

[1] bringing the condensed purine derivative described in the above (1) into contact with a protein capable of binding to the condensed purine derivative in the presence of a tested substance to assay the binding amount of the condensed purine derivative to the protein,
[2] bringing the condensed purine derivative into contact with the protein in the absence of the tested substance to assay the binding amount of the condensed purine derivative to the protein,
[3] comparing the assayed amount in the presence of the tested substance with that in the absence of the tested substance, and
[4] selecting a substance which inhibits the binding of the condensed purine derivative to the protein from the tested substances.

(5) A screening method of a substance which is capable of changing the activity of a protein capable of binding to the condensed purine derivative described in the above (1), which comprises:

[1] bringing a tested substance into contact with a protein capable of binding to the condensed purine derivative described in the above (1) to assay the activity of the protein,
[2] assaying the activity of the protein which is out of contact with the tested substance,
[3] comparing the assayed amount in the presence of the tested substance with that in the absence of the tested substance, and
[4] selecting a substance which is capable of changing the activity of the protein from the tested substances.

(6) A screening method of a substance which is capable of suppressing the activity of a protein capable of binding to the condensed purine derivative described in the above (1), which comprises:

[1] bringing a tested substance into contact with a protein capable of binding to the condensed purine derivative described in the above (1) to assay the activity of the protein,
[2] assaying the activity of the protein which is out of contact with the tested substance,
[3] comparing the assayed amount in the presence of the tested substance with that in the absence of the tested substance, and
[4] selecting a substance which is capable of suppressing the activity of the protein from the tested substances.

(7) A screening method of a substance which is capable of changing the function of a protein capable of binding to the condensed purine derivative described in the above (1), which comprises:

[1] bringing a tested substance into contact with a transformant which expresses a protein capable of binding to the condensed purine derivative described in the above (1) to assay the cell response of the transformant,
[2] assaying the cell response of the transformant in the absence of the tested substance,
[3] comparing the assayed amount in the presence of the tested substance with that in the absence of the tested substance, and
[4] selecting a substance which is capable of changing the cell response of the transformant from the tested substances.

(8) A screening method of a substance which is capable of changing the expression level of a gene encoding a protein capable of binding to the condensed purine derivative described in the above (1), which comprises:

[1] bringing a tested substance into contact with a transformant which expresses a protein capable of binding to the condensed purine derivative described in the above (1) to assay the expression level of the gene encoding the protein in the transformant,
[2] assaying the expression level of the gene encoding the protein in the transformant in the absence of the tested substance,
[3] comparing the assayed amount in the presence of the tested substance with that in the absence of the tested substance, and
[4] selecting a substance which is capable of changing the expression level of the gene encoding the protein from the tested substances.

(9) A screening method of a substance which is capable of changing the expression level of a protein capable of

binding to the condensed purine derivative described in the above (1), which comprises:

[1] bringing a tested substance into contact with a transformant which expresses a protein capable of binding to the condensed purine derivative described in the above (1) to assay the expression level of the protein in the transformant,

[2] assaying the expression level of the protein in the transformant in the absence of the tested substance,
[3] comparing the assayed amount in the presence of the tested substance with that in the absence of the tested substance, and
[4] selecting a substance which is capable of changing the expression level of the protein from the tested substances.

(10) The screening method according to any one of the above (1) to (9), wherein the condensed purine derivative is a compound represented by the following formula (III):

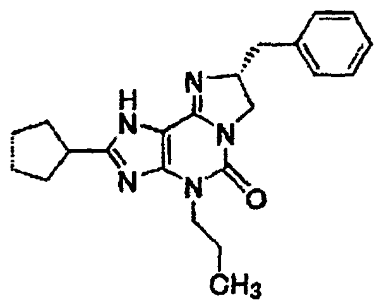

(III)

(11) The screening method according to any one of the above (2) and (4) to (9), wherein the purine derivative described in the above (1) is immobilized on a carrier.
(12) The screening method according to the above (11), wherein the carrier is a Sepharose particle.
(13) The screening method according to the above (11) or (12), wherein the condensed purine derivative is a compound represented by formula (IV):

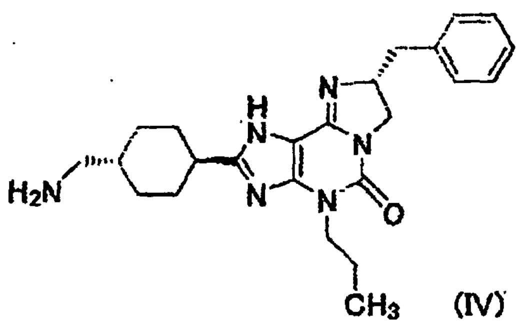

(IV)

(14) A carrier which is immobilized with the condensed purine derivative described in the above (1).
(15) The carrier according to the above (14), wherein the condensed purine derivative is the condensed purine derivative represented by formula (IV) described in the above (13).
(16) The carrier according to the above (14) or (15), wherein the carrier is a Sepharose particle.
(17) The screening method according to any one of the above (2) and (4) to (13), wherein the protein capable of binding to the condensed purine derivative described in the above (1) is a protein selected from the allowing [i] to [iii]:

[i] a protein which comprises the amino acid sequence represented by any one of SEQ ID NOs:1 to 3;
[ii] a protein which consists of an amino acid sequence in which at least one amino acid is deleted, substituted, inserted or added in the amino acid sequence represented by any one of SEQ ID NOs:1to 3, and is capable of binding to the condensed purine derivative described in the above (1); and
[iii] a protein which consists of an amino acid sequence having a homology of 60% or more with the amino

acid sequence represented by any one of SEQ ID NOs:1 to 3, and is capable of binding to the condensed purine derivative described in the above (1).

(18) A substance obtainable by the screening method according to any one of the above (1) to (13) and (17) or a pharmaceutically acceptable salt of the substance.
(19) A medicament which comprises the substance according to the above (18) or a pharmaceutically acceptable salt of the substance as an active ingredient.
(20) A therapeutic agent of diabetes mellitus, which comprises the substance according to the above (18) or a pharmaceutically acceptable salt of the substance as an active ingredient.
(21) An insulin secretion-promoting agent, which comprises the substance according to the above (18) or a pharmaceutically acceptable salt of the substance as an active ingredient.
(22) An insulin secretion-promoting agent, which comprises a compound represented by the following formula (V):

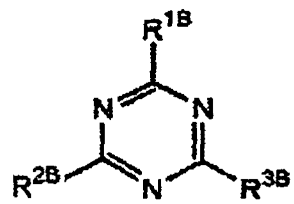
(V)

[wherein $R^{1B}$, $R^{2B}$ and $R^{3B}$ are the same or different and each represents amino, mono- or di-substituted amino, or a substituted or unsubstituted N-containing heterocyclic group (wherein the heterocyclic group binds to the triazine ring at the N atom)] or a pharmaceutically acceptable salt thereof as an active ingredient.
(23) A method for promoting insulin secretion, which comprises administering an effective amount of the compound represented by formula (V) described in the above (22) or a pharmaceutically acceptable salt thereof.
(24) Use of the compound represented by formula (V) described in the above (22) or a pharmaceutically acceptable salt thereof for the manufacture of an insulin secretion-promoting agent.

The present invention is described below in detail.

1. Condensed purine derivative used in the screening method of the present invention

The condensed purine derivative used in the screening method of the present invention is a compound selected from compounds represented by the following formula (I):

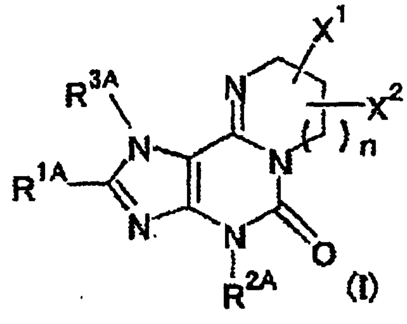
(I)

(wherein $R^{1A}$ represents hydrogen, lower alkyl, substituted or unsubstituted aryl, or a substituted or unsubstituted aromatic heterocyclic group; $R^{2A}$ represents hydrogen, lower alkyl, substituted or unsubstituted aralkyl, substituted or unsubstituted aryl, or a substituted or unsubstituted aromatic heterocyclic group; $R^{3A}$ represents hydrogen, lower alkyl, or substituted or unsubstituted aralkyl; $X^1$ and $X^2$ each independently represents hydrogen, lower alkyl, substituted or unsubstituted aralkyl, or substituted or unsubstituted aryl; and n is an integer of 0 to 3) or compounds represented by the following formula (II):

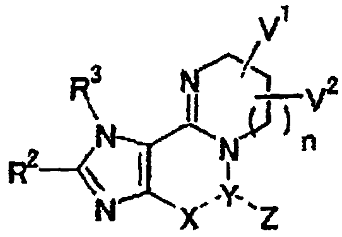

(II)

{wherein X--Y--Z represents $R^1$N-C=O ($R^1$ represents hydrogen, substituted or unsubstituted lower alkyl, substituted or unsubstituted aralkyl, substituted or unsubstituted aryl or a substituted or unsubstituted aromatic heterocyclic group) or N=C-W [wherein W represents halogen, a substituted or unsubstituted aromatic heterocyclic group, a substituted or unsubstituted alicyclic heterocyclic group, -$NR^4R^5$ (wherein $R^4$ and $R^5$ are the same or different and each represents hydrogen, substituted or unsubstituted lower alkyl, substituted or unsubstituted aryl, or substituted or unsubstituted aralkyl, or $R^4$ and $R^5$ together with adjacent nitrogen represent a heterocyclic group), -$OR^6$ (wherein $R^6$ represents substituted or unsubstituted lower alkyl, substituted or unsubstituted aryl, or substituted or unsubstituted aralkyl), -$SR^{6a}$ (wherein $R^{6a}$ has the same meaning as $R^6$), substituted or unsubstituted lower alkyl or cyano]; $R^2$ represents hydrogen, substituted or unsubstituted lower alkyl, substituted or unsubstituted aralkyl substituted or unsubstituted aryl, a substituted or unsubstituted aromatic heterocyclic group, a substituted or unsubstituted alicyclic heterocyclic group, halogen, lower alkylthio, -$NR^7R^8$(wherein $R^7$ and $R^8$ have the same meanings as $R^4$ and $R^5$ as described above, respectively), -$CO_2H$, lower alkoxycarbonyl, -COHal (wherein Hal represents halogen), -$CONR^9R^{10}$ (wherein $R^9$ and $R^{10}$ have the same meanings as $R^4$ and $R^5$ as described above, respectively), or -CHO; $R^3$ represents hydrogen, lower alkyl, substituted or unsubstituted aralkyl, or lower alkoxyalkyl; n represents an integer of 0 to 3; $V^1$ represents hydrogen, substituted or unsubstituted lower alkyl, substituted or unsubstituted aralkyl, substituted or unsubstituted aryl, or a substituted or unsubstituted aromatic heterocyclic group; and $V^2$ represents substituted lower alkyl or a substituted or unsubstituted aromatic heterocyclic group, and when $V^1$ represents hydrogen, lower alkyl, substituted or unsubstituted aralkyl or substituted or unsubstituted aryl and (a) X--Y--Z represents $R^{1a}$N-C=O (wherein $R^{1a}$ represents the same groups defined for $R^1$ except the substituted lower alkyl), and $R^2$ represents substituted lower alkyl, substituted or unsubstituted aralkyl, a substituted or unsubstituted alicyclic heterocyclic group, halogen, lower alkylthio, -$NR^7R^8$ (wherein $R^7$ and $R^8$ have the same meanings as described above, respectively), -$CO_2H$, lower alkoxycarbonyl, -COHal (wherein Hal has the same meaning as described above), -$CONR^9R^{10}$ (wherein $R^9$ and $R^{10}$ have the same meanings as described above, respectively), or -CHO, (b) X--Y--Z represents $R^1$N-C=O (wherein $R^1$ has the same meaning as described above) and $R^3$ represents lower alkoxyalkyl, (c) X--Y--Z represents $R^{1b}$N-C=O (wherein $R^{1b}$ represents substituted lower alkyl), (d) X--Y--Z represents N=C-W (wherein W has the same meaning as described above), and $R^2$ represents substituted or unsubstituted lower alkyl, substituted or unsubstituted aralkyl, substituted or unsubstituted aryl, a substituted or unsubstituted aromatic heterocyclic group, a substituted or unsubstituted alicyclic heterocyclic group, halogen, lower alkylthio, -$NR^7R^8$ (wherein $R^7$ and $R^8$ have the same meanings as described above, respectively), -$CO_2H$, lower alkoxycarbonyl, -COHal (wherein Hal has the same meaning as described above), -$CONR^9R^{10}$ (wherein $R^9$ and $R^{10}$ have the same meanings as described above, respectively), or -CHO, or (e) X--Y--Z represents N=C-W (wherein W has the same meaning as described above), and $R^3$ represents lower alkyl, substituted or unsubstituted aralkyl, or lower alkoxyalkyl $V^2$ may represent lower alkyl, substituted or unsubstituted aralkyl, or substituted or unsubstituted aryl) (hereinafter referred to as "condensed purine derivative").

In the definition of each of the groups in formula (I), alkyl having 1 to 9 carbon atoms can be used as the lower alkyl moiety in the lower alkyl or a substituent containing a lower alkyl moiety (for example, aralkyl). The alkyl includes linear, branched or cyclic alkyls and alkyls of combinations thereof.

The cyclic alkyl moiety in cyclic alkyl or alkyl may contain 1 or at least 2 rings. The lower alkyl represented by $R^{1A}$ is preferably linear or branched lower alkyl or mono- to tri-cyclic lower alkyl (for example, cyclopropyl, cyclobutyl, cyclopentyl, cyclohexyl, 3-noradamantyl and the like). The lower alkyl represented by $R^{2A}$ is preferably linear or branched lower alkyl or monocyclic lower alkyl.

The linear or branched lower alkyl includes, for example, methyl, ethyl, *n*-propyl, isopropyl, *n*-butyl, isobutyl, *sec*-butyl, *tert*-butyl, *n*-pentyl, neopentyl, *n*-hexyl, *n*-heptyl, *n*-octyl, *n*-nonyl and the like. The cyclic lower alkyl includes, for example, cyclopropyl, cyclobutyl, cyclopentyl, cyclohexyl, cycloheptyl, cyclooctyl, noradamantyl, adamantyl and the like. Among these, *tert*-butyl and cyclopentyl are preferred.

As the aryl or the aryl moiety in substituent containing an aryl moiety (aralkyl or the like), a monocyclic aromatic ring or an aromatic ring having at least two condensed rings can be used. More specifically, aryl having about 6 to 14

carbon atoms which constitute the ring(s) is preferable. Preferred examples include phenyl, naphthyl, indenyl, anthranyl and the like. As the aralkyl, for example, aralkyl having 7 to 15 carbon atoms can be used. Specific examples include benzyl, phenetyl, benzhydryl, naphthylmethyl and the like.

As the aromatic heterocyclic group, a monocyclic aromatic heterocyclic group or an aromatic heterocyclic group having at least two condensed rings can be used. The type and number of heteroatom contained in the aromatic heterocyclic group is not particularly limited. For example, one or at least two heteroatoms selected from the group consisting of nitrogen, sulfur and oxygen can be contained. More specifically, an aromatic heterocyclic group having about 6 to 14 carbon atoms which constitute the ring(s) is preferred and preferred examples include furyl, thienyl, pyrrolyl, imidazolyl, pyrazolyl, triazolyl, tetrazolyl, oxazolyl, thiazolyl, pyridyl, pyrazinyl, pyrimidinyl, pyridazinyl, triazinyl, indolyl indazolyl, benzoimidazolyl, benzoxazolyl, benzothiazolyl, quinolyl, isoquinolyl, phthalazinyl, naphthyridinyl, quinoxalyl, quinazolyl, cinnolyl, purinyl and the like.

When a substituent is present in the aryl ring or the aromatic heterocyclic ring, the type and number of the substituent are not particularly limited. When at least two substituents are present, they are the same or different. For example, 1 to 3 substituents can be contained on the ring. The substituent on the aryl ring or the aromatic heterocyclic ring includes, for example, substituted or unsubstituted lower alkyl, substituted or unsubstituted lower alkenyl, substituted or unsubstituted lower alkynyl, substituted or unsubstituted lower aralkyl, substituted or unsubstituted lower aryl, hydroxyl, substituted or unsubstituted lower alkoxy, substituted or unsubstituted lower aralkyloxy, substituted or unsubstituted lower aryloxy, substituted or unsubstituted lower aroyl, lower alkoxycarbonyl, substituted or unsubstituted lower alkylthio, substituted or unsubstituted lower alkylsulfonyl, carboxyl, substituted or unsubstituted carbamoyl, substituted or unsubstituted lower alkanoyl, halogen (wherein the term "halogen" in the present specification may be any of fluorine, chlorine, bromine and iodine), nitro, amino, mono- or di-lower alkyl-substituted amino, cyano and the like.

In the above-described functional groups, those described above can be used as the lower alkyl moiety in the functional group containing lower alkyl or lower alkyl moiety (for example, aralkyl, lower alkoxy, aralkyloxy, lower alkoxycarbonyl, lower alkylthio, lower alkylsulfonyl, lower alkanoyl, mono- or di-lower alkyl-substituted amino or the like). When the lower alkyl or the lower alkyl moiety contains a substituent, the number and type of the substituent are not particularly limited. When the lower alkyl or the lower alkyl moiety contains at least two substituents, the substituents are the same or different. For example, the lower alkyl or the lower alkyl moiety can contain 1 to 3 substituents. The substituents include, for example, hydroxyl halogen, carboxyl, sulfo, phosphono, an ester group derived from an acid group thereof (lower alkyl ester, aralkyl ester, aryl ester, etc.), and the like. Specific examples of the alkyl having a substituent(s) include hydroxylethyl, trifluoromethyl and the like. In the di-lower alkyl-substituted amino, two alkyls are the same or different.

In the above-described functional groups, the aralkyl or the aralkyl moiety in the aralkyloxy has the same meaning as the aralkyl described above, and the aryl, the aryloxy or the aryl moiety in the aroyl has the same meaning as the aryl described above. As the lower alkenyl, linear or branched alkenyl having 2 to 6 carbon atoms can be used Examples include vinyl, allyl, 1-propenyl, methacryl, butenyl, crotyl, pentenyl, hexenyl and the like. As the lower alkynyl, linear or branched alkynyl having 2 to 6 carbon atoms can be used. Examples include ethynyl, propynyl, butynyl, pentynyl, hexynyl and the like. The number of unsaturated bonds in the lower alkenyl or alkynyl is not particularly limited, but preferably, the number is one.

When the lower alkenyl, the lower alkynyl, the aralkyl, the aryl, the arallcyloxy, the aryloxy and the aroyl have a substituent, the number, type or substitution position of the substituent is not particularly limited. When these groups have two or more substituents, they are the same or different. These groups may have, for example, 1 to 3 substituents. As the substituent, substituents listed for the lower alkyl can be used.

In formula (I), additionally, the substitution position of $X^1$ or $X^2$ is not particularly limited They can individually be substituted on any position on the ring. When $X^1$ or $X^2$ is a substituent except hydrogen, the steric configuration of a carbon atom to which the substituent binds may be any of S or R. n is preferably 0.

In the definition of each of the groups in formula (II), linear, branched and cyclic alkyl and alkyl in combination thereof having 1 to 10 carbon atoms, can be used as the lower alkyl moiety in the lower alkyl, the lower alkylthio, the lower alkoxycarbonyl and the lower alkoxyalkyl. The cyclic lower alkyl can contain one or at least two rings. The linear or branched lower alkyl includes, for example, methyl, ethyl, *n*-propyl, isopropyl, *n*-butyl, isobutyl, *sec*-butyl, *tert*-butyl, *n*-pentyl, neopentyl, *n*-hexyl, *n*-heptyl, *n*-octyl, *n*-nonyl, *n*-decyl and the like. The cyclic lower alkyl includes, for example, cyclopropyl, cyclopropylmethyl, cyclobutyl, cyclopentyl, cyclohexyl, cycloheptyl, cyclooctyl, 1-methylcyclohexyl, 4-methylcyclohexyl, noradamantyl, adamantyl, bicyclo[2.2.1]heptyl, bicyclo[2.2.2]octyl, bicyclo[3.3.0]octyl, bicyclo [33.1]nonyl and the like.

The alkylene moiety in the lower alkoxyalkyl and the aralkyl has the same meaning as the linear or branched lower alkyl from which hydrogen atom is preliminarily removed.

The aryl and the aryl moiety in aralkyl include a monocyclic ring or a ring having at least two condensed rings, and include, for example, phenyl, naphthyl, indenyl, anthranyl and the like having 6 to 14 carbon atoms which constitute the ring.

The aromatic heterocyclic group includes, for example, a 5- or 6-membered monocyclic aromatic heterocyclic group containing at least one atom selected from nitrogen, oxygen and sulfur, and a 3- to 8-membered rings-condensed bicyclic or tricyclic condensed aromatic heterocyclic group containing at least one atom selected from nitrogen, oxygen and sulfur. Specific examples include those having 5 to 14 carbon atoms which constitute the ring(s), such as furyl, thienyl, pyrrolyl, imidazolyl, pyrazolyl, triazolyl, tetrazolyl, oxazolyl, thiazolyl, pyridyl, pyrazinyl, pyrimidinyl, pyridazinyl, triazianyl, indolyl, indazolyl, benzoimidazolyl, benzoxazolyl, benzothiazolyl, quinolyl, isoquinolyl, phthalazinyl, naphthyridinyl, quinoxalyl, quinazolyl, cinnolyl and purinyl.

The alicyclic heterocyclic group includes, for example, a 5- or 6-membered monocyclic alicyclic heterocyclic group containing at least one atom selected from nitrogen, oxygen and sulfur, and a 3- to 8-membered ring-condensed bicyclic or tricyclic condensed alicyclic heterocyclic group containing at least one atom selected from nitrogen, oxygen and sulfur. Specific examples include pyrrolidinyl, 2,5-dioxopyrrolidinyl, thiaxolidinyl, oxazolidinyl, 2-oxooxazolidinyl, piperidinyl, piperazinyl, homopiperazinyl, morpholinyl, thiomorpholinyl, tetrahydropyranyl, tetrahydrothiopyranyl, tetrahydrofuryl, tetrahydroquinolyl, tetrahydroisoquinolyl, tetrahydroquinoxalinyl, octahydroquinolyl, dihydroindolyl, 1,3-dioxoisoindolyl, 1,3-dioxohnyl, 1,3-dioxolan-2-spirocyclopentyl and the like.

The heterocyclic group formed together with the adjacent nitrogen includes, for example, a 5- or 6-membered monocyclic heterocyclic group containing at least nitrogen (wherein the monocyclic heterocyclic group may contain additional nitrogen, oxygen or sulfur), and a 3- to 8-membered, ring-condensed bicyclic or tricyclic condensed heterocyclic group containing at least one nitrogen atom (wherein the condensed heterocyclic group may contain additional nitrogen, oxygen or sulfur). More specifically, the heterocyclic group includes, for example, pyrrolidinyl, thiazolidinyl, oxazolidinyl, piperidino, homopiperidino, piperazinyl, homopiperazinyl, morpholino, thiomorpholino, tetrahydroquinolyl, tetrahydroisoquinolyl octahydroquinolyl, benzimidazolyl, indazolyl, indolyl, isoindolyl, purinyl, dihydroindolyl, pyrrolyl, pyrazolyl, triazolyl, tetrazolyl, imidazolyl and the like.

The halogen includes atoms of fluorine, chlorine, bromine and iodine.

The substituents for the substituted aryl, the substituted aralkyl, the substituted aromatic heterocyclic group and the substituted alicyclic heterocyclic group are the same or different and include 1 to 3 substituents, for example, substituted or unsubstituted lower alkyl substituted or unsubstituted aryl, substituted or unsubstituted aryloxy, substituted or unsubstituted aroyl, substituted or unsubstituted aralkyl, substituted or unsubstituted aralkyloxy, substituted or unsubstituted lower alkenyl, substituted or unsubstituted lower alkynyl, substituted or unsubstituted lower alkoxy, substituted or unsubstituted lower alkoxycarbonyl, substituted or unsubstituted lower alkylthio, substituted or unsubstituted lower alkylsulfonyl, substituted or unsubstituted lower alkanoyl, mono- or di-lower alkyl-substituted carbamoyl, mono- or di-lower alkyl-substituted amino, halogen, carboxyl, hydroxyl, nitro, amino, cyano and the like. Herein, the lower alkenyl includes, for example, linear or branched lower alkenyl having 2 to 6 carbon atoms, such as vinyl, allyl, 1-propenyl, methacryl, butenyl, crotyl, pentenyl, hexenyl and the like. The lower alkynyl includes, for example, linear or branched lower alkynyl having 2 to 6 carbon atoms, such as ethynyl, propynyl, butynyl, pentynyl, hexynyl and the like. The number of unsaturated bonds in the lower alkenyl or alkynyl is not particularly limited, but, preferably, the number is one. The lower alkyl and the lower alkyl moiety in the lower alkoxy, the lower alkoxycarbonyl, the lower alkylthio, the lower alkylsulfonyl, the lower alkanoyl, the mono- or di-lower alkyl-substituted carbamoyl and the mono- or di-lower alkyl-substituted amino have the same meanings as the above-described lower alkyl. The alkylene moiety in the aralkyl and aralkyloxy has the same meaning as the above-described alkylene moiety. The aryl and the aryl moiety in the aryloxy and the aroyl have the same meanings as the above-described aryl. The halogen has the same meaning as the above-described halogen. The substituents in the substituted lower alkyl, the substituted aryl, the substituted aryloxy, the substituted aroyl, the substituted aralkyl, the substituted aralkyloxy, the substituted lower alkenyl, the substituted lower alkynyl, the substituted lower alkoxy, the substituted lower alkoxycarbonyl, the substituted lower alkylthio, the substituted lower alkylsulfonyl and the substituted lower alkanoyl are the same or different and include, for example, hydroxyl with 1 to 3 substituents, the halogen having the same meaning as described above, carboxyl, sulfo, phosphono, ester derived from an acidic group thereof (lower alkyl ester, aralkyl ester, aryl ester, etc.; wherein the lower alkyl moiety, the aralkyl moiety and the aryl moiety in these esters have the same meanings as described above), and the like. In the di-lower alkyl-substituted carbamoyl and the di-lower alkyl-substituted amino, two lower alkyls binding to the carbamoyl and the amino, respectively, are the same or different.

The substituents in the substituted lower alkyl are the same or different and include 1 to 3 substituents, for example, lower alkoxy, hydroxyl, cyano, azide, carboxyl, phosphono or ester derived from the acidic group thereof (lower alkyl ester, aralkyl ester, aryl ester, *etc.*; wherein the lower alkyl moiety, the aralkyl moiety and the aryl moiety in these esters have the same meanings as described above), lower alkylthio, lower alkylaminocarbonyl, lower alkoxycarbonyl, a substituted or unsubstituted aromatic heterocyclic group, a substituted or unsubstituted alicyclic heterocyclic group, $-NR^{11}R^{12}$ (wherein $R^{11}$ and $R^{12}$ are the same or different and each represents hydrogen, lower alkyl, lower alkanoyl, aryl, aralkyl or aralkyloxy, or $R^{11}$ and $R^{12}$ together with adjacent nitrogen atom form a heterocyclic group), halogen, arylsulfonyloxy which may be substituted with lower alkyl, lower alkylsulfonyl, lower alkylsulfonyloxy, trifluoromethanesulfonyloxy and the like.

Herein, the lower alkyl and the lower alkyl moiety in the lower alkoxy, the lower alkylthio, the lower alkylaminocarbonyl, the lower alkoxycarbonyl, the lower alkanoyl, the arylsulfonyloxy which may be substituted with lower alkyl the lower alkylsulfonyl and the lower alkylsulfonyloxy have the same meanings as the lower alkyl described above. The aryl and the aryl moiety in the aralkyl, the aralkyloxy and the arylsulfonyloxy have the same meanings as the aryl described above. The alkylene moiety in aralkyl has the same meaning as the alkylene moiety. The halogen, the aromatic heterocyclic group, the alicyclic heterocyclic group, and the heterocyclic group formed together with the adjacent nitrogen have the same meanings as described above, respectively. The substituents in the substituted aromatic heterocyclic group and the substituted alicyclic heterocyclic group have the same meanings as described above.

Furthermore, in formula (II), the substitution position of $V^1$ or $V^2$ is not particularly limited. $V^1$ or $V^2$ may be substituted on any position in each of the rings. Additionally, when $V^1$ or $V^2$ is a substituent except hydrogen atom, the steric configuration of a carbon atom to which the substituent binds may be any of S or R. n is preferably 0.

The compound used in the present invention is preferably a compound of formula (I) wherein $R^1$ is hydrogen, lower alkyl, substituted or unsubstituted aryl, or a substituted or unsubstituted aromatic heterocyclic group; $R^2$ is a lower alacyl, or substituted or unsubstituted aryl; $R^3$ is hydrogen, or substituted or unsubstituted aralkyl; $X^1$ is hydrogen, lower alkyl, or substituted or unsubstituted aralkyl; $X^2$ is hydrogen; and n is 0; or a compound of formula (II) wherein X--Y-- Z is $R^1$N-C=O (wherein $R^1$ is hydrogen, substituted or unsubstituted lower alkyl, substituted or unsubstituted aralkyl, substituted or unsubstituted aryl, or a substituted or unsubstituted aromatic heterocyclic group); $R^2$ is hydrogen atom, substituted or unsubstituted lower alkyl, substituted or unsubstituted aralkyl substituted or unsubstituted aryl or a substituted or unsubstituted aromatic heterocyclic group; $R^3$ is hydrogen, or lower alkyl; $V^1$ is hydrogen; $V^2$ is substituted lower alkyl; and n is 0. More preferably, the compound is a compound of formula (I) wherein $R^1$ is hydrogen, *n*-propyl, cyclopentyl or *tert*-butyl; $R^2$ is *n*-propyl, ethyl or benzyl; $R^3$ is hydrogen, methyl or benzyl; $X^1$ is hydrogen, lower alkyl, or substituted or unsubstituted aralkyl; $X^2$ is hydrogen; n is 0; or a compound of formula (II) wherein X--Y--Z is $R^1$N-C=O (wherein $R^1$ is ethyl, n-propyl, cyclopropylmethyl, or benzyl); $R^2$ is *n*-propyl, cyclobutyl or cyclopentyl; $R^3$ is hydrogen; $V^1$ is hydrogen; $V^2$ is picolyl; and n is 0.

More preferable condensed purine derivatives used in the present invention are specifically compounds shown in Table 1 below.

Table 1-1

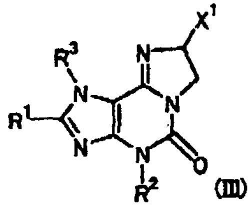

(III)

| Compound No. | $R^1$ | $R^2$ | $R^3$ | $X^1$ |
|---|---|---|---|---|
| 1 | 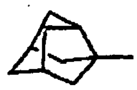 | $(CH_2)_2CH_3$ | H | 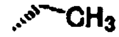 |
| 2 | 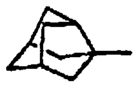 | $(CH_2)_2CH_3$ | H | 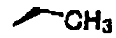 |
| 3 | 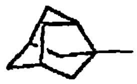 | $(CH_2)_2CH_3$ | H | 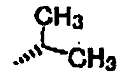 |
| 4 | 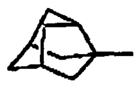 | $(CH_2)_2CH_3$ | H | 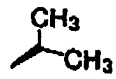 |
| 5 | 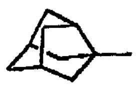 | $(CH_2)_2CH_3$ | H | H |
| 6 | 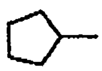 | $(CH_2)_2CH_3$ | H | 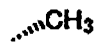 |
| 7 | 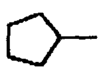 | $(CH_2)_2CH_3$ | H | 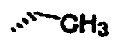 |
| 8 | 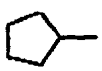 | $(CH_2)_2CH_3$ | H | 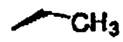 |
| 9 | 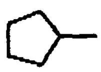 | $(CH_2)_2CH_3$ | H | 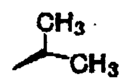 |
| 10 | 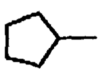 | $(CH_2)_2CH_3$ | H | 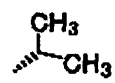 |
| 11 | 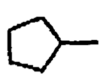 | $(CH_2)_2CH_3$ | H | 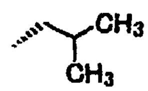 |
| 12 | 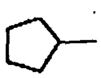 | $(CH_2)_2CH_3$ | H | 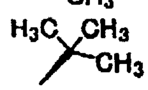 |
| 13 | 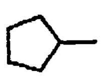 | $(CH_2)_2CH_3$ | H | 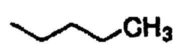 |
| 14 | 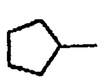 | $(CH_2)_2CH_3$ | H | 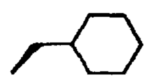 |

Table 1-2

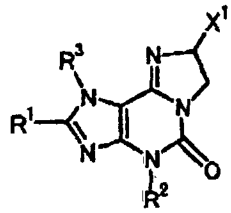

(III)

| Compound No. | $R^1$ | $R^2$ | $R^3$ | $X^1$ |
|---|---|---|---|---|
| 15 | 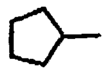 | $(CH_2)_2CH_3$ | H | 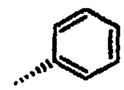 |
| 16 | 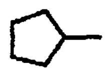 | $(CH_2)_2CH_3$ | H | 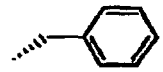 |
| 17 | 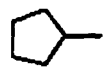 | $(CH_2)_2CH_3$ | H | 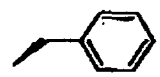 |
| 18 | 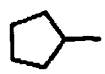 | $(CH_2)_2CH_3$ | H | 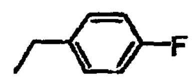 |
| 19 | 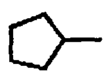 | $(CH_2)_2CH_3$ | H | 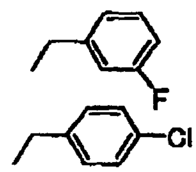 |
| 20 | 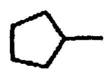 | $(CH_2)_2CH_3$ | H | |
| 21 | 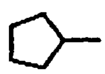 | $(CH_2)_2CH_3$ | H | 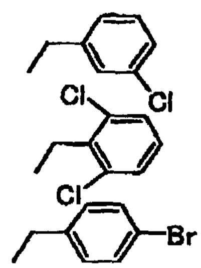 |
| 22 | 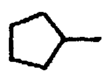 | $(CH_2)_2CH_3$ | H | |
| 23 | 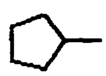 | $(CH_2)_2CH_3$ | H | |
| 24 | 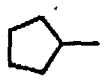 | $(CH_2)_2CH_3$ | H | 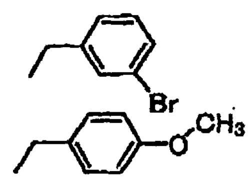 |
| 25 | 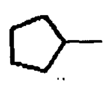 | $(CH_2)_2CH_3$ | H | |
| 26 | 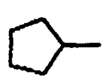 | $(CH_2)_2CH_3$ | H | 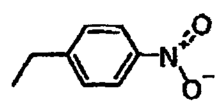 |

Table 1-3

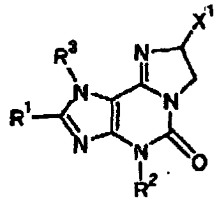

(III)

| Compound No. | $R^1$ | $R^2$ | $R^3$ | $X^1$ |
|---|---|---|---|---|
| 27 | 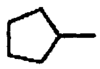 | $(CH_2)_2CH_3$ | H | 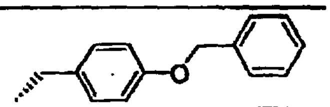 |
| 28 | 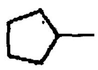 | $(CH_2)_2CH_3$ | H | 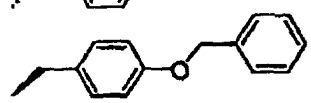 |
| 29 | 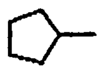 | $(CH_2)_2CH_3$ | $CH_3$ | 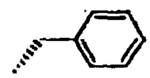 |
| 30 | 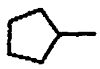 | $(CH_2)_2CH_3$ | H | 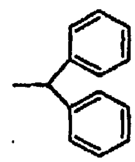 |
| 31 | 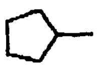 | $(CH_2)_2CH_3$ | H | 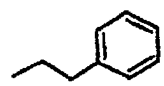 |
| 32 | 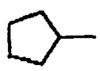 | 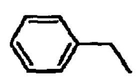 | | 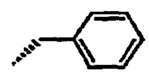 |

Table 1-4

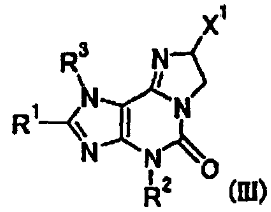

(III)

| Compound No. | $R^1$ | $R^2$ | $R^3$ | $X^1$ |
|---|---|---|---|---|
| 33 | H | $(CH_2)_2CH_3$ | H | 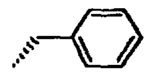 |
| 34 | H | $(CH_2)_2CH_3$ | 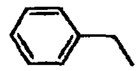 | 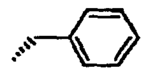 |
| 35 | 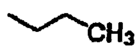 | $(CH_2)_2CH_3$ | H | 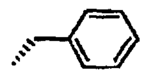 |
| 36 | 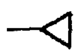 | $(CH_2)_2CH_3$ | H | 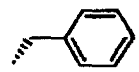 |
| 37 | 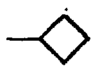 | $(CH_2)_2CH_3$ | H | 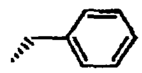 |
| 38 | 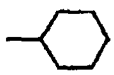 | $(CH_2)_2CH_3$ | H | 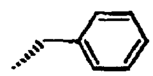 |
| 39 | 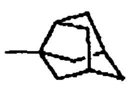 | $(CH_2)_2CH_3$ | H | 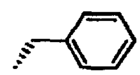 |
| 40 | 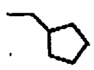 | $(CH_2)_2CH_3$ | H | 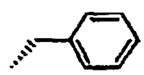 |
| 41 | 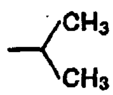 | $(CH_2)_2CH_3$ | H | 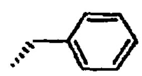 |
| 42 | 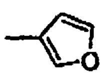 | $(CH_2)_2CH_3$ | H | 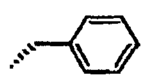 |
| 43 | 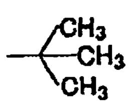 | $(CH_2)_2CH_3$ | H | 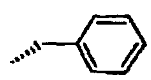 |
| 44 | 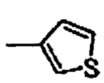 | $(CH_2)_2CH_3$ | H | 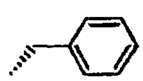 |
| 45 | 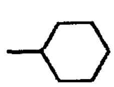 | $(CH_2)_2CH_3$ | H | 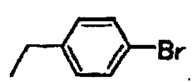 |

Table 1-5

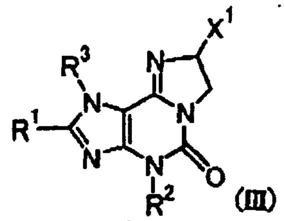

(III)

| Compound No. | $R^1$ | $R^2$ | $R^3$ | $X^1$ |
|---|---|---|---|---|
| 46 | 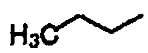 | $(CH_2)_2CH_3$ | H | 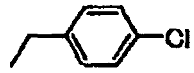 |
| 47 | 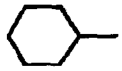 | $(CH_2)_2CH_3$ | H | 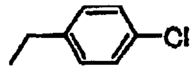 |
| 48 | 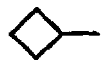 | $(CH_2)_2CH_3$ | H | 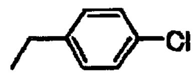 |
| 49 | 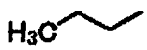 | $(CH_2)_2CH_3$ | H | 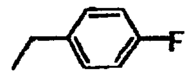 |
| 50 | 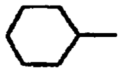 | $(CH_2)_2CH_3$ | H | 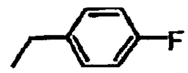 |
| 51 | 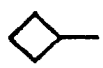 | $(CH_2)_2CH_3$ | H | 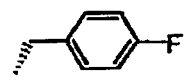 |
| 52 | 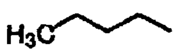 | $(CH_2)_2CH_3$ | H | 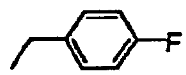 |
| 53 | 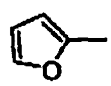 | $(CH_2)_2CH_3$ | H | 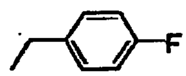 |
| 54 | 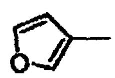 | $(CH_2)_2CH_3$ | H | 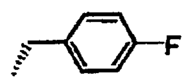 |
| 55 | 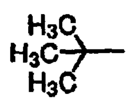 | $(CH_2)_2CH_3$ | H | 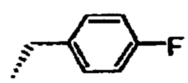 |
| 56 | 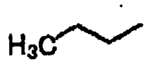 | $(CH_2)_2CH_3$ | H | 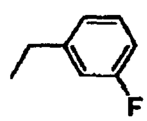 |
| 57 | 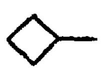 | $(CH_2)_2CH_3$ | H | 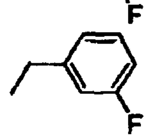 |

Table 1-6

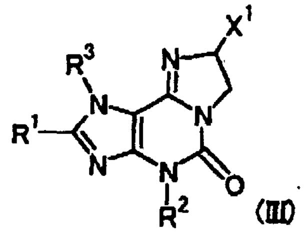

(III)

| Compound No. | $R^1$ | $R^2$ | $R^3$ | $X^1$ |
|---|---|---|---|---|
| 58 | 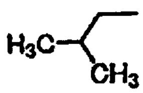 | $(CH_2)_2CH_3$ | H | 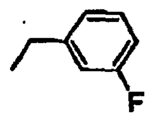 |
| 59 | 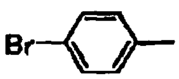 | $(CH_2)_2CH_3$ | H | 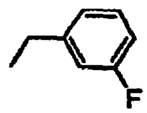 |
| 60 | 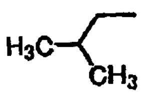 | $(CH_2)_2CH_3$ | H | 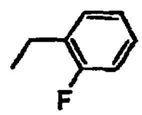 |
| 61 | 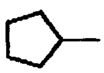 | $(CH_2)_2CH_3$ | H | 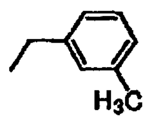 |
| 62 | 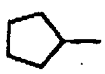 | $(CH_2)_2CH_3$ | H | 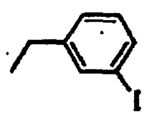 |
| 63 | 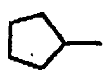 | $(CH_2)_2CH_3$ | H | 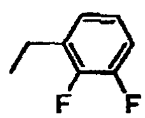 |
| 64 | 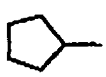 | $(CH_2)_2CH_3$ | H | 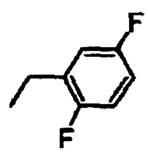 |
| 65 | 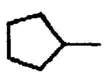 | $(CH_2)_2CH_3$ | H | 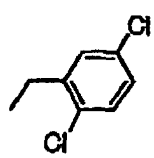 |

Table 1-7

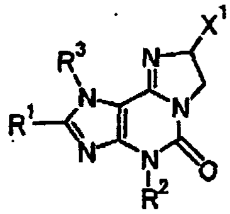

(III)

| Compound No. | $R^1$ | $R^2$ | $R^3$ | $X^1$ |
|---|---|---|---|---|
| 66 | 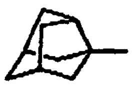 | $(CH_2)_2CH_3$ | H | 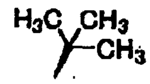 |
| 67 | 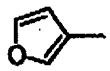 | $(CH_2)_2CH_3$ | H | 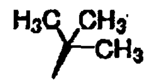 |
| 68 | 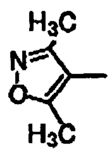 | $(CH_2)_2CH_3$ | H | 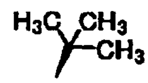 |
| 69 | 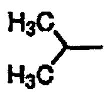 | $(CH_2)_2CH_3$ | H | 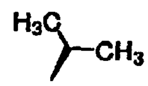 |
| 70 | 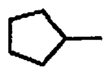 | $(CH_2)_2CH_3$ | H | 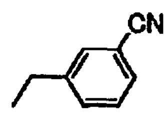 |
| 71 | 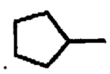 | $CH_2CH_3$ | H | 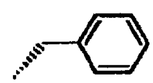 |
| 72 | 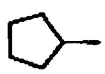 | 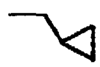 | H | 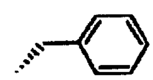 |
| 73 | 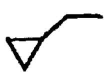 | $(CH_2)_2CH_3$ | H | 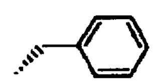 |
| 74 | 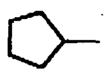 | $(CH_2)_2CH_3$ | H | 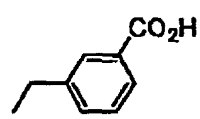 |

Table 1-8

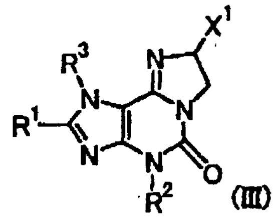 (III)

| Compound No. | $R^1$ | $R^2$ | $R^3$ | $X^1$ |
|---|---|---|---|---|
| 75 | 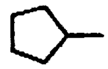 | $(CH_2)_2CH_3$ | H | 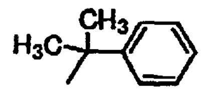 |
| 76 | 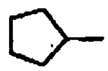 | $(CH_2)_2CH_3$ | H | 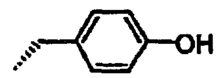 |
| 77 | 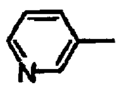 | $(CH_2)_2CH_3$ | H | 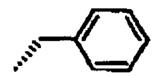 |
| 78 | 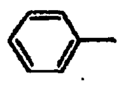 | $(CH_2)_2CH_3$ | H | 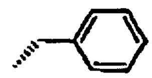 |
| 79 | 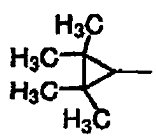 | $(CH_2)_2CH_3$ | H | 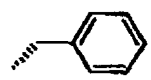 |
| 80 | 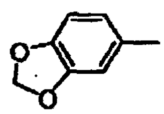 | $(CH_2)_2CH_3$ | H | 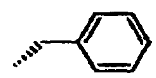 |
| 81 | 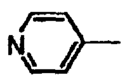 | $(CH_2)_2CH_3$ | H | 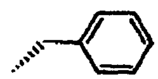 |
| 82 | 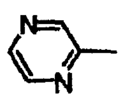 | $(CH_2)_2CH_3$ | H | 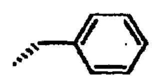 |
| 83 | 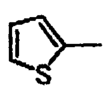 | $(CH_2)_2CH_3$ | H | 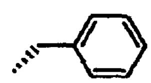 |
| 84 | 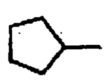 | $CH_3$ | H | 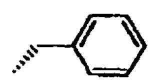 |
| 85 | 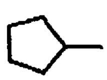 | $C_6H_5$ | H | 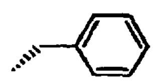 |

Table 1-9

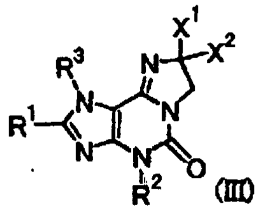

(III)

| Compound No. | $R^1$ | $R^2$ | $R^3$ | $X^1$ | $X^2$ |
|---|---|---|---|---|---|
| 86 | 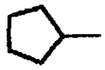 | $(CH_2)_2CH_3$ | H | 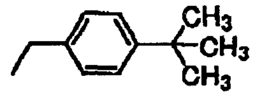 | H |
| 87 | 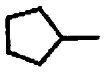 | $(CH_2)_2CH_3$ | H | 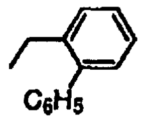 | H |
| 88 | 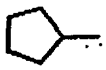 | $(CH_2)_2CH_3$ | H | 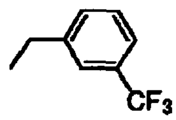 | H |
| 89 | 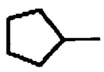 | $(CH_2)_2CH_3$ | H | 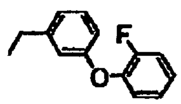 | H |
| 90 | 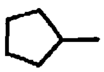 | $(CH_2)_2CH_3$ | H | 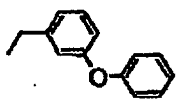 | H |
| 91 | 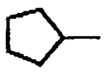 | $(CH_2)_2CH_3$ | H | 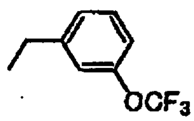 | H |
| 92 | 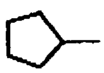 | $(CH_2)_2CH_3$ | H | 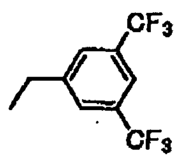 | H |
| 93 | 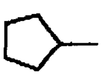 | $(CH_2)_2CH_3$ | H | | 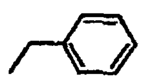 |

Table 1-10

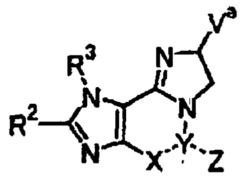 (IV)

|X--Y--Z = $R^1N-C=O$|

| Compound No. | $R^1$ | $R^2$ | $R^3$ | $V^a$ |
|---|---|---|---|---|
| 94 | $CH_3(CH_2)_2$ | 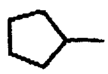 | H | 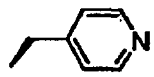 |
| 95 | $CH_3(CH_2)_2$ | 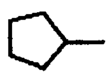 | H | 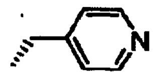 |
| 96 | $CH_3(CH_2)_2$ | 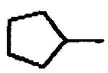 | H | 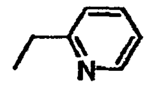 |
| 97 | $CH_3(CH_2)_2$ | 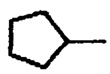 | H | 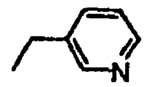 |
| 98 | $CH_3(CH_2)_2$ | 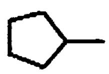 | H | 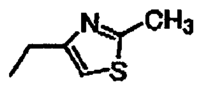 |
| 99 | $CH_3(CH_2)_2$ | 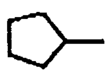 | H | 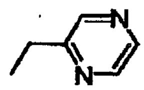 |
| 100 | $CH_3(CH_2)_2$ | 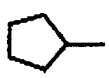 | H | 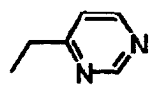 |
| 101 | $CH_3(CH_2)_2$ | $CH_3(CH_2)_2$ | H | 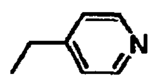 |
| 102 | $CH_3(CH_2)_2$ | 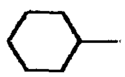 | H | 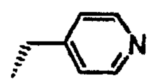 |
| 103 | $CH_3(CH_2)_2$ | 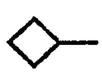 | H | 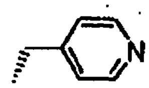 |
| 104 | $CH_3(CH_2)_2$ | $(CH_3)_3C$ | H | 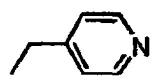 |
| 105 | $CH_3(CH_2)_2$ | 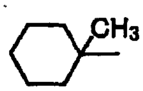 | H | 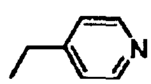 |
| 106 | $CH_3(CH_2)_2$ | H | H | 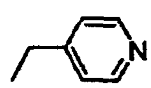 |

Table 1-11

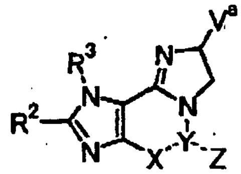

(IV)

|X--Y--Z = $R^1$N-C=O|

| Compound No. | $R^1$ | $R^2$ | $R^3$ | $V^a$ |
|---|---|---|---|---|
| 107 | 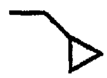 | 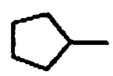 | H | 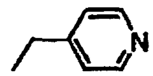 |
| 108 | 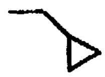 | $(CH_3)_3C$ | H | 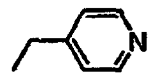 |
| 109 | $CH_2CH_3$ | 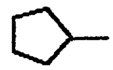 | H | 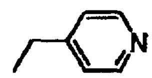 |
| 110 | $(CH_2)_2CH_3$ | 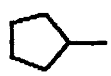 | 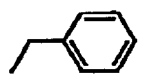 | 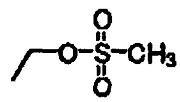 |
| 111 | $(CH_2)_2CH_3$ | 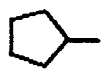 | 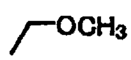 | 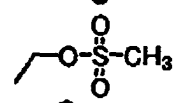 |
| 112 | $(CH_2)_2CH_3$ | 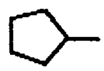 | 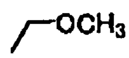 | 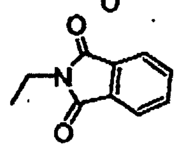 |
| 113 | $(CH_2)_2CH_3$ | 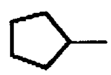 | H | 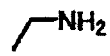 |
| 114 | $(CH_2)_2CH_3$ | 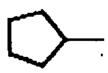 | H | 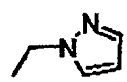 |
| 115 | $(CH_2)_2CH_3$ | 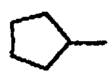 | H | 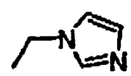 |
| 116 | $(CH_2)_2CH_3$ | 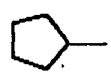 | H | 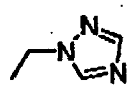 |
| 117 | $(CH_2)_2CH_3$ | 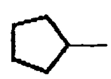 | H | 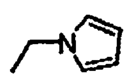 |
| 118 | $(CH_2)_2CH_3$ | 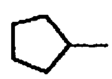 | H | 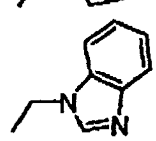 |

Table 1-12

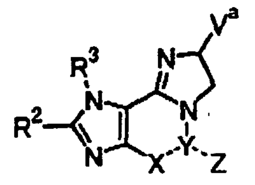

(IV)

X--Y--Z = $R^1$N–C=O

| Compound No. | $R^1$ | $R^2$ | $R^3$ | $V^a$ |
|---|---|---|---|---|
| 119 | $(CH_2)_2CH_3$ | 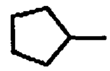 | H | 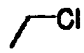 |
| 120 | $(CH_2)_2CH_3$ | 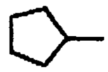 | H | 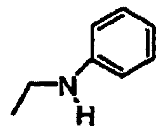 |
| 121 | $(CH_2)_2CH_3$ | 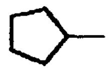 | H | 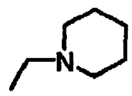 |
| 122 | $(CH_2)_2CH_3$ | 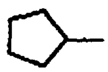 | H | 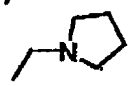 |
| 123 | $(CH_2)_2CH_3$ | 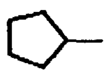 | H | 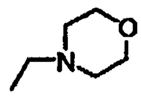 |
| 124 | $(CH_2)_2CH_3$ | 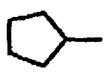 | H | 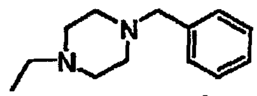 |
| 125 | $(CH_2)_2CH_3$ | 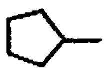 | H | 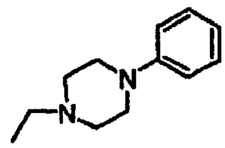 |
| 126 | $(CH_2)_2CH_3$ | 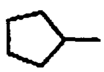 | H | 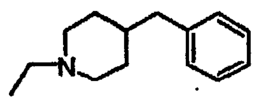 |
| 127 | $(CH_2)_2CH_3$ | 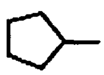 | H | 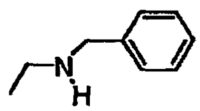 |
| 128 | $CH_3(CH_2)_2$ | Br | 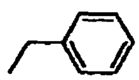 | 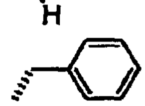 |
| 129 | $CH_3(CH_2)_2$ | $SCH_3$ | 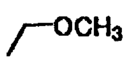 | 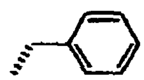 |
| 130 | $CH_3(CH_2)_2$ | 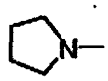 | 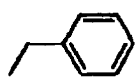 | 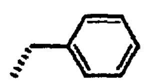 |

Table 1-13

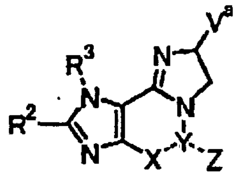

(IV)

|X--Y--Z = $R^1$N-C=O|

| Compound No. | $R^1$ | $R^2$ | $R^3$ | $V^a$ |
|---|---|---|---|---|
| 131 | $(CH_2)_2CH_3$ | 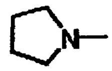 | H | 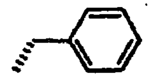 |
| 132 | $(CH_2)_2CH_3$ | 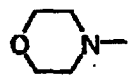 | H | 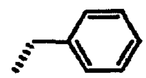 |
| 133 | $(CH_2)_2CH_3$ | 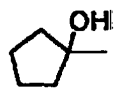 | H | 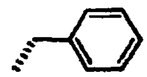 |
| 134 | $(CH_2)_2CH_3$ | CHO | 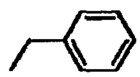 | 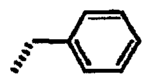 |
| 135 | $(CH_2)_2CH_3$ | $ClCH_2$ | 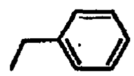 | 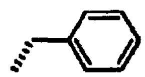 |
| 136 | $(CH_2)_2CH_3$ | $(CH_3)_2NHCH_2$ | H | 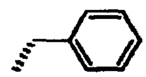 |
| 137 | $(CH_2)_2CH_3$ | 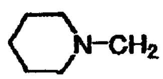 | H | 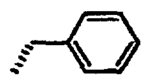 |
| 138 | $(CH_2)_2CH_3$ | $CH_3CH_2OCH_2$ | H | 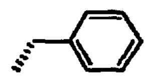 |
| 139 | $(CH_2)_2CH_3$ | 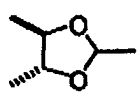 | H | 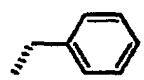 |
| 140 | $(CH_2)_2CH_3$ | $CO_2CH_3$ | 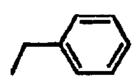 | 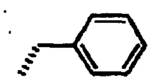 |
| 141 | $(CH_2)_2CH_3$ | 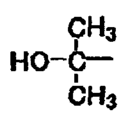 | 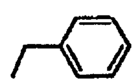 | 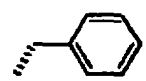 |
| 142 | $(CH_2)_2CH_3$ | 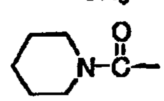 | H | 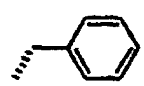 |
| 143 | $(CH_2)_2CH_3$ | 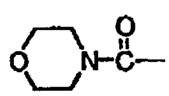 | H | 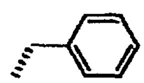 |

Table 1-14

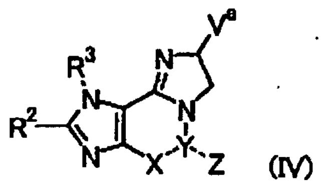 (IV)

|X--Y--Z = $R^1$N—C=O|

| Compound No. | $R^1$ | $R^2$ | $R^3$ | $V^a$ |
|---|---|---|---|---|
| 144 | $CH_3(CH_2)_2$ | 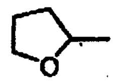 | H | 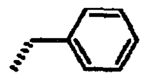 |
| 145 | $CH_3(CH_2)_2$ | 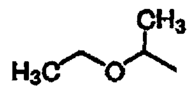 | H | 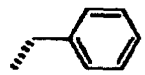 |
| 146 | $CH_3(CH_2)_2$ | 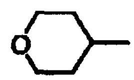 | H | 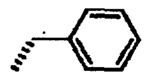 |
| 147 | $CH_3(CH_2)_2$ | 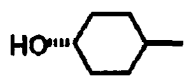 | H | 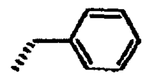 |
| 148 | $CH_3(CH_2)_2$ | 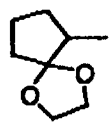 | H | 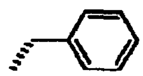 |
| 149 | $CH_3(CH_2)_2$ | 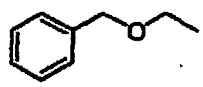 | H | 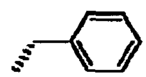 |
| 150 | $CH_3(CH_2)_2$ | 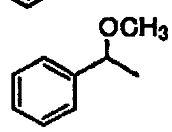 | H | 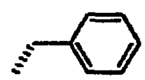 |
| 151 | $CH_3(CH_2)_2$ | 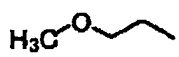 | H | 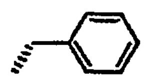 |
| 152 | $CH_3(CH_2)_2$ | 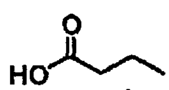 | H | 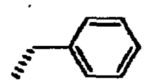 |

Table 1-15

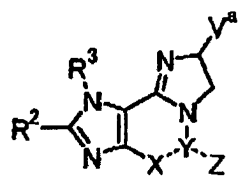 (IV)

|X--Y--Z = $R^1N-C=O$|

| Compound No. | $R^1$ | $R^2$ | $R^3$ | $V^a$ |
|---|---|---|---|---|
| 153 | $CH_3(CH_2)_2$ | 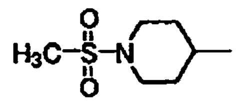 | H | 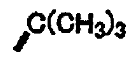 |
| 154 | $CH_3(CH_2)_2$ | 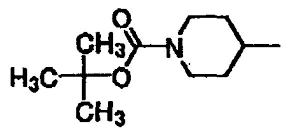 | H | 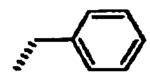 |
| 155 | $CH_3(CH_2)_2$ | 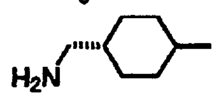 | H | 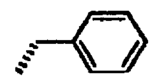 |
| 156 | $CH_3(CH_2)_2$ | 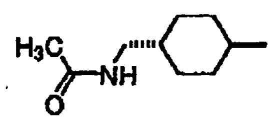 | H | 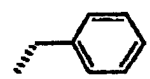 |
| 157 | $CH_3(CH_2)_2$ | $CH_3CH_2SCH_2$ | H | 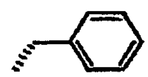 |
| 158 | $CH_3(CH_2)_2$ | 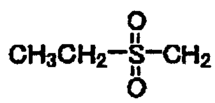 | H | 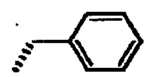 |

Table 1-16

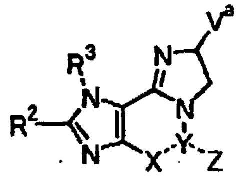

(IV)

X··Y··Z = N=C-W

| Compound No. | W | $R^2$ | $R^3$ | $V^a$ |
|---|---|---|---|---|
| 159 | Cl | 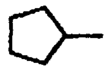 | 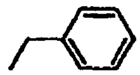 | 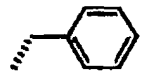 |
| 160 | 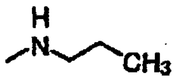 | 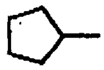 | H | 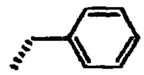 |
| 161 | 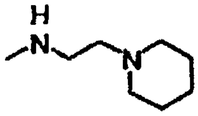 | 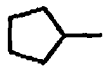 | H | 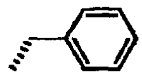 |
| 162 | 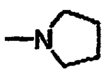 | 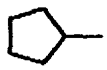 | H | 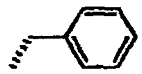 |
| 163 | 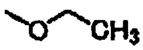 | $(CH_3)_3C$ | H | 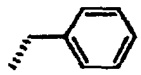 |
| 164 | 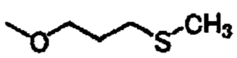 | 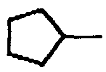 | H | 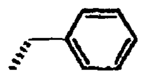 |
| 165 | 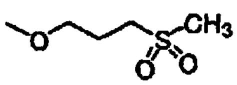 | 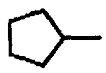 | H | 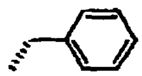 |
| 166 | $SCH_3$ | $(CH_3)_3C$ | H | 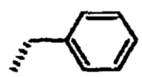 |
| 167 | $CH_2CH_3$ | 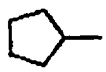 | H | 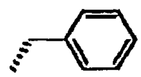 |
| 168 | C≡N | 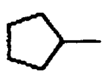 | H | 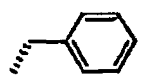 |
| 169 | 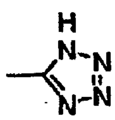 | 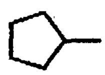 | H | 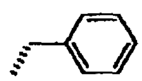 |

Table 1-17

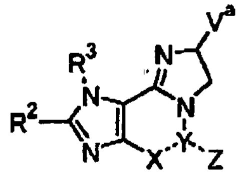

(IV)

|X--Y--Z = $R^1$N–C=O|

| Compound No. | $R^1$ | $R^2$ | $R^3$ | $V^a$ |
|---|---|---|---|---|
| 170 | 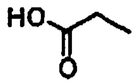 | 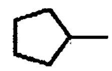 | H | 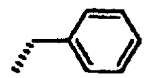 |
| 171 | 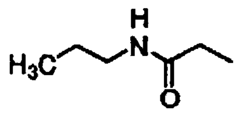 | 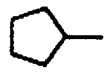 | H | 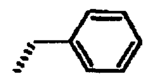 |
| 172 | 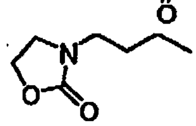 | 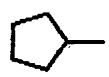 | H | 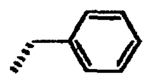 |
| 173 | 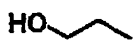 | 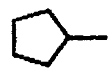 | H | 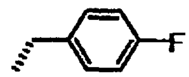 |
| 174 | 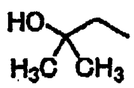 | 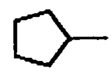 | H | 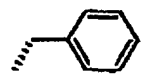 |
| 175 | 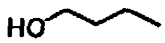 | 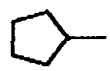 | H | 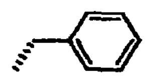 |
| 176 | 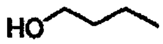 | $(CH_3)_3C$ | H | 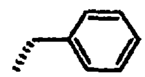 |
| 177 | 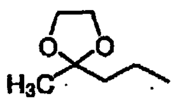 | 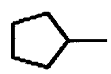 | H | 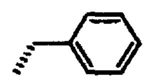 |
| 178 | 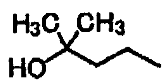 | 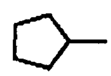 | H | 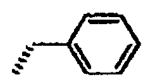 |
| 179 | 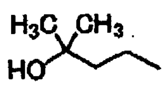 | $(CH_3)_3C$ | H | 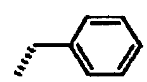 |

Table 1-18

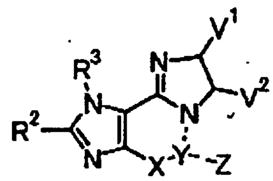

(IV)

|X--Y--Z = $R^1N-C=O$|

| Compound No. | $R^1$ | $R^2$ | $R^3$ | $V^1$ | $V^2$ |
|---|---|---|---|---|---|
| 180 | $CH_3(CH_2)_2$ | $CH_3CH_2OCH_2$ | H | 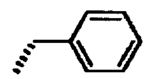 | H |
| 181 | $CH_3(CH_2)_2$ | $CH_3CH_2OCH_2$ | H | H | 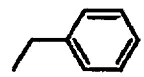 |
| 182 | $CH_3(CH_2)_2$ | 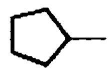 | H | H | 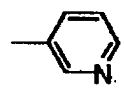 |

Table 1-19

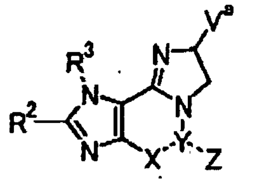 (IV)

$|X{\cdot\cdot}Y{\cdot\cdot}Z = R^1N{-}C{=}O|$

| Compound No. | $R^1$ | $R^2$ | $R^3$ | $V^a$ |
|---|---|---|---|---|
| 183 | $CH_3(CH_2)_2$ | $CH_3$ | H | 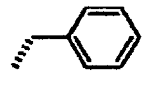 |
| 184 | $CH_3(CH_2)_2$ | $CH_3(CH_2)_3$ | H | 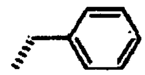 |
| 185 | $CH_3(CH_2)_2$ | $CH_3(CH_2)_2$ | H | 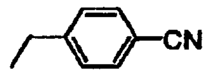 |
| 186 | $CH_3(CH_2)_2$ | 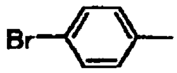 | H | 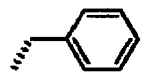 |
| 187 | $CH_3(CH_2)_2$ | 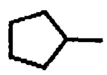 | H | 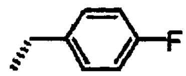 |
| 188 | $CH_3(CH_2)_2$ | 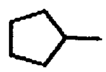 | H | 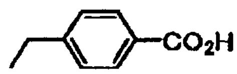 |
| 189 | 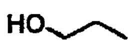 | 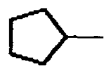 | H | 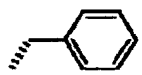 |
| 190 | 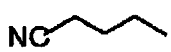 | 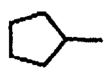 | H | 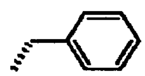 |
| 191 | 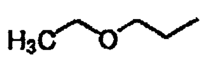 | 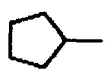 | H | 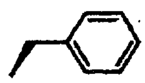 |
| 192 | $CH_3(CH_2)_2$ | 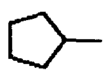 | H | 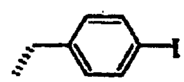 |

A more preferred compound is a compound referred to as Compound No. 16 in Table 1 above, which is represented by the following formula (III) (hereinafter sometimes referred to as "Compound 16").

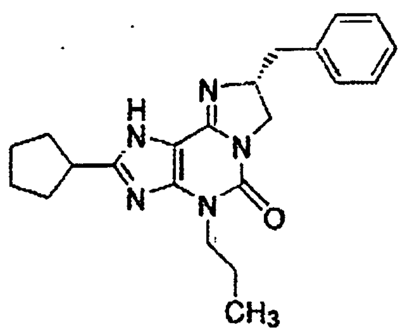

(III)

2. Method for producing condensed purine derivative used in the screening method of the present invention

Methods for producing specific compounds of the compound represented by formula (I) are specifically described in WO98/15555 and are also described in Japanese Published Unexamined Patent Application No. 204880/91; WO00/01388; *J. Med Chem.,* 35, 3578 (1992); *J. Med. Chem.,* 36, 2508 (1993); *J. Heterocyclic Chem.,* 30, 241 (1993); and the like.
Methods for producing specific compounds of the compound represented by formula (II) are specifically described in WO01/47931 and the like.
Conversion of individual functional groups in raw material compounds and intermediate compounds and conversion of emotional groups in substituents can be carried out by known methods other than the methods described in the references [for example, the method described in *Comprehensive Organic Transformations,* Second Edition, R. C.Larock, John Wiley & Sons Inc. (1999)].
All compounds which are within formulae (I) and (II) can be produced by an appropriate combination of the methods described above and the like and, if necessary, modification and variation of those methods.
The intermediate compounds and intended compounds obtained by the production methods can be isolated and purified by general purification methods in organic synthetic chemistry, for example, neutralization, filtration, extraction, washing, drying, concentration, recrystallization, and various chromatographic means. Furthermore, the intermediate compounds may be subjected to the following reactions without particular purification.
In the production of salts of the compounds represented by formulae (I) and (II), compounds in free form are dissolved or suspended in an appropriate solvent, and appropriate acid or base is added thereto to form salts, if necessary, followed by separation and purification. Substances of interest obtained in salt forms may be converted to free forms, which are then converted to desired salts.

3. Screening method of substance having anti-diabetic activity which uses the condensed purine derivative and a pancreatic β cell or a treated product of the cell

A substance having anti-diabetic activity can be selected by

[1] bringing the condensed purine derivative described above in the above item 1 into contact with a pancreatic β cell or a treated product of the cell in the presence of a tested substance to assay the binding amount of the condensed purine derivative to the pancreatic β cell or the treated product of the cell,
[2] bringing the condensed purine derivative into contact with a pancreatic β cell or a treated product of the cell in the absence of the tested substance to assay the binding amount of the condensed purine derivative to the pancreatic β cell or the treated product of the cell,
[3] comparing the assayed amount in the presence of the tested substance with that in the absence of the tested substance, and
[4] selecting a substance which inhibits the binding of the condensed purine derivative to the pancreatic β cell or the treated product of the cell.

[1] Method for producing labeled condensed purine derivative

In the above-described screening method, the condensed purine derivative described in the above item 1 can be used as it is. However, the condensed purine derivative which is labeled is preferably used.
The compound labeled includes, for example, condensed purine derivatives labeled with radioactive labels

such as $^3H$, $^{125}I$ and $^{13}C$, fluorescence labels such as FITC (fluorescein isothiocyanate), and biotin labels. Preferably, the condensed purine derivative is radiologically labeled with $^3H$.

The condensed purine derivative described in the above item 1 can be labeled by well known methods. In radioactive labeling, the method includes, for example, a method which uses a radiolabeled raw material commercially available for synthetically preparing the condensed purine derivative used in the screening method described in the above item 2, and a method in which a condensed purine derivative having halogen as a functional group is directly bringing into contact with a radioactive element to introduce the radioactive element into the condensed purine derivative described in the above item 1.

Specifically, when a labeled form of Compound 16 is used in the screening method, the R form of the compound referred to as Compound 23 in Table 1 is catalytically hydrogenated in the presence of palladium in $^3H$ gas atmosphere to obtain $^3H$-labeled Compound 16.

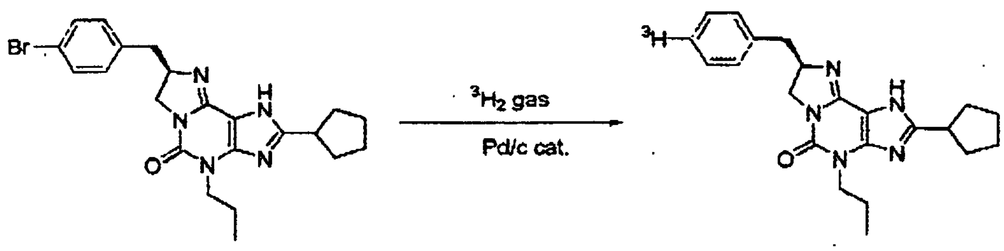


[2] Method for preparing a pancreatic β cell or a treated product of the cell

A pancreatic β cell or a treated product of the cell used in the screening method of the present invention may be a cell derived from any animal or a treated product of the cell. Preferable examples include pancreatic β cell derived from rat, mouse, dog or humans or the like and a treated product of the cell.

Additionally, an organ or tissue containing a pancreatic β cell can be used in the screening method of the present invention, so long as specific binding of the organ or tissue to the condensed purine derivative is observed.

Specifically, pancreas and Langerhans islands derived from animals such as rat, mouse, dog, and humans, a pancreatic β cell itself which can be isolated from pancreas, or a cell culture obtained by culturing the cell, or a product prepared by treating them can be used in the screening method of the present invention.

As the pancreatic β cell, pancreatic β cell itself, which can be isolated from pancreas by general methods, can be used. Preferably, however, immortalized or cell-lined cell is used.

Immortalized or cell-lined pancreatic β cell specifically includes, for example, BRIN-BD11 cell *[Diabetes,* 45, 1132-1140 (1996)], INS-1 cell *[Endocrinology,* 130, 167-178 (1992)], βTC cell [*Proc. Natl. Acad. Sci. USA,* 85, 9037-9041 (1988)], MIN6 cell [*Endocrinology*, 127, 126-132 (1990)], RINm5F [*Diabetes*, 31, 521-531 (1982)], H1T-T15 cell (ATCC CRL-1777) and the like. MIN6 cell is more preferred.

The treated product of a pancreatic β cell includes a treated product of a pancreatic β cell or a tissue containing the cell with a surfactant, ultrasonic wave, mechanical grinding, a solvent or an enzyme, an immobilized product of a pancreatic β cell or a tissue containing the cell, a cell membrane fraction, a protein-fractionated product, and an enzyme preparation which can be prepared from a pancreatic β cell or a tissue containing the cell by the usual method, and the like.

The treated product with a surfactant can be prepared using a surfactant which is generally used in solubilizing animal cell membranes, such as Triton X-100, NP-40, digitonin or sucrose monocapronate.

The treated product with mechanical grinding can be prepared by using a homogenizer, ultrasonic wave, a French Press or the like.

The protein-fractionated product and the enzyme preparation can be prepared from a mechanically grinding-treated product of the cell or tissue by using a method, for example, solvent extraction, salting-out using ammonium sulfate, etc, desalting, precipitation using an organic solvent, anion exchange chromatography using a resin such as diethylamino ethyl (DEAE)-Sepharose and DIAION HPA-75 (manufactured by Mitsubishi Chemical Corporation), cation exchange chromatography using a resin such as S-Sepharose FF (manufactured by Pharmacia K, K.), hydrophobic chromatography using a resin such as butyl Sepharose and phenyl Sepharose, gel filtration using a molecular sieve, affinity chromatography, chromatofocusing, and electrophoresis such as isoelectric electrophoresis, alone or in combination thereof.

[3] Method for detecting the labeled condensed purine derivative bound to a pancreatic β cell or a treated product of the cell

The labeled condensed purine derivative bound to a pancreatic β cell or a treated product of the cell can be measured by separating a labeled condensed purine derivative bound to a pancreatic β cell or a treated product of the cell from the unbound labeled condensed purine derivative and then detecting the labeled condensed purine derivative.
The method for separating the labeled condensed purine derivative bound to a pancreatic β cell or a treated product of the cell from the unbound labeled condensed purine derivative includes a separation method by centrifugation in the case where a binding experiment is carried out by using a pancreatic β cell or an insoluble treated product of the cell. It includes a method which separates a fraction containing the protein or the enzyme sample or the like by gel filtration in the case where a treated product of the pancreatic β cell is a soluble substance such as a protein fraction or an enzyme preparation.
When the labeled condensed purine derivative is labeled with a radioactive isomer, the derivative can be detected and assayed by using a liquid scintillation counter or the like. When the derivative is labeled with a fluorescent substance, the derivative can be detected and assayed by using a fluorescence detector or the like. When the derivative is labeled with biotin, the derivative can be detected and assayed by reacting the derivative with avidin to which an appropriate enzyme is added and assaying the activity of the enzyme. Based on the assayed amount, the amount of the labeled condensed purine derivative bound to a pancreatic β cell or a treated product of the cell can be determined.

[4] Method for assaying insulin secretion-promoting activity

It can be confirmed that a substance selected as a substance which inhibits the binding of the condensed purine derivative to a pancreatic β cell or a treated product of the cell is a substance having anti-diabetic activity, for example, by allowing the selected substance into contact with a pancreatic β cell or a tissue containing the cell in the presence of glucose so that insulin secretion from the pancreatic β cell is promoted by the contact to the substance [*Diabetorogia,* 36, 1139 (1993)].
The screening methods in the above items [1] to [3] as a screening method of a substance having anti-diabetic activity, which comprises selecting a substance which inhibits the binding of the condensed purine derivative to a pancreatic β cell or a treated product of the cell by using the binding activity of the condensed purine derivative thereto as a marker, are far more excellent methods in terms of, for example, operability and rapidity, in comparison with a direct screening method of a substance promoting the activity promoting insulin secretion of pancreatic β cell by using the activity as a marker.

4. Screening method of a substance having anti-diabetic activity by using a protein capable of binding to the condensed purine derivative

[1] Method for selecting a protein capable of binding to the condensed purine derivative

As the method for selecting a protein capable of binding to the condensed purine derivative used in the present invention, any known method can be used, so long as it is a method for selecting a protein specifically binding to the condensed purine derivative. For example, the method includes affinity purification method in which the condensed purine derivative is immobilized onto an insoluble polymer called carrier and is mixed with an extract solution of a cell used for evaluation of the binding activity, followed by washing, to identify a molecule binding to the condensed purine derivative [*Current Protocols in Protein Science* (Chapter 9), John Wiley & Sons (2001)].

(1) Method for preparing a carrier

As the carrier used for selecting a protein capable of binding to the condensed purine derivative used in the present invention, any carrier can be used, so long as it is a carrier capable of immobilizing the condensed purine derivative. Preferably, a carrier having a functional group capable of binding to the condensed purine derivative by chemical reaction on the surface of the carrier and having less non-specific adsorption of the protein and great operability is preferred.
Specifically, the material of the carrier includes, for example, agarose, dextran, cellulose, polyacrylamide, polystyrene and the like. Regarding the size of the carrier, particles of a diameter of 10 nm to 1 mm, preferably 50 nm to 100 μm, can be used. As the form of the carrier, it may be porous, perforans, non-porous or the like. The carrier preferably used in the present invention includes, for example, NHS-activated Sepharose 4 Fast Flow (manufactured by Amersham Biosciences), Affi-Gel 10 (manufactured by Bio-Rad), and the like
As the condensed purine derivative capable of being immobilized onto the carrier in the present invention,

any condensed purine derivative can be used, so long as it is capable of being immobilized onto the carrier. Examples. of the condensed purine derivative capable of being immobilized onto the carrier include the compound represented by the following formula (IV) (hereinafter referred to as "Compound 155"):

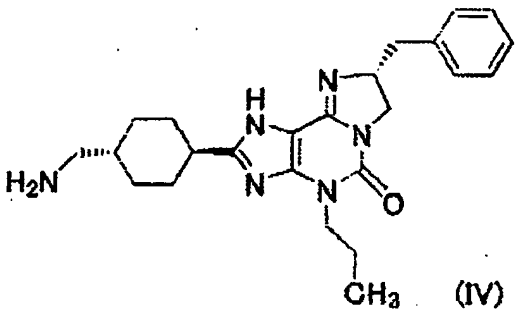

(IV)

As the chemical reaction for binding the condensed purine derivative to the carrier, any reaction can be used, so long as it is a reaction which is not disadvantageous for use in the present invention.

A condensed purine derivative-immobilized carrier can be prepared by reacting a functional group on the carrier with the condensed purine derivative. For example, a carrier having succinimidyl carboxylic acid as a functional group is brought into contact with a solution of the condensed purine derivative to react the succinimidyl carboxylic acid on the carrier with the condensed purine derivative. As the solvent and reaction conditions at that time, any combination of a solvent and reaction conditions can be used, so long as it is a combination for promoting the above immobilization reaction and is a combination which never allows the carrier having succinimidyl carboxylic acid and the condensed purine derivative to cause decomposition reactions, condensation reactions and the like except the above immobilization reaction to such a level that these reactions inhibit the objective of the present invention. Specifically, the carrier having succinimidyl carboxylic acid is brought into contact with a solution containing the condensed purine derivative at 1 μmol/l to 1 mol/l, and the reaction is carried out at 0°C to 100°C for one second to 1,000 hours while allowing the mixture to stand or gently stirring it by using a rotator or mixer or the like to thereby prepare the condensed purine derivative-immobilized carrier. The solvent includes, for example, an aqueous solution containing a salt, a surfactant and a buffering component, water, *N,N*-dimethylformamide (DMF), dimethylsulfoxide (DMSO), tetrahydrofuran (THF), dioxane, methanol and the like.

After the completion of the reaction, the condensed purine derivative-immobilized carrier is washed so as to remove the unreactive condensed purine derivative. Any known washing method capable of stably and efficiently recovering the condensed purine derivative-immobilized carrier and efficiently removing the unreactive condensed purine derivative can be used. When the carrier is, for example, a particle, the reaction mixture is centrifuged (15,000 rpm, 5 minutes, room temperature) after the completion of the reaction; the supernatant is removed, while the pellet is suspended in a solvent; and then, centrifugation and supernatant removal are repeated in the same manner, to wash the carrier. When the carrier is a plate, the plate is washed with a solvent. Also, if necessary, the solvent can be substituted with a solvent suitable for storage and for mixing with the cell extract solution. The solvent includes, for example, an aqueous solution containing a salt, a surfactant and a buffering component, water, DMF, DMSO, THF, dioxane, methanol and the like.

When the unreactive functional group remains on the condensed purine derivative-immobilized carrier, the unreactive functional group can be blocked, if necessary. Blocking can be carried out by reacting a compound which reacts with the functional group but does not react with the condensed purine derivative immobilized on the carrier, with the condensed purine derivative-immobilized carrier. For blocking, any compound can be used, so long as it is a compound which reacts with the functional group but does not react with the condensed purine derivative immobilized on the carrier. When the functional group of the carrier is succinimidyl carboxylic acid, for example, Tris and ethanolamine can be used to block the unreactive succinimidyl carboxylic acid on the condensed purine derivative-immobilized carrier.

The condensed purine derivative used in the present invention may also be immobilized on the carrier via a spacer compound. As the spacer compound, any compound can be used, so long as the compound is not disadvantageous for use in the present invention. For example, the spacer compound includes spacer compounds commercially available from, for example, Pierece, Molecular Probes and Shear Water. Specifically, the spacer compound includes, for example, succinimidyl 6-[3-(2-pyridyldithio)propionamide]hexanoate (LC-SPDP) (manufactured by Pierece), succinimidyl 3-(2-pyridyldithio)propionate (S-1531) (manufactured by Molecular Probes), and the like. Additionally, 6-aminohexanoic acid and 4-amino-n-butyric acid can also be used as the spacer compound.

The method for binding the condensed purine via the spacer compound to the carrier may be any method, so long as the method is not disadvantageous for use in the present invention. The method includes, for example, a method in which a spacer compound is introduced into a carrier and then the condensed purine derivative is bound to the spacer compound and a method in which a spacer compound is bound to the condensed purine derivative and then the spacer compound is immobilized onto the carrier.

Additionally, the condensed purine derivative-immobilized carrier can be prepared by chemically protecting one of the functional groups of the spacer compound, reacting the spacer compound with a carrier or the condensed purine derivative, deprotecting the resulting product and reacting then the product with the condensed purine derivative or the carrier.

In the method wherein the spacer compound is introduced into a carrier and then is bound to the condensed purine derivative, for example, a carrier having a succinimidyl carboxylic acid group as a functional group reacts with a spacer compound which is a carboxylic acid compound having primary or secondary amine to prepare a carrier having a carboxyl group on the surface of the carrier. The carboxylic acid compound having primary amine or secondary amine includes, for example, 6-aminohexanoic acid, 4-amino-n-butyric acid and the like.

As the solvent and reaction conditions at that time, any combination of a solvent and reaction conditions can be used, so long as it is a combination for promoting the above immobilization reaction and is a combination which never allows the carrier and the spacer compound to cause decomposition reactions, condensation reactions and the like except the above immobilization reaction to such a level that these reactions inhibit the immobilization reaction. Specifically, a carrier is brought into contact with a 1 μmol/l to 50 mol/l solution containing a compound having primary or secondary amine, for example, 6-aminohexanoic acid and 4-aminobutyric acid, and then the reaction is carried out at 0°C to 100°C for one second to 1,000 hours while allowing the mixture to stand or gently stirring it by using a rotator or mixer or the like to thereby prepare a carrier having a carboxyl group on the surface. The solvent includes, for example, water, DMF, DMSO, THF, dioxane, methanol and the like.

In order to remove the unreactive spacer compound after the completion of the reaction, the carrier is washed. As the washing method, any known method can be used, so long as it is a method capable of stably and efficiently recovering the spacer compound-immobilized carrier and efficiently removing the unreactive spacer compound. When the carrier is a particle, for example, the reaction mixture is centrifuged (at 15,000 rpm; for 5 minutes; at room temperature) after the completion of the reaction, the supernatant is removed, and the precipitate is suspended in a solvent, followed by centrifugation and removal of the supernatant in the same manner as described above, and the procedure is repeated to wash the carrier. When the carrier is a plate, the plate is washed with a solvent. If necessary, the solvent may be substituted with a solvent suitable for the following reaction in the same manner.

The condensed purine derivative-immobilized carrier can be prepared by reacting the carboxyl group on the thus obtained carrier with the condensed purine derivative. Specifically, a spacer compound-immobilized carrier is brought into contact with a solution of the condensed purine derivative to react the carboxyl group on the carrier with the condensed purine derivative.

As the solvent and reaction conditions at that time, any combination of a solvent and reaction conditions can be used, so long as it is a combination'for promoting the above immobilization reaction and is a combination which never allows the spacer compound-immobilized carrier and the condensed purine derivative to cause decomposition reactions, condensation reactions and the like except the above immobilization reaction to such a level that these reactions inhibit the immobilization reaction. Specifically, a spacer-immobilized carrier is brought into contact with a 1 μmol/l to 50 mol/l solution containing the condensed purine derivative, and the reaction is carried out at 0°C to 100°C for one second to 1,000 hours while allowing the mixture to stand or gently stirring it by using a rotator or mixer or the like to thereby prepare a condensed purine derivative immobilized-carrier on the surface thereof. The solution includes, for example, a solution of the condensed purine dissolved in an aqueous solution containing a salts, a surfactants and a buffering component, water, DMF, DMSO, THF, dioxane, methanol and the like.

In order to remove the unreactive condensed purine derivative after the completion of the reaction, the condensed purine derivative immobilized-carrier is washed by the method described above.

When the unreactive functional group still remains on the condensed purine derivative-immobilized carrier, the unreactive functional group can be blocked by the above-described method.

Other methods for preparing a condensed purine derivative immobilized-carrier include a method the condensed purine derivative is allowed to react with a spacer compound to synthetically prepare a complex of the spacer compound-condensed purine derivative so as to bind the condensed purine derivative on the carrier, and then the compound is bound onto the carrier.

The complex of the spacer compound-condensed purine derivative can be prepared by reacting the spacer compound with the condensed purine derivative. As the solvent and reaction conditions at that time, any combination of a solvent and reaction conditions can be used, so long as it is a combination for promoting the above reaction and is a combination which never allows the spacer, the condensed purine derivative and the complex of the spacer compound-condensed purine derivative to cause decomposition reactions, condensation reactions and the like except the

above binding reaction to such a level that these reactions inhibit the formation reaction of the complex of the spacer compound and the condensed purine derivative.

Specifically, for example, a spacer compound containing 1 μmol/l to 1 mol/l succinimidyl carboxylic acid and the condensed purine derivative at 1 μmol/l to 1 mol/l are mixed, and the reaction is carried out at 0 °C to 100 °C for one second to 1,000 hours while allowing the mixture to stand or gently stirring it by using a rotator or mixer or the like to thereby prepare the complex of the spacer-condensed purine derivative. The solvent includes, for example, an aqueous solution containing a salt, a surfactant and a buffering component, water, DMF, DMSO, THF, dioxane, methanol and the like.

The produced complex of the spacer compound-condensed purine derivative can be isolated and purified by thin-layer chromatography, HPLC, biphasic separation or the like.

The condensed purine derivative-immobilized carrier can be prepared by reacting a carrier with the complex of the spacer compound-condensed purine derivative as prepared by the above-described method.

As the solution and reaction conditions at that time, any combination of a solution and reaction conditions can be used, so long as it is a combination for promoting the above immobilization reaction and is a combination which never allows the carrier and the complex of the spacer compound-condensed purine derivative to cause decomposition reactions, condensation reactions and the like except the above immobilization reaction to such a level that these reactions inhibit the binding reaction of the complex and the carrier.

Specifically, the method includes an immobilization method similar to the method in which the carrier is brought into contact with a solution of the complex of the spacer-condensed purine derivative at 1 μmol/l to 1 mol/l and the above-described spacer compound is immobilized onto the carrier.

The condensed purine derivative immobilized-carrier as obtained in such manner is brought into contact with a solvent for storage. The solvent and storage conditions at that time may be any combination of a solvent and storage conditions, so long as the condensed purine derivative immobilized-carrier is stable in the solvent under the storage conditions. Specifically, the solvent includes, for example, an aqueous solution containing a salt, a surfactant and a buffering component, water, DMF, DMSO, THF, dioxane, methanol and the like. The storage is carried out at room temperature or less. More preferably, the storage is carried out in darkness in atmosphere substituted with inert gas such as helium gas or argon gas.

(2) Method for preparing a cell extract solution containing a protein capable of binding to the condensed purine derivative (hereinafter referred to as "condensed purine derivative-binding protein").

The origin of the condensed purine derivative-binding protein used in the screening method of the present invention is not particularly limited, so long as the protein specifically binds to the condensed purine derivative. The protein is preferably a protein derived from a eukaryote, more preferably a protein derived from an animal, and most preferably a protein derived from a mammalian apparatus, organ, tissue or cell.

As the apparatus, organ or tissue, any known tissue can be used, and organs and tissues such as pancreas and Langerbans islands are preferably used. As the cell, any known cell can be used, and pancreatic β cell lines, primary cell cultures of pancreatic B cell and the like are preferably used. Specifically, the β cell lines include, for example, BRIN-BD11 cell [*Diabetes,* 45, 1132-1140 (1996)], INS-1 cell [*Endocrinology,* 130, 167-178 (1992)], βTC cell [*Proc. Natl. Acad Sci. USA,* 85, 9037-9041 (1988)]. MIN6 cell [*Endocrinology,* 127, 126-132 (1990)], HIT-T15 cell (ATCC CRL-1777), RINm5F cell [*Diabetes*, 31, 521-531 (1982)], and the like. The primary cell cultures of the pancreatic β cell include, for example, cells prepared by culturing cells recovered from tissues such as Langerhans islands for a short period of time.

These cells can be cultured by the method described in *The Guide to Animal Cell Culture Method* [Japan Scientific Societies Press (1993)].

As the method for preparing a sample containing the condensed purine derivative-binding protein from the above-described apparatus, organ, tissue or cell, any known method can be used, so long as the protein can be recovered by the method. The sample can be prepared by using, for example, the method described in *Fundamental Experimental Method of Proteins and Enzymes,* the 2nd edition (Nankodo, 1994). As the method for disrupting the apparatus, organ, tissue or cell, any known disruption method can be used, and the protein sample can be prepared by a combination of, for example, mechanical disruption using an apparatus such as a wearing blender, a Polytron, a Dounce type homogenizer and an ultrasonicator, physical disruption using a hypotonic solution, procedures of solubilization using a surfactant, centrifugation, and the like.

The protein sample can be stored at a refrigerated state or at a frozen state.

(3) Screening method of the condensed purine derivative-binding protein used in the present invention

The condensed purine derivative-binding protein can be screened by using the condensed purine derivative-immobilized carried which can be prepared by the method in the above (1) and the protein sample prepared by the method in the (2) above.

As the screening method of the condensed purine derivative-binding protein, any known method can be used, so long as it is a method in which the condensed purine derivative can be screened. The protein can be screened by using, for example, the method described in *Fundamental Experimental Method of Proteins and Enzymes,* the 2nd edition (Nankodo, 1994),.

Specifically, the condensed purine derivative-immobilized carrier prepared by the method in the above (1) is dispersed into a protein sample solution prepared by the method in the (2) above, and the reaction is carried out at 0°C to 50°C for one second to 1,000 hours while allowing the dispersion to stand or gently stirring it with a rotator-mixer or the like to thereby adsorb the condensed purine derivative-binding protein onto the condensed purine derivative-immobilized carrier. After the completion of the binding reaction, the condensed purine derivative-immobilized carrier is washed to remove proteins which are non-specifically adsorbed onto the condensed purine derivative-immobilized carrier. As the solution used in such binding or washing, any solution can be used, so long as the solution can be used in the present invention, and examples include the solution described in *Fundamental Experimental Method of Proteins and Enzymes,* the 2nd edition (Nankodo, 1994).

The condensed purine derivative-binding protein after the protein is adsorbed on the condensed purine derivative-immobilized carrier can be eluted from the condensed purine derivative-immobilized carrier by a means which reduces the binding to the condensed purine derivative. As the means which reduces the binding to the condensed purine derivative, any means can be used, so long as the means can be used in the method of the present invention. The means includes, for example, addition of a denaturing agent, increase of a salt concentration, heating, addition of a surfactant and the like. The denaturing agent includes SDS, urea, guanidine hydrochloride and the like; the salt includes sodium chloride and potassium chloride and the like; and the surfactant includes Nonidet P40, Triton, Tween and digitonin and the like. Additionally, the heating means raising the temperature to 30 to 100°C. The protein capable of binding the condensed purine derivative as thus purified can be detected by a protein analysis method such as SDS-PAGE, two dimensional electrophoresis or mass spectrometry.

A protein which specifically binds to the condensed purine derivative can be screened by comparing the binding ability of the protein capable of binding the condensed purine derivative to the condensed purine derivative-immobilized carrier with that to a carrier treated by only blocking.

(4) Method for identifying condensed purine derivative-binding protein

The condensed purine derivative-binding protein can be separated by SDS-PAGB, stained with Coomassie-Brilliant Blue (CBB) and subsequently scissored out from the gel. The protein in the gel can be fractionated with lysyl endopeptidase and endoproteases having specific cleavage sites such as trypsin into peptide fragments. The resulting peptide fragments are purified by reverse phase HPLC and the like to determine partial amino acid sequences of the protein by using a gas phase protein sequencer or a mass spectrometer. By using the information about these partial amino acid sequences, homology search to the amino acid sequence database of PTR, SwissProt and the like and the nucleotide sequence database such as GenBank is carried out to find the homology of the protein to known proteins.

The protein identified by the methods described above includes a protein comprising the amino acid sequence represented by SEQ ID NO:2. The protein is known as an enzyme having NADPH-dependent redox activity of retinol and short-chain alcohol [*Biochimica et Biophysica Acta,* 1338, 47 (1997)].

(5) Condensed purine derivative-binding protein used in the screening method of the present invention

As the condensed purine derivative-binding protein used in the screening method of the present invention, any protein can be used, so long as it is identified by the methods in the above (3) and (4). The origin thereof is not particularly limited. Examples include

(i) a protein which comprises the amino acid sequence represented by any one of SEQ ID NOs:1 to 3;
(ii) a protein which consists of an amino acid sequence in which at least one amino acid is deleted, substituted, inserted or added in the amino acid sequence represented by any one of SEQ ID NOs:1 to 3, and is capable of binding to the condensed purine derivative; and
(iii) a protein which consists of an amino acid sequence having a homology of 60% or more with the amino acid sequence represented by any one of SEQ ID NOs:1 to 3, and is capable of binding to the condensed purine derivative.

The protein which consists of an amino acid sequence in which at least one amino acid is deleted, substituted, inserted or added in the amino acid sequence represented by any one of SEQ ID NOs:1 to 3 can be obtained, for

example, by introducing mutation(s) to a DNA encoding a protein comprising the amino acid sequence represented by any one of SEQ ID NO:1 to 3 according to a method for introducing site-directed mutagenesis described in *Molecular Cloning, A Laboratory Manual,* Third Edition, Cold Spring Harbor Laboratory Press (1989) (hereinafter referred to as "*Molecular Cloning,* Third Edition"); *Current Protocols in Molecular Biology*, John Wiley & Sons (1987-1997) (hereinafter referred to as "*Current Protocols in Molecular Biology*")*; Nucleic Acids. Research,* 10*,* 6487 (1982); *Proc. Natl. Acad. Sci.* USA, 79, 6409 (1982); *Gene,* 34, 315 (1985); *Nucleic Acids. Research,* 13, 4431 (1985); *Proc. Natl. Acad. Sci. USA,* 82, 488 (1985) and the like.

The number of the amino acids which are deleted, substituted, inserted or added is not particularly limited; however, it is such a number that deletion, substitution, insertion or addition can be carried out by a known method such as method for introducing site-directed mutation(s). The number is 1 to several tens, preferably 1 to 20, more preferably 1 to 10, and most preferably 1 to 5.

The deletion, substitution, insertion or addition of at least one amino acid residue in the amino acid sequence represented by any one of SEQ ID NOs:1 to 3 means that one or at least two amino acids are deleted, substituted, inserted or added at any position in the same sequence. The deletion, substitution or addition can be carried out in the same amino acid sequence simultaneously. Also, the amino acid residue substituted or added can be natural or non-natural. The natural amino acid residue includes L-alanine, L-asparagine, L-asparatic acid, L-glutamine, L-glutamic acid, glycine, L-histidine, L-isoleucine, L-leucine, L-lysine, L-methionine, L-phenylalanine, L-proline, L-serine, L-threonine, L-tryptophan, L-tyrosine, L-valine, L-cysteine, and the like.

Herein, examples of amino acid residues which are substituted with each other are shown below. Amino acid residues in the same group can readily be substituted with each other.

GroupA:
leucine, isoleucine, norleucine, valine, norvaline, alanine, 2-aminobutanoic acid, methionine, *O*-methylserine, *t*-butylglycine, *t*-butylalanine, cyclohexylalanine;
Group B:
asparatic acid, glutamic acid, isoasparatic acid, isoglutamic acid, 2-aminoadipic acid, 2-aminosuberic acid;
Group C:
asparagine, glutamine;
Group D:
lysine, arginine, ornithine, 2,4-diaminobutanoic acid, 2,3-diaminopropionic acid;
Group E:
proline, 3-hydrozylproline, 4-hydroxylproline;
Group F:
serine, threonine, homoserine;
Group G:
phenylalanine, tyrosine.

Also, in order that the protein which consists of an amino acid sequence in which at least one amino acid is deleted, substituted, inserted or added in the amino acid sequence represented by any one of SEQ ID NOs:1 to 3 is a protein capable of binding to the condensed purine derivative, the protein has a homology of 60% or more, generally 80% or more, preferably 95% or more, and more preferably 98% or more, with the amino acid sequence represented by any one of SEQ ID NOs:1 to 3.

The homology of an amino acid sequence or a nucleotide sequence can be determined by using the algorithm BLAST by Karlin and Altschl [*Proc. Natl. Acad. Sci*. *USA,* 90,5873 (1993)] or FASTA [*Methods Enzymol.,* 183, 63 (1990)]. The programs called BLASTN and BLASTX have developed based on the above algorithm BLAST [*J. Mol. Biol.,* 215, 403 (1990)]. In the case of analyzing a nucleotide sequence by BLASTN based on BLAST, for example, the parameter can be set to score = 100, wordlength = 12. Also, in the case of analyzing an amino acid sequence by BLASTX based on BLAST, for example, the parameter can be set to score = 50, wordlength = 3. When BLAST and Gapped BLAST programs are used, a default parameter of each program can be used. The specific analysis methods of using the above programs are known (http://www.ncbi.nlm.nih.gov.).

It can be confirmed by the following method that a protein which consists of an amino acid sequence in which at least one amino acid is deleted, substituted, inserted or added in the amino acid sequence represented by any one of SEQ ID NOs:1 to 3 is a protein which is capable of binding to the condensed purine derivative.

The condensed purine derivative-immobilized carrier obtained by the method in the above (1) is brought into contact with the protein in a solvent which does not inhibit the binding of the condensed purine derivative to the protein for a sufficient period of time. After the reaction, the carrier is separated by centrifugation and is suspended in a solvent having no activity of dissociating the binding of the condensed purine derivative to the protein. The same procedures are repeated several times to wash the carrier.

The protein binding to the condensed purine derivative can be eluted from the condensed purine derivative-immobilized carrier by a means which reduces the binding to the condensed purine derivative. The means which reduces the binding of the condensed purine derivative to the bound protein includes the method described in the above (3). In the case where a band of the protein can be confirmed by SDS-polyacrylamide electrophoresis when the eluted protein is subjected to electrophoresis, the protein is a condensed purine derivative-binding protein.

[2] Method for preparing condensed purine derivative-binding protein used in the screening method of the present invention

(1) Preparation from organs and the like

The condensed purine derivative-binding protein used in the screening method of the present invention may by any known method, so long as it can be used for the method of the present invention, and it can be prepared by using the method described in *Fundamental Experimental Method of Proteins and Enzymes,* the 2nd edition (Nankodo, 1994).

The condensed purine derivative-binding protein used in the screening method of the present invention can be prepared by disrupting an apparatus, an organ, a tissue or a cell of a eukaryote as an unpurified fraction, a partially purified fraction and a purified fraction. The eukaryote may be any known organism, is preferably a mammal. The apparatus, organ and tissue may be any known apparatus, organ and tissue and include, for example, pancreas, Langerhans islands and the like. The cells may be any known cell and includes, for example, BRIN-BD11 cell [*Diabetes,* 45, 1132-1140 (1996)], INS-1 cell [*Endocrinology,* 130 167-178 (1992)], βTC cell [*Proc. Natl. Acad. Sci. USA,* 85. 9037-9041 (1988)], MIN6 cell [*Endocrinology,* 127, 126-132 (1990)], HIT-T15 cell (ATCC CRL-1777), RINm5F [*Diabetes,* 31, 521-531 (1982)] and the like.

As the method for disrupting the apparatus, organ, tissue or cell, any known disruption method can be used. A protein traction containing the condensed purine derivative-binding protein can be prepared by a combination of, for example, mechanical disruption using an apparatus such as a wearing blender, a Polytron, a Dounce type homogenizer and an ultrasonicator, physical disruption using a hypotonic solution, and procedures of solubilization using a surfactant, centrifugation, and the like.

The protein faction can be stored at a refrigerated state or at a frozen state.

The method for purifying the condensed purine derivative-binding protein. from the protein fraction includes, for example, a purification method by ion exchange chromatography, hydrophobic chromatography, gel filtration, affinity chromatography using a condensed purine derivative-immobilized carrier, and the like, alone or in combination thereof.

The amount of the condensed purine derivative-binding protein can be assayed by immunoblotting using an antibody reacting against the condensed purine derivative-binding protein.

The level of the binding activity of the purified protein to the condensed purine derivative can be assayed by using the condensed purine derivative-immobilized carrier as prepared by the method in the above [1](1). Specifically, the binding complex of the condensed purine derivative and the condensed purine derivative-binding protein is treated by a means which reduces the binding to thereby elute the condensed purine derivative-binding protein from the binding complex. The means which reduces the binding of the condensed purine derivative to the bound protein includes the method in the above [1](3). In the case when a band of the protein can be confirmed by SDS-polyacrylamide electrophoresis when the eluted protein is subjected to electrophoresis, the level of the binding activity of the protein contained in the fractionated fraction can be assayed.

When the activity of the condensed purine-bound protein is known, the binding activity can be assessed by assaying the activity of the protein.

Regarding the condensed purine derivative-binding protein comprising the amino acid sequence represented by any one of SEQ ID NOs:1 to 3 (hereinafter referred to as "p31"), the binding activity of the protein can be assayed by assaying the activity of the protein according to the method described in *Current Protocols in Protein Science,* John Wiley & Sons (2001).

Furthermore, the method for purifying the condensed purine derivative-binding protein from the protein fraction prepared above also includes a purification method by affinity chromatography using an antibody which reacts with the protein. In other words, a monoclonal antibody or a polyclonal antibody which reacts with the protein is obtained, the antibody is crosslinked with a carrier for chromatography by using the method described in, for example, Harlow, E. and Lane, D., *Using antibodies: A laboratory manual*, Cold Spring Harbor Laboratory Press, Cold Spring Harbor, New York, USA (1999) to prepare an affinity column, and the condensed purine derivative-binding protein can be purified from the protein fraction or a partially purified fraction by using the column.

(2) Preparation of condensed purine derivative-binding protein using a recombinant cell

The condensed purine derivative-binding protein used in the present invention can be prepared by using a DNA encoding the protein. The DNA can be obtained, using a synthetic DNA which can be prepared based on the amino acid sequence of the condensed purine derivative-binding protein identified in the above [1](4) and encodes the amino acid sequence, by using a technique such as colony hybridization or plaque hybridization (*Molecular Cloning,* Third Edition) for a cDNA library prepared from the cell, tissue or the like which produces the protein.
Furthermore, the nucleotide sequence of the DNA encoding the protein comprising the amino acid sequence can be obtained by using the amino acid sequence as a query and screening known databases.
The DNA obtainable by the above method includes, for example,

(i) a DNA comprising the nucleotide sequence represented by any one of SEQ ID NOs:4 to 6;
(ii) a DNA encoding the protein described in any one of (i) to (iii) in the above item [1](5); and
(iii) a DNA which hybridizes with a DNA comprising the nucleotide sequence represented by any one of SEQ ID NOs:4 to 6 under stringent conditions and encodes a protein which is capable of binding to the condensed purine derivative.

The DNA which is hybridizable under stringent conditions is a DNA obtained by colony hybridization, plaque hybridization, Southern hybridization or the like using, as a probe, a part or a full length of a DNA consisting of a complementary sequence of the nucleotide sequence represented by any one of SEQ ID NOs:4 to 6. Specifically, the DNA includes a DNA which can be identified by carrying out hybridization at 65°C in the presence of 0.9 mol/l NaCl using a filter on which a DNA prepared from colonies or plaques is immobilized, and then washing the filter with 0.1× to 2× SSC solution (the composition of 1× SSC solution contains 150 mmol/l sodium chloride and 15 mmol/l sodium citrate) at 65°C.
The hybridization can be carried out in accordance with a known method described in, for example, *Molecular Cloning,* Second Edition; *Current Protocols in Molecular Biology, DNA Cloning 1: Core Techniques, A Practical Approach,* Second Edition, Oxford University (1995) or the like. Specifically, the DNA which is hybridizable includes a DNA having a homology of at least 60% or more, preferably 80% or more, more preferably 95% or more, and most preferably 98% or more, with the nucleotide sequence represented by any one of SEQ ID NOs:4 to 6 when calculated based on the above parameters using above BLAST, FASTA or the like.
The condensed purine derivative-binding protein can be produced by expressing the DNA encoding the condensed purine derivative-binding protein in a host cell according to the method described below by using a method described in *Molecular Cloning,* Third Edition, *Current Protocols in Molecular Biology* or the like.
Based on the full length cDNA, if necessary, a DNA fragment of an appropriate length containing a region which encodes the protein can be prepared.
In addition, if necessary, a DNA is prepared by substituting a nucleotide(s) in the nucleotide sequence of the region encoding the condensed purine derivative-binding protein so that it has the most suitable codons for the expression in the host cell. The DNA is useful for efficient production of the condensed purine derivative-binding protein.
A recombinant vector is prepared by inserting the DNA fragment or the full length cDNA into a downstream of the promoter of an appropriate expression vector.
The recombinant vector is introduced into a host cell suitable for the expression vector.
Any bacterium, yeast, animal cell, insect cell, plant cell and the like can be used as the host cell, so long as it can express the gene of interest.
The expression vector which can be used is one which can replicate autonomously in the above host cell or those which can be integrated into a chromosome and have a promoter at such a position that the DNA encoding the condensed purine derivative-binding protein can be transcribed.
When a procaryote such as bacterium is used as the host cell, it is preferred that the recombinant vector which comprises the DNA encoding the condensed purine derivative-binding protein can replicate autonomously in the procaryote, and that the vector comprises a promoter, a ribosome binding sequence, the DNA of the present invention and a transcription termination sequence. The vector may further comprise a gene regulating the promoter.
The expression vector includes pBTrp2, pBTac1 and pBTac2 (all manufactured by Boehringer Mannheim), pKK233-2 (manufactured by Pharmacia), pSE280 (manufactured by Invitrogen), pGEMEX-1 (manufactured by Promega), pQE-8 (manufactured by QIAGEN), pKYP10 (Japanese Published Unexamined Patent Application No. 110600/83), pKYP200 [*Agric. Biol. Chem.,* 48, 669 (1984)], pLSA1 [*Agric. Biol. Chem.,* 53, 277 (1989)], pGEL1 [*Proc. Natl. Acad. Sci. USA,* 82, 4306 (1985)], pBluescript II SK(-) (manufactured by Stratagene), pTrs30 [prepared from *Escherichia coli* JM109/pTrS30 (FERM BP-5407)], pTrs32 [prepared from *Escherichia coli* JM109/pTrS32 (FERM BP-5408)], pGHA2 [prepared from *Escherichia coli* IGHA2 (FERM BP-400), Japanese Published Unexamined Patent Application No. 221091/85], pGKA2 [prepared from *Escherichia coli* IGKA2 (FERM BP-6798), Japanese Published Unexamined Patent Application No. 221091/85], pTerm2 (US4686191, US4939094, US5160735), pSupex, pUB110, pTP5, pC194, pEG400 [*J. Bacteriol.*, 172, 2392 (1990)], pGEX (manufactured by Pharmacia), pET system (manufactured by Novagen), and the like.

Any promoter can be used, so long as it can function in the host cell. Examples include promoters derived from *Escherichia coli*, phage and the like, such as *trp* promoter ($P_{trp}$), *lac* promoter, $P_L$ promoter, $P_R$ promoter and T7 promoter. Also, artificially designed and modified promoters, such as a promoter in which two $P_{trp}$ are linked in tandem ($P_{trp}\times2$), *tac* promoter, *lac*T7 promoter and *let*I promoter, can be used.

It is preferred to use a plasmid in which the space between Shine-Dalgarno sequence, which is the ribosome binding sequence, and the initiation codon is adjusted to an appropriate distance (for example, 6 to 18 nucleotides).

The transcription termination sequence to express the DNA of the condensed purine derivative-binding protein is not essential for the recombinant vector of the present invention. However, it is preferred to lie a transcription terminating sequence immediately downstream of the structural gene.

The host cells include microorganisms belonging to the genera *Escherichia, Serratia, Bacillus, Brevibacterium, Corynebacterium, Microbacterium*, *Pseudomonas* and the like. Examples include *Escherichia coli* XL1-Blue, *Escherichia coli* XL2-Blue, *Escherichia coli* DH1, *Escherichia coli* MC1000, *Escherichia coli* KY3276, *Escherichia coli* W1485, *Escherichia coli* JM109, *Escherichia coli* HB101, *Escherichia coli* No.49, *Escherichia coli* W3110, *Escherichia coli* NY49, *Escherichia coli* GI698, *Escherichia coli* TB1, *Serratia ficaria, Serratia fonticola, Serratia liquefaciens, Serratia marcescens, Bacillus subtilis, Bacillus amyloliquefaciens, Brevibacterium ammoniagenes, Brevibacterium immariophilum* ATCC14068, *Brevibacterium saccharolyticum* ATCC14066, *Brevibacterium flavum* ATCC14067, *Brevibacterium lactofermentum* ATCC13869, *Corynebacterium glutamicum* ATCC13032, *Corynebacterium glutamicum* ATCC13869, *Corynebacterium acetoacidophilum* ATCC13870, *Microbacterium ammoniaphilum* ATCC15354, *Pseudomonas putida, Pseudomonas* sp. D-0110 and the like.

Introduction of the recombinant vector can be carried out by any methods for introducing DNA into the above-described host cells, such as the method using a calcium ion [*Proc. Natl. Acad. Sci. USA*, 69, 2110 (1972)], the protoplast method (Japanese Published Unexamined Patent Application No. 248394/88) and methods described in *Gene*, 17, 107 (1982) and *Molecular & General Genetics*, 168, 111 (1979).

When yeast is used as the host cell, the expression vector includes YEp13 (ATCC 37115), YEp24 (ATCC 37051), YCp50 (ATCC 37419), pHS19, pHS15 and the like.

Any promoter can be used so long as it can function in yeast. Examples include a promoter of a gene in a glycolysis system such as hexose kinase, PHO5 promoter, PGK promoter, GAP promoter, ADH promoter, gal 1 promoter, gal 10 promoter, a heat shock polypeptide promoter, MF$\alpha$1 promoter, CUP1 promoter and the like.

The host cell includes yeast strain belonging to the genera *Saccharomyces, Schizosaccharomyces, Kluyveromyces, Trichosporon, Schwanniomyces, Pichia, Candida* and the like. Examples include *Saccharomyces cerevisiae, Schizosaccharomyces pombe, Kluyveromyces lactis, Trichosporon pullulans, Schwanniomyces alluvius, Candida utilis* and the like.

Introduction of the recombinant vector can be carried out by any of the methods for introducing a DNA into yeast, such as electroporation [*Methods. Enzymol.*, 194, 182 (1990)], the spheroplast method [*Proc. Natl. Acad. Sci. USA*, 75, 1929 (1978)], the lithium acetate method [*J. Bacteriol.*, 153, 163 (1983)] and a method described in *Proc. Natl. Acad Sci. USA*, 75, 1929 (1978).

When an animal cell is used as the host, the expression vector includes pcDNAI and pcDM8 (manufactured by Invitrogen), pAGE107 [Japanese Published Unexamined Patent Application No. 22979/91, *Cytotechnology*, 3, 133 (1990)], pAS3-3 (Japanese Published Unexamined Patent Application No. 227075/90), pcDNAI/Amp (manufactured by Invitrogen), pREP4 (manufactured by Invitrogen), pAGE103 [*J. Biochem.*, 101, 1307 (1987)], pAGE210 and the like.

Any promoter can be used, so long as it can function in an animal cell. Examples include a promoter of IE (immediate early) gene of cytomegalovirus (CMV), SV40 early promoter, a metallothionein promoter, a promoter of retrovirus, a heat shock promoter, SR$\alpha$ promoter and the like. Also, the enhancer of the IE gene of human CMV can be used together with the promoter.

The host cell includes human Namalwa cell, monkey COS cell, Chinese hamster ovary (CHO) cell, HST5637 (Japanese Published Unexamined Patent Application No. 299/88) and the like.

Introduction of the recombinant vector into an animal cell can be carried out by any method, so long as it is a method for introducing a DNA into an animal cell. Examples include electroporation [*Cytotechnology*, 3, 133 (1990)], the calcium phosphate method (Japanese Published Unexamined Patent Application No. 227075/90), the lipofection method [*Proc. Natl. Acad. Sci. USA*, 84, 7413 (1987)], and the method described in *Virology*, 52, 456 (1973).

When an insect cell is used as the host, the polypeptide can be expressed by a method described in, for example, *Current Protocols in Molecular Biology, Baculovirus Expression Vectors, A Laboratory Manual*, W.H. Freeman and Company, New York (1992), *Bio/Technology*, 6, 47 (1988) or the like.

Specifically, a recombinant gene transfer vector and baculovirus are co-transfected into an insect cell to obtain a recombinant virus in a supernatant of the culture of its insect cell, and then an insect cell is infected with the resulting

recombinant virus to produce the protein.

The transfer vector used in the method includes pVL1392, pVL1393 and pBlueBacIII (all manufactured by Invitrogen), and the like.

The baculovirus includes *Autographa californica* nuclear polyhedrosis virus which infects insects of the family *Barathra* and the like.

The insect cell includes *Spodoptera frugiperda* ovary cells Sf9 and Sf21 [*Baculovirus Expression Vectors, A Laboratory Manual,* W.H. Freeman and Company, New York (1992)], *Trichoplusia ni* ovary cell High 5 (manufactured by Invitrogen) and the like.

The method for co-transfecting the above transfer vector and the above baculovirus for the preparation of the recombinant virus includes the calcium phosphate method (Japanese Published Unexamined Patent Application No. 227075/90), the lipofection method [*Proc. Natl. Acad Sci. USA,* 84, 7413 (1987)] and the like.

When a plant cell is used as the host cell, the expression vector includes Ti plasmid, a tobacco mosaic virus vector, and the like.

As the promoter, any promoter can be used, so long as it can function in a plant cell Examples include 35S promoter of cauliflower mosaic virus (CaMV), rice actin 1 promoter and the like.

The host cell includes a plant cell and the like, such as tobacco, potato, tomato, carrot, soybean, rape, alfalfa, rice, wheat and barley.

The method for introducing the recombinant vector may be any method, so long as it is a method for introducing a DNA into a plant cell, and examples include *Agrobacterium* method (Japanese Published Unexamined Patent Application No. 140885/84, Japanese Published Unexamined Patent Application No. 70080/85, WO94/00977), electroporation (Japanese Published Unexamined Patent Application No. 251887/85), the method using a particle gun (Japanese Patent No. 2606856 and Japanese Patent No. 2517813) and the like.

The condensed purine derivative-binding protein can be produced by culturing the transformant thus obtained in a medium to produce and accumulate the condensed purine derivative-binding protein in the culture, and recovering it from the culture.

Culturing of the transformant in a medium is carried out according to the usual method used in culturing of the host.

When the transformant is obtained by using, as a host, a prokaryote such as *Escherichia coli,* or a eukaryote such as yeast, the medium for culturing the transformant may be any of a natural medium and a synthetic medium, so long as it contains a carbon source, a nitrogen source, inorganic salts and the like which can be assimilated by the transformant and the transformant can be cultured efficiently.

Any carbon source can be used, so long as the transformant can assimilate, and it includes carbohydrates, such as glucose, fructose, sucrose, molasses containing them, starch and starch hydrolysate; organic acids, such as acetic acid and propionic acid; alcohols, such as ethanol and propanol; and the like.

The nitrogen source includes ammonia, various ammonium salts of inorganic acids or organic acids, such as ammonium chloride, ammonium sulfate, ammonium acetate and ammonium phosphate; other nitrogen-containing compounds; peptone; meat extract; yeast extract; corn steep liquor, casein hydrolysate; soybean meal and soybean meal hydrolysate; various fermented cells and digested matter thereof; and the like.

The inorganic salt includes potassium dihydrogen phosphate, dipotassium hydrogen phosphate, magnesium phosphate, magnesium sulfate, sodium chloride, ferrous sulfate, manganese sulfate, copper sulfate, calcium carbonate and the like.

Culturing is usually carried out under aerobic conditions by shaking culture, submerged spinner culture under aeration or the like. The culturing temperature is preferably from 15 to 40°C, and the culturing time is generally from 16 hours to 7 days. The pH of the medium is preferably maintained at 3.0 to 9.0 during the culturing. The pH can be adjusted using inorganic or organic acid, an alkali solution, urea, calcium carbonate, ammonia or the like.

Also, if necessary, antibiotics, such as ampicillin and tetracycline, can be added to the medium during culturing.

When a microorganism transformed with an expression vector containing an inducible promoter is cultured, an inducer can be added to the medium, if necessary. For example, isopropyl-β-D-thiogalactopyranoside or the like can be added to the medium when a microorganism transformed with an expression vector containing *lac* promoter is cultured; or indoleacrylic acid or the like can be added thereto when a microorganism transformed with an expression vector containing *trp* promoter is cultured.

The medium for culturing a transformant obtained using an animal cell as the host includes generally used RPMI 1640 medium [*The Journal of the American Medical Association*, 199, 519 (1967)], Eagle's MEM medium [*Science*, 122, 501 (1952)], Dulbecco's modified MEM medium [*Virology,* 8, 396 (1959)], and 199 Medium [*Proceeding of the Society for the Biological Medicine,* 73, 1 (1950)], as well as media to which fetal calf serum or the like has been added to the above media and the like.

Culturing is generally carried out at pH 6 to 8 and at 30 to 40°C for 1 to 7 days in the presence of 5% $CO_2$ or the like.

Furthermore, if necessary, antibiotics such as kanamycin and penicillin, can be added to the medium during the culturing.

The medium for culturing a transformant obtained using an insect cell as the host includes generally used TNM-FH medium (manufactured by Pharmingen), Sf-900 II SFM (manufactured by Life Technologies), ExCell 400 and ExCell 405 (both manufactured by JRH Biosciences), Grace's Insect Medium [*Nature*, 195, 788 (1962)] and the like.

Culturing is generally carried out at pH 6 to 7 and at 25 to 30°C for 1 to 5 days or the like.

Furthermore, if necessary, antibiotics such as gentamicin can be added to the medium during the culturing.

A transformant obtained by using a plant cell as the host cell can be used as the cell or after differentiating to a plant cell or organ. The medium used in the culturing of the transformant includes Murashige and Skoog (MS) medium, White medium, media to which a plant hormone, such as auxin or cytokinine, has been added, and the like.

Culturing is carried out generally at a pH 5 to 9 and at 20 to 40°C for 3 to 60 days.

Also, antibiotics, such as kanamycin and hygromycin, can be added to the medium during the culturing, if necessary.

As described above, the condensed purine derivative-binding protein can be produced by culturing a transformant derived from a microorganism, animal cell, insect cell or plant cell containing the recombinant vector to which a DNA encoding the condensed purine derivative-binding protein is inserted according to the general culturing method to produce and accumulate the condensed purine derivative-binding protein, and recovering the condensed purine derivative-binding protein from the culture.

When expression is carried out by using a yeast, animal cell, insect cell or plant cell, a glycosylated or sugar chain-added polypeptide can be obtained.

The condensed purine derivative-binding protein, can be produced as a fusion protein conjugated with a marker peptide or the like or a secretory protein conjugated with a signal sequence according to the method described in *Molecular Cloning,* Third Edition as well as. the condensed purine derivative-binding protein as it is.

The marker peptide to be fused includes, for example, myc polypeptide, β-galactosidase, protein A, IgG binding region of protein A, chloramphenicol acetyltransferase, poly(Arg), poly(Glu), protein G, maltose-binding protein, glutathione S-transferase, polyhistidine chain (His-tag), S peptide, DNA binding protein domain, Tac antigen, thioredoxin, green fluorescent protein, FLAG peptide and epitopes of any antibodies. The signal sequence includes the signal sequence of any secretory protein [Akio Yamakawa, *Experimental Medicine,* 13, 469-474 (1995)].

Examples of the method for producing the condensed purine derivative-binding protein include a method of intracellular expression in a host cell, a method of extracellular secretion from a host cell, and a method of production on a host cell membrane outer envelope. The method can be selected by changing the host cell to be used or the protein to be produced.

When the condensed purine derivative-binding protein is produced in a host ceD or on a host cell membrane outer envelope, condensed purine derivative-binding protein can be positively secreted extracellularly in accordance with the method of Paulson *et al.* [*J. Biol. Chem.,* 264, 17619 (1989)], the method of Lowe *et al.* [*Proc. Natl. Acad. Sci. USA,* 86, 8227 (1989), *Genes Develop.,* 4, 1288 (1990)], the methods described in Japanese Published Examined Patent Application No. 336963/93 and Japanese Published Examined Patent Application No. 823021194 and the like.

Specifically, by using gene recombination technique, the condensed purine derivative-binding protein can positively be secreted extracellularly from the host cell by allowing the condensed purine derivative-binding protein in the form where a signal peptide is added before the polypeptide containing the active site of the condensed purine derivative-binding protein.

Also, its production amount can be increased in accordance with the method described in Japanese Published Examined Patent Application No. 227075/90 using a gene amplification system using a dihydrofolate reductase gene.

In addition, the condensed purine derivative-binding protein can also be produced by using a gene-introduced animal individual (transgenic non-human animal) or a plant individual (transgenic plant) which is constructed by the redifferentiation of an animal or plant cell into which the gene is introduced.

When the transformant is an animal individual or a plant individual, condensed purine derivative-binding protein can be produced in accordance with a general method by rearing or cultivating it to thereby produce and accumulate the protein and then recovering the protein from the animal or plant individual.

Examples of the method for producing the condensed purine derivative-binding protein using an animal individual include a method in which the condensed purine derivative-binding protein is produced in an animal constructed by introducing a gene in accordance with a known method [*American Journal of Clinical Nutrition*, 63, 627S (1996); *Bio/Technology,* 9, 830 (1991)].

In the case of an animal individual, condensed purine derivative-binding protein can be produced by rearing a transgenic non-human animal into which a DNA encoding condensed purine derivative-binding protein is introduced to thereby produce and accumulate the protein in the animal, and then recovering the protein from the animal. Examples of the place of the animal where the composition is produced and accumulated include milk (Japanese Published Examined Patent Application No. 309192/88) and eggs of the animal. As the promoter used in this case, any promoter

can be used, so long as it can function in an animal. Preferred examples include mammary gland cell-specific promoters such as α casein promoter, β casein promoter, β lactoglobulin promoter, whey acidic protein promoter and the like.

Example of the method for producing the condensed purine derivative-binding protein using a plant individual include a method in which condensed purine derivative-binding protein is produced by cultivating a transgenic plant into which a DNA encoding condensed purine derivative-binding protein is introduced by a known method [*Tissue Culture,* 20 (1994); *Tissue Culture,* 21 (1995); *Trends in Biotechnology,* 15, 45 (1997)] to produce and accumulate the condensed purine derivative-binding protein, and then recovering the condensed purine derivative-binding protein from the plant.

In order to isolate and purify the condensed purine derivative-binding protein prepared from the transformant, general methods for enzyme isolation and purification can be used. For example, when the condensed purine derivative-binding protein is intracellularly expressed in a dissolved state, the cells after culturing are recovered by centrifugation, suspended in an aqueous buffer and then disrupted using an ultrasonic oscillator, a French press, a Manton Gaulin homogenizer, a dynomill or the like to obtain a cell-free extract. A purified preparation of the condensed purine derivative-binding protein can be obtained from a supernatant obtained by centrifuging the cell-free extract according to a general enzyme isolation purification technique such as solvent extraction, salting out, desalting with ammonium sulfate or the like, precipitation using an organic solvent, anion exchange chromatography using a resin such as diethylaminoethyl (DEAE)-Sepharose and DIAION HPA-75 (manufactured by Mitsubishi Chemical), cation exchange chromatography using a resin such as S-Sepharose FF (manufactured by Pharmacia), hydrophobic chromatography using a resin such as butyl-Sepharose and phenyl-Sepharose, gel filtration using a molecular sieve, affinity chromatography, chromatofocusing, electrophoresis such as isoelectric focusing, and the like which may be used alone or in combination thereof.

Also, when the condensed purine derivative-binding protein is expressed intracellularly by forming an insoluble body, the cells are recovered, disrupted and centrifuged in the same manner, and the insoluble body of the condensed purine derivative-binding protein is recovered as a precipitation faction. The recovered insoluble body of the condensed purine derivative-binding protein is solubilized using a protein denaturing agent. The condensed purine derivative-binding protein is made into a normal three-dimensional structure by diluting or dialyzing the solubilized solution, and then a purified preparation of the condensed purine derivative-binding protein is obtained by the same isolation purification method.

When the condensed purine derivative-binding protein or a derivative of the protein in which a sugar chain is added to the condensed purine derivative-binding protein is secreted extracellularly, the protein or the derivative thereof can be recovered from the culture supernatant. That is, the culture, is treated by a technique such as centrifugation to obtain a culture supernatant, and a purified preparation can be obtained From the soluble fraction by the same isolation purification method.

Furthermore, the condensed purine derivative-binding protein can be produced by using a DNA encoding the condensed purine derivative-binding protein under *in vitro* transcription/translation system.

As the *in vitro* transcription/translation system, an *in vitro* transcription/translation system can be used according to a known method [Kigawa, *et al., J. Biomol. NMR,* 6, 129 (1998); Spirin A.S., *et al., Science,* 242, 1162 (1988); Kigawa T. & Yokoyama S., *J. Biochem.,* 110, 166 (1991)]. Specifically, DNA encoding the condensed purine derivative-binding protein is conjugated to the downstream of a promoter such as SP6, T7 and T3, the promoter is allowed to react with an RNA polymerase specific to each promoter to thereby prepare a large amount of the condensed purine derivative-binding protein RNA *in vitro.* The, by using a translation system of a cell-free system, for example, a translation system using rabbit reticulocyte lysate, wheat germ extract solution or *Escherichia coli* extract solution, the condensed purine derivative-binding protein can be produced. By the same isolation and purification methods as described above, a purified preparation can be obtained from the reaction solution.

Additionally, the condensed purine derivative-binding protein used in the screening method of the present invention can be produced by chemical synthetic methods such as Fmoc method (fluorenylmethyloxycarbonyl method) and tBoc method (*t*-butyloxycarbonyl method). Moreover, the condensed purine derivative-binding protein can be chemically synthesized by a peptide synthesizer manufactured by Advanced ChemTech, PerkinElmer, Pharmacia, Protein Technology Instrument, SynthecellVega Biomolecules Corporation, PerSeptive or Shimadzu Corporation.

The condensed purine derivative-binding protein includes, for example, a protein comprising the amino acid sequence represented by any one of SEQ ID NOs:1 to 3.

[3] Screening method of a substance having anti-diabetic activity by using the condensed purine derivative and the condensed purine derivative-binding protein

The substance having anti-diabetic activity can be obtained by

(i) bringing the condensed purine derivative into contact with a condensed purine derivative-binding protein in the

presence of a tested substance to assay the binding amount of the condensed purine derivative to the protein,
(ii) bringing the condensed purine derivative into contact with the condensed purine derivative-binding protein in the absence of the tested substance to assay the binding amount of the condensed purine derivative to the protein,
(iii) comparing the assayed amount in the presence of the tested substance with that in the absence of the tested substance, and
(iv) selecting a substance which inhibits the binding of the condensed purine derivative to the protein from the tested substances.

The condensed purine derivative used in the above-described method includes the compound in the above item 1 which is produced by the method in the above item 2, while the condensed purine derivative-binding protein includes the protein in the above item 4[1] which is produced by the method in the above item 4[2]. Specifically, the protein includes proteins (i) to (iii) described in the item above item 4[1](5).

The method for assaying the amount of the condensed purine derivative bound to the condensed purine derivative-binding protein includes, for example, a method in which either one molecule of the condensed purine derivative and the condensed purine derivative-binding protein is immobilized on a solid phase such as a multi-well plate, a chromatographic carrier, a bead such as latex, a chip for surface plasmon resonance or a target for mass spectrometry through physical adsorption, a covalent bond or binding via an antibody which reacts with the condensed purine derivative-binding protein or a molecule which specifically recognizes a peptide selected from a tag peptide, a maker peptide and a signal peptide, the resulting solid phase is treated for blocking or the like to suppress non-specific adsorption if necessary, the other molecule is added, the other molecule non-adsorbed is washed and removed, and the condensed purine derivative-binding protein as the other molecule or the bound other molecule is detected based on the activity of the condensed purine derivative-binding protein, the binding profile thereof, the occurrence of surface plasmon resonance, or the signal intensity by mass spectrometry to thereby assay a binding amount of the condensed purine derivative and the condensed purine derivative-binding protein.

A substance having anti-diabetic activity can be selected by comparing the amount of the condensed purine derivative bound to the condensed purine derivative-binding protein in the presence of a tested substance with the amount thereof in the absence of the tested substance, using any one of the methods described above and using the inhibitory activity of the tested substance on the binding between the condensed purine derivative and the condensed purine derivative-binding protein as a marker.

It can be confirmed by the promotion of insulin secretion from a pancreatic β cell by the substance in bringing the pancreatic β cell or a tissue containing the cell into contact with the substance selected above in the presence of glucose that the substance is a substance having anti-diabetic activity, for example, insulin secretion-promoting activity [*Diabetorogia,* 36, 1139 (1993)].

[4] Screening method of a substance capable of binding to a condensed purine-binding protein by using the condensed purine derivative and a pancreatic β cell or a treated product of the cell

A substance binding to the condensed purine derivative-binding protein can be obtained by

(i) bringing the condensed purine derivative into contact with a pancreatic β cell or a treated product of the cell in the presence of a tested substance to assay the binding amount of the condensed purine derivative to pancreatic β cell or the treated product of the cell,
(ii) bringing the condensed purine derivative into contact with a pancreatic β cell or a treated product of the cell in the absence of the tested substance to assay the binding amount of the condensed purine derivative to pancreatic β cell or the treated product of the cell,
(iii) comparing the assayed amount in the presence of the tested substance with that in the absence of the tested substance,
(iv) selecting a substance suppressing the binding of the condensed purine derivative to pancreatic β cell or a treated product of the cell from the tested substances, and
(v) selecting a substance binding to the condensed purine derivative-binding protein from the substances obtained in the step (v).

The steps (i) to (iv) can be carried out according to the method in the above item 3. The method for selecting a substance capable of binding to the condensed purine derivative-binding protein from substances selected in the steps (i) to (iv) includes a method which comprises assaying and comparing the binding activity of the substance preliminarily labeled in bringing the substance labeled into contact with a pancreatic β cell or a treated product of the cell and the binding activity thereof in bringing the labeled substance into contact with a cell except a pancreas-derived cell or tissue or a treated product of the cell or tissue; based on the label, and selecting a substance having high binding

activity to a pancreatic β cell or a treated product of the cell. The binding activity can be assayed according to the method of the above item 3[3].

[5] Screening method of a substance capable of binding to the condensed purine derivative-binding protein by using the condensed purine derivative and the condensed purine derivative-binding protein

A substance capable of binding to the condensed purine derivative-binding protein can be obtained from substances selected at the steps in the above items 4[4](i) to (iv) according to the method in the above item 4[3] by using the condensed purine derivative-binding protein as obtained by the methods in the above items 4[1] and [2], instead of a pancreatic β cell or a treated product of the cell according to the method in the above item 4[4].
The binding of the condensed purine derivative to the condensed purine derivative-binding protein can be assayed by producing the condensed purine derivative-binding protein as a fusion protein with another protein, and specifically detecting a peptide selected from a tag peptide, a marker peptide or a signal peptide, using a substance having affinity to the protein fused. When β-galactosidase, chloramphenicol/acetyltranferase, glutathione S-transfesase or thioredoxin is used as a peptide to be fused, the binding can be detected by using the enzyme activity as a marker, when green fluorescent protein (GFP) or the like is used, the binding can be detected by using the fluorescence as a marker; when protein A, the IgG binding region of protein A, poly(Arg), poly(Glu), protein G, maltose-bound protein, polyhistidine chain (His-tag), a DNA binding polypeptide domain or the like is used, the binding can be detected by using the binding to its specific binding molecule as a marker; and when myc polypeptide, S peptide, Tac antigen, FLAG peptide, an epitope of any antibody or the like are used, the binding can be detected by using the binding to its specific antibody as a marker.
β-Galactosidase, chloramphenicol/acetyltransferase, glutathione S-transferase, thioredoxin, green fluorescent protein (GFP), protein A, the IgG binding region of protein A, poly(Arg), poly(Glu), protein G, maltose-bound protein, polyhistidine chain (His-tag), DNA binding protein domain or the like can also be detected by using the binding to its specific antibody as a marker.
Furthermore, by modifying either one or both of the condensed purine derivative-binding protein and the condensed purine derivative with a molecule having specificity to binding to biotin or the like, a fluorescent reagent, an RI labeling reagent or the like and then using the resulting modified protein or the modified condensed purine derivative in the reaction system, the binding specificity to biotin or the like can be used for binding to a solid phase or the interaction can be detected by using the binding specificity to biotin or the like, fluorescence, radioactivity or the like as a marker.
Labeling the protein and the polypeptide with an appropriate fluorescent dye and utilizing fluorescence emitted only when the condensed purine derivative-binding protein and the condensed purine derivative bind to each other by the fluorescent resonance energy transfer (FRET), the interaction between the condensed purine derivative binding protein and the condensed purine derivative can be detected in a solution system.

[6] Screening method of a substance which is capable of changing the activity of a protein capable of binding the condensed purine derivative by using the condensed purine derivative and the condensed purine derivative-binding protein

A substance which is capable of changing the activity of a protein capable of binding to the condensed purine derivative can be obtained by

(i) bringing a tested substance into contact with a condensed purine derivative-binding protein to assay the activity of the protein,
(ii) assaying the activity of the protein which is out of contact with the tested substance,
(iii) comparing the assayed amount in the presence of the tested substance with that in the absence of the tested substance, and
(iv) selecting a substance which is capable of changing the activity of the protein from the tested substances.

The condensed purine derivative for use in the method above includes the compound in the above item 1 produced by the method in the above item 2. The condensed purine derivative-binding protein includes the protein in the above item 4[1] produced by the method in the above item 4[2].
When the activity of the protein is known, the activity of the enzyme can be assayed according to a known method.
For example, when a protein comprising the amino acid sequence represented by SFQ ID NO:1 is used as the protein, the activity can be assayed according to the method described in *Biochem. Biophys. Acta,* 1338, 47 (1977).
Specifically, by using NADP, NADPH, or alcohol or aldehyde such as retinol, retinal and p-chlorobenzaldehyde

[*Biochimica et Biophysica Acta,* 1337, 47 (1997)] which is capable of being a substrate, the elimination rate of the substrate or generation rate of a product by the protein can be monitored by spectrometry, fluorometry or high performance liquid chromatography to thereby assay the activity of the protein.

[7] Screening method of a substance which is capable of suppressing the activity of the condensed purine derivative-binding protein by using the condensed purine derivative and the condensed purine derivative-binding protein

A substance which is capable of suppressing the activity of the condensed purine derivative-binding protein can be selected as a substance which reduces the activity of the protein among substances which are capable of modifying the activity of the condensed purine derivative-binding protein obtained by the method in the above item [6].

Also, a substance which is capable: of suppressing the activity of the condensed purine derivative-binding protein can be obtained by preparing the condensed purine derivative-binding protein and a substrate derivative of the condensed purine derivative-binding protein, and adding a tested substance to a system for detecting the binding of both to select a compound which is capable of suppressing the binding of the condensed purine derivative-binding protein and the substrate derivative of the condensed purine derivative-binding protein by using the inhibition of the binding by the tested substance as a market.

Specifically, a substance which is capable of suppressing the activity of the condensed purine derivative-binding protein can be selected among compounds which are capable of binding to the condensed purine derivative-binding protein selected by the method in the above item [3].

Additionally, a substance which is capable of suppressing the activity of a condensed purine derivative-binding protein can be selected by

(i) assaying the binding of the condensed purine derivative-binding protein to a substrate derivative of the condensed purine derivative-binding protein in the presence of a tested substance,
(ii) assaying the binding of the protein to the substrate derivative of the protein in the absence of the tested substance,
(iii) comparing the assayed amount in the presence of the tested substance with that in the absence of the tested substance, and
(iv) selecting a substance which is capable of suppressing the binding of the protein to the substrate derivative of the protein from the tested substances.

Herein, the substrate derivative of the condensed purine derivative-binding protein means a substance derived from substances which are capable of being substrates of the condensed purine derivative-binding protein. Specifically, for example, when a protein comprising the amino acid sequence represented by SEQ ID NO:1 is used as the condensed purine derivative-binding protein, the protein substrate derivative specifically includes derivatives of substances which are capable of being substrates of the protein, such as derivatives of NADP and NADPH, and derivatives of alcohol or aldehyde including retinol, retinal, p-chlorobenzaldehyde and the like [*Biochimica et Biophysica Acta,* 1338, 47 (1997)].

A more specific method includes a method in which either one molecule of the condensed purine derivative-binding protein or a substrate derivative of the condensed purine derivative-binding protein is immobilized on a solid phase such as a multi-well plate, a chromatographic carrier, a bead such as latex, a chip for measuring surface plasmon resonance or a target for mass spectrometry through physical adsorption, a covalent bond or binding via an antibody which reacts with the condensed purine derivative-binding protein or a molecule which specifically recognizes a peptide selected from a tag peptide, a maker peptide and a signal peptide, the resulting solid phase is treated for blocking to suppress non-specific adsorption if necessary, the other molecule is added, the other molecule non-adsorbed is washed and removed, and the other molecule bound is detected based on the activity of the condensed purine derivative-binding protein, the binding profile thereof, the occurrence of surface plasmon resonance, or the signal intensity by mass spectrometry is detected to assay the amount of the condensed purine derivative-binding protein bound to the substrate derivative of the condensed purine derivative-binding protein.

By a combination of two or more methods selected from any methods of the above items [5] to [7], a compound having preferable activity as an insulin secretion-promoting agent can be screened among substances selected by the methods in the above items [5] to [7]. If necessary, a compound having preferable activity as an insulin secretion-promoting agent can be screened by the method in the following item [11].

The compound having preferable activity as an insulin secretion-promoting agent means a compound having low activity unpreferable as an insulin secretion-promoting agent and includes, for example, a compound having reduced side effects of known insulin promoters, for example body weight loss, bone marrow suppression, liver disorders, renal toxicity and nerve toxicity.

[8] Screening method of a substance which is capable of changing the function of the condensed purine derivative-binding protein by using the condensed purine derivative and the condensed purine derivative-binding protein

A substance which is capable of changing the function of the condensed purine derivative-binding protein can be obtained by

(i) bringing a tested substance into contact with a transformant which expresses the condensed purine derivative-binding protein to assay the cell response of the transformant,
(ii) assaying the cell response of the transformant in the absence of the tested substance,
(iii) comparing the assayed amount in the presence of the tested substance with that in the absence of the tested substance, and
(iv) selecting a substance which is capable of changing the cell response of the transformant from the tested substances.

The transformant which expresses the condensed purine derivative-binding protein can be prepared by the method in the above item 4[2](2).
The cell response of the transformant when a tested substance and the transformant are brought into contact with each other includes, for example, change in intracellular information transmission, gene transcription, sugar uptake, growth and the like. These changes can be detected by known methods.
More specific cell response includes arachidonic acid release, acetylcholine release, intracellular $Ca^{2+}$ release, intracellular cAMP generation, intracellular cGMP generation, inositol phosphoric acid generation, the change of the potential of cell membrane, the phosphorylation of intracellular protein, c-fos activation, pH reduction, the aggregation or dispersion of melanin dye, the promotion of insulin secretion and the like.

[9] Screening method of a substance which is capable of changing the expression level of a gene encoding the condensed purine derivative-binding protein by using the condensed purine derivative and the condensed purine derivative-binding protein

A substance which is capable of changing the expression level of a gene encoding the condensed purine derivative-binding protein can be obtained by

(i) bringing a tested substance into contact with a transformant which expresses the condensed purine derivative-binding protein to assay the expression level of the gene encoding the protein in the transformant,
(ii) assaying the expression level of the gene encoding the protein in the transformant in the absence of the tested substance,
(iii) comparing the assayed amount in the presence of the tested substance with that in the absence of the tested substance, and
(iv) selecting a substance which is capable of changing the expression level of the gene encoding the protein from the tested substances.

A specific method comprises preparing a total RNA or poly$(A)^+$ RNA from each of transformant cells prepared by the above method in the above item 4[2](2) according to general methods, which express the condensed purine derivative-binding protein in contact with a tested substance or in no contact with the tested substance, assaying the expression level of the gene encoding the condensed purine derivative-binding protein by RT-PCR [*PCR Protocols,* Academic Press (1990)] using primer DNAs prepared from the DNA encoding the condensed purine derivative-binding protein, and comparing each of the expression levels to thereby obtain a substance which is capable of changing the expression level of the gene encoding the condensed purine derivative-binding protein.

[10] Screening method of a substance which is capable of changing the expression level of the condensed purine derivative-binding protein by using the condensed purine derivative and the condensed purine derivative-binding protein

A substance which is capable of changing the expression level of the condensed purine derivative-binding protein can be obtained by

(i) bringing a tested substance into contact with a transformant which expresses the condensed purine derivative-binding protein to assay the expression level of the protein in the transformant,
(ii) assaying the expression level of the protein in the transformant in the absence of the tested substance,

(iii) comparing the assayed amount in the presence of the tested substance with that in the absence of the tested substance, and
(iv) selecting a substance which is capable of changing the expression level of the protein from the tested substances.

The transformant which expresses the condensed purine derivative-binding protein can be prepared by the method in the above item 4[2](2).
The method for assaying the expression level of the condensed purine derivative-binding protein includes immunological assay methods.
The immunological assay methods include, for example, sandwich ELISA using two types of monoclonal antibodies having different epitopes, among antibodies reacting with the condensed purine derivative-binding protein in a liquid phase, and radioimmunoassay using the condensed purine derivative-binding protein as preliminarily labeled with radioactive isomers such as $^{125}I$ and an antibody recognizing the protein.
The antibody reacting with the condensed purine derivative-binding protein can be prepared as a polyclonal antibody, a monoclonal antibody or the like, by a known method using the protein and partially purified peptides of the protein.

[11] Evaluation of insulin secretion-promoting activity

As the evaluation system of an insulin secretion-promoting agent, any known method can be used.
The method using an animal includes,, for example, a method comprising carrying out an oral glucose test using Wistar rats during the administration of a tested substance and selecting a tested substance which has an effect of suppressing blood glucose but does not cause hypoglycemia (WO00/01388).
The method using resected tissues or culture cells includes, for example, selecting a tested substance promoting insulin secretion at such a level that the tested substance does not promote insulin secretion when the tested substance is added at a low glucose concentration but promotes insulin secretion when the tested substance is added at a high glucose concentration than the level caused singly by a high glucose concentration, using BRIN-BD11 cell [*Diabetes*, 45, 1132-1140 (1996)], INS-1 cell [*Endocrinology*, 130, 167-178 (1992)], βTC cell [*Proc. Natl. Acad. Sci. USA,* 85, 9037-9041 (1988)] or MIN6 cell [*Endocrinology*, 127, 126-132 (1990)] as cultured β cells having insulin secretion potencies depending on the physiological change of glucose concentration of 5 to 10 mmol/l, among pancreatic Langerhans islands, primary culture of pancreatic β cell, and cultured β cell.
Among the cultured β cell lines, pancreatic β cell MIN6 cell [*Endocrinology,* 127, 126-132 (1990)] preserves the properties of pancreatic β cells in biological organisms, in light of that the insulin content therein and the insulin secretion amount via glucose stimulation are close to those in biological pancreatic β cells so that the MIN6 cell makes a response to glucose concentration to raise the insulin secretion [*Endocrinology,* 127, 126-132- (1990); *Diabetologia*, 36, 139-1145 (1993)]. Additionally, the MIN6 cell is more preferred since the MIN6 cell responds to sulfonylurea for use as a therapeutic diabetic agent such as glibenclamide to promote the insulin secretion [*Cellular Signaling,* 5, 777-786 (1993)].
Culturing of the MIN6 cell and insulin secretion test using MIN6 cell can be carried out according to the method described in *Diabetologia,* 36, 1139-1145 (1993).
Additionally, the influence of a compound on the insulin secretion activity of the cell can be determined by assaying the insulin amount in the cell culture supernatants collected as follows.
MIN6 cell cultured in a 24-well plate is washed twice with 1 ml of buffer A containing 2 mmol/l glucose [119 mmol/l sodium chloride, 4.74 mmol/l potassium chloride, 2.54 mmol/l calcium chloride, 1.19 mmol/l magnesium sulfate, 1.19 mmol/l potassium dihydrogen phosphate, 10 mmol/l 2-[4-(2-hydroxylethyl)-1-piperadinyl]ethanesulfonic acid, 0.1% bovine serum albumin, pH 7.3], and is then incubated in 1 ml of the buffer A containing 2 mmol/l glucose at 37°C for 45 minutes. After incubation, the culture supernatant is exchanged with various concentrations of a test compound and buffer A (1 ml) containing 8.4 mmol/l glucose, for additional incubation at 37°C for 45 minutes, to collect the culture supernatant. The antibody-reactive insulin secreted in the culture supernatant is diluted with a phosphate buffer containing 1% bovine serum albumin, 0.1% Tween 20, and 0.12% EDTA 2Na, and 0.1% sodium azide, for assaying by radioimmunoassay method. Preparing a standard curve using human insulin as a standard substance, individual insulin values can be calculated on the basis of the curve. By defining the insulin value obtained when dimethylsulfoxide as a solvent is added instead of a test compound as 100%, the relative percentage (%) of each binding amount can be calculated.

[12] Compound having insulin secretion-promoting activity as selected by the method of the present invention

The compound having insulin secretion-promoting activity as selected by the method of the present invention

includes a compound represented by the following formula (V):

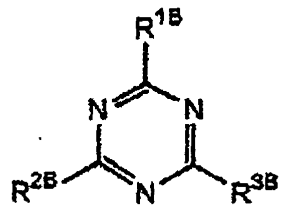

(V)

[wherein $R^{1B}$, $R^{2B}$ and $R^{3B}$ are the same or different and each represents amino, mono- or di-substituted amino, or a substituted or unsubstituted heterocyclic group containing N (wherein the heterocyclic group binds to the triazine ring via the N atom)].

The heterocyclic group binding to the triazine ring via the N atom includes, for example, pyrrolyl, imidazolyl pirazolyl, triazolyl, tetrazolyl indolyl, indazolyl, benzoimidazolyl, purinyl, pyrrolidinyl, 2,5-dioxopyrrolidinyl, thiazolinidyl, oxazolidinyl, 2-oxooxazolidinyl, piperidinyl, piperazinyl, homopiperazinyl, morpholinyl, thiomorpholinyl, tetrahydroquinolyl, tetrahydroisoquinolyl, tetrahydroquinoxalyl, octahydroquinolyl, dihydroindolyl, 1,3-dioxoisoindolynyl and the like.

In the definition of each of the groups in formula (V), substitution means substitution with, for example, substituted or unsubstituted lower alkyl, substituted or unsubstituted aralkyl, substituted or unsubstituted aryl, a substituted or unsubstituted aromatic heterocyclic group, or a substituted or unsubstituted alicyclic heterocyclic group.

Herein, the substituted or unsubstituted lower alkyl, the substituted or unsubstituted aralkyl, the substituted or unsubstituted aryl, the substituted or unsubstituted aromatic heterocyclic group, and the substituted or unsubstituted alicyclic heterocyclic group have the same meanings as the substituted or unsubstituted lower alkyl, the substituted or unsubstituted aralkyl, the substituted or unsubstituted aryl, the substituted or unsubstituted aromatic heterocyclic group, and the substituted or unsubstituted alicyclic heterocyclic group have in the definitions in formula (II).

More specifically, the compound includes compounds represented by Compound Nos. 193 to 200 in Table 2 shown below.

Table 2

| Compound No. | Structure | Compound No. | Structure |
|---|---|---|---|
| 193 | 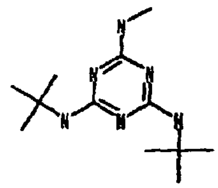 | 197 | 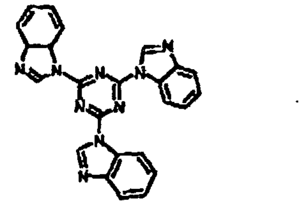 |
| 194 | 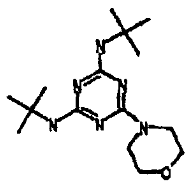 | 198 | 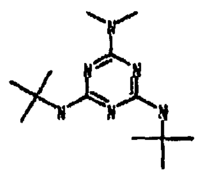 |
| 195 | 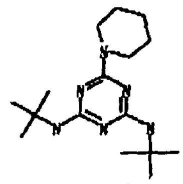 | 199 | 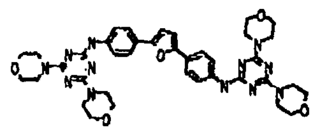 |
| 196 | 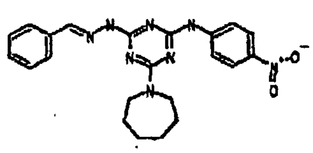 | 200 | 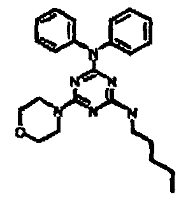 |

The above compounds can be synthesized according to the method described in WO01/47921 or a known general synthetic method and can also be purchased from ChemBrige.

[13] Medicament comprising the substance selected by the method of the present invention

The medicament comprising the substance selected by the method of the present invention as an active ingredient can be administered as the active ingredient alone, but generally, it is preferable to provide it as a pharmaceutical formulation produced by an appropriate method well known in the technical field of manufacturing pharmacy, by mixing it with at least one pharmaceutically acceptable carrier. Preferably, a sterile solution dissolved in water or an aqueous carrier such as an aqueous solution of a salt, glycine, glucose, human albumin or the like is used. Also, a pharmaceutically acceptable additive, including a buffering agent or an isotonizing agent which leads the formulation solution to physiological conditions, such as sodium acetate, sodium chloride, sodium lactate, potassium chloride and sodium citrate. Furthermore, the medicament can be stored by freeze-drying and be dissolved in an appropriate solvent when it is used.

It is desirable to select a route of administration which is most effective in treatment. Examples include oral administration and parenteral administration, such as buccal, tracheal, rectal, subcutaneous, intramuscular, intravenous or the like. The dosage form includes sprays, capsules, tablets, granules, syrups, emulsions, suppositories, injections, ointments, tapes and the like. Examples of the pharmaceutical preparation suitable for oral administration include emulsions, syrups, capsules, tablets, powders, granules and the like. For example, liquid preparations, such as emulsions and syrups, can be produced by using, as additives, water; saccharides such as sucrose, sorbitol and fructose; glycols such as polyethylene glycol and propylene glycol; oils such as sesame oil, olive oil and soybean oil; antiseptics such as p-hydroxylbenzoic acid esters; flavors such as strawberry flavor and peppermint; and the like. Capsules, tablets, powders, granules and the like can be produced using, as additive, fillers such as lactose, glucose,

sucrose and mannitol; disintegrating agents such as starch and sodium alginate; lubricants such as magnesium stearate and talc; binders such as polyvinyl alcohol, hydroxylpropylcellulose and gelatin; surfactants such as fatty acid ester; plasticizers such as glycerine; and the like.

Examples of the pharmaceutical preparation suitable for parenteral administration include injections, suppositories, sprays and the like. Injections may be prepared using a carrier, such as a salt solution, a glucose solution, a mixture of both thereof or the like. Suppositories may be prepared using a carrier such as cacao butter, hydrogenated fat, carboxylic acid or the like. Also, sprays may be prepared using the antibody composition as such or using a carrier which does not stimulate the buccal or airway mucous membrane of the patient and can facilitate absorption of the antibody composition by dispersing it as fine particles. Examples of the carrier include lactose, glycerol and the like. Depending on the properties of the antibody composition and the carrier, it is possible to produce pharmaceutical preparations such as aerosols, dry powders and the like. In addition, the components exemplified as additives for oral preparations can also be added to the parenteral preparations.

Although the clinical dose or the frequency of administration varies depending on the objective therapeutic effect, administration method, treating period, age, body weight and the like, it is usually 10 µg/kg to 8 g/kg per day and per adult.

BRIEF DESCRIPTION OF THE DRAWINGS

Fig. 1A is a graph showing the saturation curve of the binding with a $^3$H-labeled compound represented by Compound No. 16 when pancreatic β cell was used. In the figure, ○-○ represents the total binding amount; circle ○---○ circle represents the non-specific binding amount and ●-● represents the specific binding amount.

Fig. 1B is the Scatchard plot of the A above.

Fig. 2 shows the correlation between the inhibitory activity of the condensed purine derivative on the specific binding of the $^3$H-labeled compound represented by Compound No. 16 when pancreatic β cell was used and the insulin secretion-promoting activity.

Fig. 3A is a graph showing the saturation curve of the binding with the $^3$H-labeled compound represented by Compound No. 16 when a cell membrane fraction of pancreatic β cell was used. In the figure, ▲-▲ represents the total binding amount; ○--○ represents the non-specific binding amount and ●-● represents the specific binding amount.

Fig. 3B is the Scatchard plot of the A above.

Fig. 4 shows the correlation between the inhibitory activity of the condensed purine derivative on the specific binding of the $^3$H-labeled compound represented by Compound No. 16 when a cell membrane fraction of pancreatic β cell was used and the insulin secretion-promoting activity. Numerical figures attached on the graphs represent compound No. 95* expresses the tartrate salt of Compound No. 95.

Fig. 5 is a graph showing the binding of a $^3$H-labeled compound represented by Compound No. 16 when a solubilized membrane fraction of pancreatic β cell was used.

Fig. 6 shows the chart of the results of the SDS-PAGE analysis of recombinant His-p31 using 9% acrylamide geL Lane 1 shows purified recombinant His-p31; and Lane M shows a molecular marker by electrophoresis. The arrow shows the position of the recombinant His-p31 protein.

Fig. 7 shows a graph showing the correlation between the inhibitory activity of each compound on the binding between the His-p31 protein and a compound represented by Compound No. 155 (hereinafter referred to as "Compound 155"), as calculated using BIAcore of each compound and the activity promoting insulin secretion. Numerical figures attached on the plots on the graphs represent compound No.

Fig. 8 shows the screening of an insulin secretion-promoting agent, using a cell extract solution of MIN6 cell and the condensed purine derivative-immobilized particle. SDS-PAGE on 9% acrylamide gel is shown. Lane 1 shows a protein purified from the MIN6 cell extract solution, using a particle without immobilized Compound 155; Lane 2 shows the p31 protein purified from the MIN6 cell extract solution, using a particle with immobilized Compound 155; Lane 3 shows a protein purified from the MIN6 cell extract solution preliminarily treated with a compound represented by Compound No. 16, using a particle with immobilized Compound 155; Lane 4 shows a protein purified from the MIN6 cell extract solution preliminarily treated with a compound represented by Compound No. 17, using a particle with immobilized Compound 155; and Lane M shows a molecular marker by electrophoresis.

The arrow shows the position of the p31 protein.

BEST MODE FOR CARRYING OUT THE INVENTION

The present invention is described below in Examples and Test Examples. However, the present invention is

not limited to these examples.

Example 1

Synthesis of labeled condensed purine derivative:

The $^3H$-labeled Compound 16 represented by the following formula (VI) (hereinafter referred to as "[$^3H$]-labeled Compound 16") was synthesized by Amersham Pharmacia by the $^3H$-gas method using the R form of a compound represented by Compound No. 23 in Table 1 above, which can be synthesized by the method described in WO00/01388:

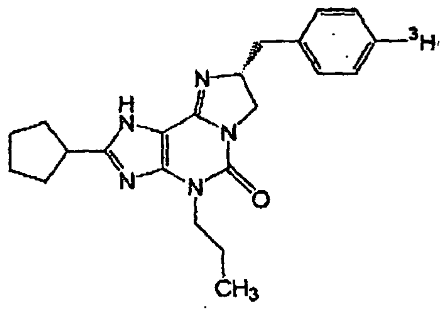

(VI)

Example 2

Preparation of solution of disrupted pancreatic β cell:

MIN6 cell [*Endocrinology,* 127, 126-132 (1990)] was cultured in a DMEM culture medium (Dulbecco's Modified Eagle Medium; manufactured by Nissui Pharmaceutical.) containing 15% fetal bovine serum and 5 g/l glucose in 5% $CO_2$ at 37°C in a cell culture flask (182 $cm^2$; manufactured by Gliner) to reach confluence. The following procedures were carried out on ice.

After the MIN6 cell reached confluence, the culture medium was removed. Then, the cell was washed twice with 30 ml of ice-cold phosphate buffered physiological saline (PBS). Subsequently, 3 ml of ice-cold Tris-HCl buffer [10 mmol/l Tris-HCl, 100 mmol/l sodium chloride (NaCl), 2 mmol/l ethylenediaminetetraacetic acid (EDTA), 10 μg/ml leupeptin, 1 mmol/l phenylmethanesulfonyl fluoride (PMSF), pH 7.4] was added. Using a cell scraper, the cell attached on the flask was scraped and recovered with a Pasteur pipette. The procedure was carried out once more to obtain a cell suspension of 6 ml in total per 182-$cm^2$ flask. After the cell in the cell suspension was disrupted using a homogenizer, the resulting solution was centrifuged using a centrifuge Himac CF7D2 (manufactured by Hitachi, Ltd.) at 3,500 rpm at 4°C for 10 minutes to recover the supernatant, which was used as a solution of disrupted cell.

Example 3

Preparation of membrane fraction of pancreatic β cell:

Using a centrifuge Himac CP100α (manufactured by Hitachi, Ltd.) with a rotor RP50T (manufactured by Hitachi, Ltd.), the solution of disrupted cell as obtained in Example 2 was centrifuged at 35,000 rpm at 4°C for 30 minutes. The supernatant was discarded. The Tris-HCl buffer cooled on ice was added to the remaining precipitate, which was then suspended with a syringe having an 18-gauge (manufactured by Terumo) injection needle (manufactured by Terumo). Furthermore, the resulting suspension was centrifuged by using a centrifuge Himac CP100α and a rotor RP50T at 35,000 rpm at 4°C for 30 minutes. The resulting supernatant was discarded. An appropriate amount of ice-cold Tris-HCl buffer was added to the remaining precipitate, which was then suspended with a syringe having a 25-gauge (manufactured by Terumo) injection needle (manufactured by Terumo). The resulting suspension was defined as the membrane fraction of MIN6 cell.

Example 4

Preparation of solubilized membrane fraction of pancreatic β cell:

To the membrane fraction of Example 3, 6 ml of an ice-cold solubilization buffer [20 mmol/l Tris-HCl, 500 mmol/l potassium chloride (KCl), 2 mmol/l EDTA, 10 μg/ml leupeptin, 1 mmol/l PMSF, 1 w/v% digitonin, pH 7.4] was added to suspend the membrane fraction, which was then homogenized by using a homogenizer and left to stand on ice for 30 minutes. Subsequently, the supernatant obtained by centrifuging the homogenate using the centrifuge Himac CP100α and a rotor RP50T at 35,000 rpm at 4°C for 60 minutes was recovered as a solubilized membrane fraction. To the resulting precipitate, 3 ml of the ice-cold solubilization buffer was again added. The above procedures were further carried out twice. The resulting supernatants were all mixed together and defined as solubilized membrane fraction.

Example 5

Binding experiment using pancreatic β cell and labeled condensed purine derivative:

MIN6 cell cultured to almost confluence in a 24-well plate for cell culture (manufactured by Corning) was washed twice with KRH buffer (119 mmol/l NaCl, 4.74 mmol/l KCl, 2.54 mmol/l calcium chloride ($CaCl_2$), 1.19 mmol/l magnesium sulfate ($MgSO_4$), 1.19 mmol/l potassium dihydrogen phosphate ($KH_2PO_4$), 10 mmol/l HEPES, 2 mmol/l glucose, 0.1 w/v% bovine serum albumin (BSA), pH 7.3). Subsequently, the KRH buffer was added at 1 ml per well, for preincubation at 37°C for 45 minutes. After preincubation, the supernatant was discarded, and 0.15 ml of the KRH buffer, 10 mmol/l [$^3$H]-labeled Compound 16 and a test compound to a final concentration of 1 μmol/l were added for incubation at 4°C for one hour or longer until the reaction reached equilibrium. Subsequently, the cell was washed twice using the KRH buffer. The KRH buffer was added at 2 ml per well, which was then left to stand at room temperature for 20. minutes. The cell was again washed twice by using the KRH buffer. The radioactivity remaining in each well was counted with a liquid scintillation counter to determine the binding amount.

The difference between the binding amount of the [$^3$H]-labeled Compound 16 (total binding amount) under conditions with no addition of any test compound and the binding amount of the [$^3$H]-labeled Compound 16 (non-specific binding amount) under conditions in the presence of the Compound 16 at 10 μmol/l was defined as specific binding amount.

Fig. 1A shows the saturation curve of binding and Fig. 1B shows the Scatchard plot. These figures indicate that the use of pancreatic β cells enabled the detection of the binding specific to the condensed purine derivative. Additionally, Table 3 shows the measured values of the inhibitory activity of each of the condensed purine derivatives on the binding with the [$^3$H]-labeled Compound 16 and the activities promoting insulin secretion. Fig. 2 shows the correlation between the inhibitory activity and the insulin secretion-promoting activity. The correlation coefficient between the two activities is 0.679, which is indicated to be a significant correlation.

Table 3

| Compound No. | Binding inhibitory activity (%) | Insulin secretory activity (%) |
|---|---|---|
| 16 | 100.0 | 83.2 |
| 17 | 5.4 | 15.8 |
| 31 | 112.4 | 80.5 |
| 35 | 130.9 | 63.2 |
| 36 | 57.9 | 47.0 |
| 37 | 73.7 | 61.0 |
| 38 | 71.8 | 83.0 |
| 40 | 38.5 | 76.3 |
| 41 | 55.0 | 37.2 |
| 43 | 77.6 | 54.4 |
| 61 | 50.8 | 80.4 |
| 76 | 12.6 | 16.9 |
| 84 | 66.4 | 31.7 |
| 85 | 72.0 | 26.7 |
| 94 | 30.8 | 12.0 |

Table 3 (continued)

| Compound No. | Binding inhibitory activity (%) | Insulin secretory activity (%) |
|---|---|---|
| 95 | 81.1 | 29.1 |
| 96 | 28.9 | 28.3 |
| 97 | 19.3 | 1.5 |
| 100 | 35.1 | -3.1 |
| 116 | 7.9 | -1.7 |
| 117 | 68.6 | 43.5 |
| 138 | 60.7 | 7.8 |
| 144 | 50.5 | 28.5 |
| 146 | 65.9 | 27.0 |
| 147 | 56.8 | 45.6 |
| 175 | 49.5 | 54.7 |
| 183 | 41.6 | 22.5 |
| 184 | 78.5 | 75.0 |
| 185 | 38.4 | 30.7 |
| 186 | 76.0 | 47.1 |
| 187 | 73.5 | 85.0 |
| 188 | -7.6 | -8.0 |
| 189 | 56.4 | 44.9 |
| 190 | 57.6 | 74.3 |
| 191 | 80.6 | 62.0 |

Example 6

Binding experiment using membrane fraction of pancreatic β cell and labeled condensed purine derivative:

The Tris-HCl buffer described in Example 2 containing the membrane fraction (150 μg/ml), the [$^3$H]-labeled Compound 16 and a test compound without any radioactive label, was added to a 24-well plate for cell culture (manufactured by Coming) and incubated for one hour at room temperature. By filtration under aspiration using a harvester Filtermate 196-Unifilter 24 (manufactured by Packard), a membrane fraction was recovered on UniFilter-24GF/B filter plate (manufactured by Packard). The membrane fraction was washed four times with 1 ml of a rinse buffer (10 mmol/l Tris-HCl, 100 mmol/l NaCl, 2 mmol/l EDTA, 0.1 w/v% BSA, pH 7.4) and then dried, and Microscinti 20 (manufactured by Packard) was added thereto at 150 μl/well to count the radioactivity using Microplate Scintillation Counter Top Count (manufactured by Packard). The radioactivity without addition of the Compound 16 was defined as the total binding amount and the radioactivity was defined with addition of the Compound 16 at 10 μmol/l as the non-specific binding amount, and then the amount remaining after the non-specific binding amount was subtracted from the total binding amount was defined as the specific binding amount.

Fig. 3A shows the saturation curve of binding and Fig. 3B shows the Scatchard plot. These results indicate that the use of the membrane fraction of pancreatic β cell enabled the detection of the binding specific to the condensed purine derivative. Additionally, Fig. 4 shows the correlation between the inhibitory activity of the condensed purine derivative on the binding with the [$^3$H]-labeled Compound 16 and the insulin secretion-promoting activity. The correlation coefficient between the two activities is 0.705, which is indicated to be a significant correlation.

Example 7

Binding experiment using solubilized membrane fraction of pancreatic β cell:

Experiments were carried out by the same method as in Example 6 above, except for use of the solubilized membrane fraction at a protein concentration of 500 μg/ml. For preparing a dissolution solution to an intended concentration, the dissolution solution was diluted using a solubilization buffer with no content of any surfactant to give a 0.1 w/v% concentration of a surfactant contained in the dissolution solution. Thus, the composition of the incubation solution was 0.1 w/v% digitonin, 20 mmol/l Tris-HCl, 500 mmol/l KCl, 2 mmol/l EDTA, 10 μg/ml leupeptin, 1 mmol/l PMSF, pH 7.4.

The radioactivity without addition of a compound represented by Compound No. 16 was defined as the total

binding amount and the radioactivity with addition of the compound at 10 μmol/l was defined as the non-specific binding amount, and then the amount remaining after the non-specific binding amount was subtracted from the total binding amount was defined as the specific binding amount.

Fig. 5 shows the total binding amount and the non-specific binding amount. The results indicate that the use of the solubilized membrane fraction of pancreatic β cell enabled the detection of the binding specific to the condensed purine derivative.

Example 8

Assaying secreted insulin amount:

Culturing MIN6 cell and testing insulin secretion using MIN6 cell were carried out according to the method described in *Diabetorogia,* 36, 1139-1145 (1993).

In other words, the influence of a compound on the insulin secretion activity in the presence, of 14.5 mmol/l glucose was assessed by assaying the amount of insulin in the cell culture supernatant collected as follows.

The MIN6 cell cultured in a 24-well plate was washed twice with 1 ml of buffer A [119 mmol/l NaCl, 4.74 mmol/l KCl, 2.54 mmol/l $CaCl_2$, 1.19 mmol/l $MgSO_4$, 1.19 mmol/l $KH_2PO_4$, 10 mmol/l 2-[4-(2-hydroxylethyl)-1-piperadinyl] ethanesulfonic acid, 0.1% BSA, pH 7.3] containing 2 mmol/l glucose. Subsequently, the resulting cell was incubated in 1 ml of the buffer A containing 2 mmol/l glucose at 37°C for 45 minutes. The culture supernatant was exchanged with the buffer A (1 ml) containing test compounds of various concentrations and 8.4 mmol/l glucose and incubated at 37°C for 45 minutes to collect the culture supernatant.

Antibody-reactive insulin secreted in the culture supernatant was assayed by radioimmunoassay [*Methods in Investigative and Diagnostic Endocrinology,* Vol. 2A, 2B, North-Holland Publishing Co., Amsterdam (1973)] after the culture supernatant was diluted with phosphate buffer containing 1% BSA, 0.1% Tween 20, 0.12% EDTA 2Na, and 0.1% sodium azide. A standard curve was prepared by using human insulin as the standard substance, and individual insulin values were calculated on the basis of the curve.

Example 9

Screening method of substance having anti-diabetic activity using a culture of a microorganism as screening source

According to the method described in Example 6, a substance having anti-diabetic activity can be selected from a culture of a microorganism by using the membrane fraction of pancreatic β cells.

A microorganism isolated from soil by plate dilution method is cultured in a culture medium containing 0.5% glucose, 3.0% soluble starch, 2.0% soybean powder, 0.5% yeast extract, 0.5% corn steep liquor, 03% calcium carbonate ($CaCO_3$) (pH 7.2 before steam boiling) in a test tube with an agitator at 28°C for 4 days. After the completion of culturing, dimethylsulfoxide of the same volume as that of the culture is added for sufficient agitation, which is then separated into a solid matter and a supernatant solution with a centrifuge. To a solution containing 0.15 ml of the membrane fraction of pancreatic β cell and 10 mmol/l [$^3$H]-labeled Compound 16 as described in Example 6, 2 μl of the supernatant solution is added and incubated at room temperature for one hour. Simultaneously, a solution containing 0.15 ml of the membrane fraction of pancreatic β cells and 10 mmol/l [$^3$H]-labeled Compound 16, to which 50% dimethylsulfoxide is added instead of the culture supernatant, and the solution to which is further added 10 μmol/l of unlabeled Compound 16 are prepared and incubated in the same manner.

After the completion of incubation, the membrane fraction was recovered and washed by the method described in Example 6 to count the radioactivity remaining on the membrane. The radioactivity with addition of the unlabeled Compound 16 was defined as the non-specific binding amount and the radioactivity without addition of the unlabeled Compound 16 was defined as the total binding amount, and then the individual specific binding amounts are calculated by the method described in Example 6. Using the calculated specific binding amounts, the binding inhibition ratio of the individual culture supernatant solutions can be determined.

Binding inhibition ratio (%)

= (specific binding in the presence of 50% dimethylsulfoxide - specific

binding in the presence of culture supernatant solution)

/specific binding in the presence of 50% dimethylsulfoxide × 100

Example 10

Screening method of substance having anti-diabetic activity by using compound library as screening source:

According to the method described in Example 5, a substance having anti-diabetic activity was screened for, using the inhibitory activity of the binding between pancreatic β cells and [$^3$H]-labeled Compound 16. A tested substance was dissolved in dimethylsulfoxide and then added to give a final concentration of 1 μmol/l. The radioactivity with addition of the unlabeled Compound 16 was defined as the non-specific binding amount and the radioactivity without addition of the unlabeled Compound 16 was defined as the total binding amount, and then the individual specific binding amounts were calculated by the method described in Example 6. Using the following formula, the binding inhibition ratio was determined.

Binding inhibition ratio (%)

= (specific binding in the presence of 50% dimethylsulfoxide - specific

binding in the presence of culture supernatant solution)

/ specific binding in the presence of 50% dimethylsulfoxide × 100

As screening results of 7,000 types of compounds, a compound having a structure described in the following formula (VII) could be selected, which had an inhibition ratio of 84% at 10 μmol/l:

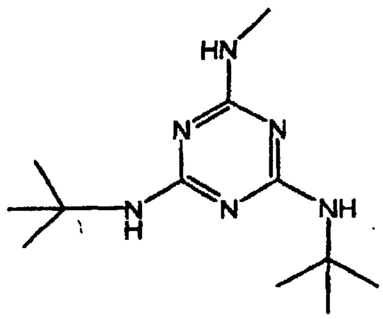

(VII)

The activity of the compound with the structure represented by formula (VII) (compound represented by Compound No. 193 in Table 4; hereinafter referred to as "Compound 193") to promote insulin secretion was assayed by the method described in Example 8. Consequently, the promoted insulin secretion level of the compound was 195% when the promoted insulin secretion level of Compound 16 was 100% (Table 4).

Compounds analogous to Compound 193, which are represented by Compound Nos. 194 to 199, were observed at 10 μmol/l to have insulin secretion-promoting activity. The compound represented by Compound No. 200 was observed at 1 μmmol/l to have an activity promoting insulin secretion (Table 4).

Table 4

| Compound No. | Structure | Insulin secretion-promoting activity (%) | Binding inhibition (%) |
|---|---|---|---|
| 193 | 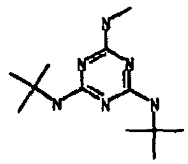 | 194.8 | 84.0 |
| 194 | 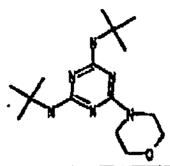 | 122.7 | 78.1 |
| 195 | 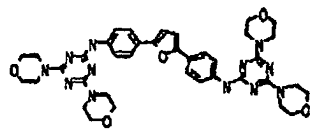 | 84.2 | 27.7 |
| 196 | 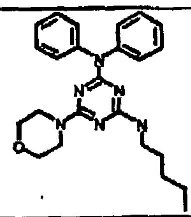 | 34.1 | 17.0 |
| 197 | 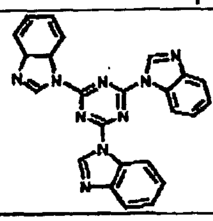 | 31.9 | 15.0 |
| 198 | 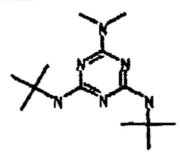 | 30.6 | 99.0 |
| 199 | 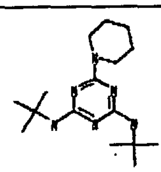 | 25.2 | 77.8 |
| 200 | 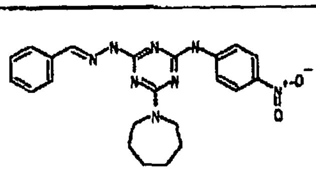 | 13.0 | 23.2 |

The above results indicate the usefulness of the screening method of the present invention.

The compounds were commercially purchased from ChemBrige or were otherwise synthesized by the method described in WO01/47921 or a known general synthetic method.

Example 11

Preparation of condensed purine derivative-immobilized particle:

As the condensed purine derivative, the compound represented by Compound No. 155 in Table 1 above was selected and used for the following experiment.

After 1 ml of NHS-activated Sepharose particle-suspended solution (manufactured by Amersham Pharmacia Biotech) was centrifuged (1,000 rpm, one minute, room temperature) to remove the supernatant, 5 ml of THF was added to the remaining pellet to disperse the particle, followed by centrifugation to remove the supernatant. The procedure was repeatedly carried out three times.

The solvent for NHS-activated Sepharose particle was exchanged with THF. A solution of the compound 196 was added to the 500 µl-pellet from the NHS-activated Sepharose particle, to disperse the NHS-activated Sepharose particle. The solvent then was THF; the volume was 500 µl; and the concentration of the Compound 155 was 6 mmol/l. Under occasional agitation, the mixture reacted together overnight at room temperature. After the completion of reaction, the particle was centrifuged (1,000 rpm, one minute, room temperature) to remove the supernatant.

In order to remove unreactive Compound 155, 5 ml THF was added to disperse the particle, followed by centrifugation (1,000 rpm, one minute, room temperature) to remove the supernatant. The procedure was repeatedly carried out three times. In order to inactivate the reaction point unreactive in particle, 5 ml of a THF solution containing 200 mmol/l monoethanolamlne was added to disperse the particle. Subsequently, a procedure centrifuging the resulting particle (1,000 rpm, one minute, room temperature) to remove the supernatant was carried out three times.

Compound 155-immobilized particle, thus obtained was dispersed in THF to 500 µl particle/ml and was then stored in darkness at 4°C.

Example 12

Preparation of cell extract solution:

Murine pancreatic β cell line MIN6 was cultured by static culturing in 425 ml of Dulbecco's Modified Eagle Medium (manufactured by Nissui Pharmaceutical Co., Ltd.) supplemented with 75 ml of FBS, 8.5 ml of 7.5% sodium hydrogen carbonate ($NaHCO_3$), 5 ml of 200 mmol/l L-glutamine, 2.5 ml of penicillin-streptomycin (a mixture solution of 1000 units/ml and 10 mg/l, respectively) in a dish in an incubator in 5% $CO_2$ at 37°C

When MIN6 reached confluence, the cell was washed twice with PBS at 37°C and then scraped with buffer A [20 mmol/l HEPES, pH 7.4, 2 mmol/l EDTA, 1 mmol/l PMSF, 1 µg/ml aprotinin] at 4°C. The cell mixture was stirred gently with a rotator at 4°C for 30 minutes. Subsequently, the cell mixture was centrifuged (12,000 rpm, 5 minutes, 4°C) to precipitate insoluble fractions. The resulting supernatant was defined as the soluble fraction. The pellet was suspended in 1 ml of buffer B [20 mmol/l HEPES, pH 7.4, 2 mmol/l EDTA, 150 mmol/l NaCl, 1% digitonin, 1 mmol/l PMSF, 1 µg/ml aprotinin] and gently stirred with a rotator at 4°C for 30 minutes. Subsequently, the mixture was centrifuged (12,000 rpm, 5 minutes, 4°C) to precipitate insoluble fractions. The resulting supernatant was defined as the solubilized membrane fraction. By the method, a soluble fraction at a protein concentration of about 3 mg/ml and the solubilized membrane fraction were obtained. The soluble fraction and the solubilized membrane fraction were stored at -80°C.

Example 13

Isolation of the condensed purine derivative-binding protein:

The solubilized membrane fraction of MIN6 as prepared in Example 12 was 10-fold diluted with the buffer B. The compound represented by Compound No. 16 (hereinafter referred to as "Compound 16") at 500 µmol/l was added to 1 ml of the resulting dilution and gently stirred at 4°C for 2 hours. As a control, the solubilized membrane fraction of MIN6 without addition of the Compound 16 was 10-fold diluted with buffer C [20 mmol/l HEPES, pH 7.4, 2 mmol/l EDTA, 150 mmol/l NaCl, 1% digitonin, 1 mmol/l PMSF, 1 µg/ml aprotinin], and 1 ml of the resulting dilution was gently stirred at 4°C for 2 hours. Then, a particle immobilizing a compound represented by Compound No. 155 (hereinafter referred to as "Compound 155-immobilized particle") as prepared in Example 8 was added to the solubilized membrane fraction solution with addition of the Compound 16 and the solubilized membrane faction solution without addition of any tested substance and gently stirred at 4°C for 30 minutes. Subsequently, the resulting solutions were centrifuged (12,000 rpm, one minute, 4°C) to precipitate the particle to thereby remove the supernatants. Then, 750 µl of the buffer C was added and stirred for 3 seconds, followed by centrifugation (12,000 rpm, one minute, 4°C), to precipitate the particle to remove the supernatant. Then, 750 µl of buffer D [20 mmol/l HEPES, pH 7.4, 2 mmol/l EDTA] was added

and stirred for 3 seconds, followed by centrifugation (12,000 rpm, one minute, 4°C), to precipitate the particle to remove the supernatant. Subsequently, 30 μl of an SDS-polyacrylamide gel electrophoresis sample buffer containing β-mercaptoethanol was added to solubilize the protein binding to Compound 155-immobilized particle, followed by SDS-polyacrylamide gel electrophoresis. Through the purification from the cell extract solution by adding Compound 16 via Compound 155-immobilized particle, p31 protein in which its specific binding to Compound 155-immobilized particle was inhibited by the compound represented by Compound No. 16 was found.

The p31 protein separated on SDS-polyacrylamide gel electrophoresis was scissored out from the gel and was allowed to react with 0.3 μg of lysyl endopeptidase (manufactured by Wako Pure Chemical) in 25 mmol/l Tris-HCl buffer, pH 8.5 at 37°C for 13 hours. A reaction supernatant containing peptide fragments obtained via digestion with lysyl endopeptidase was recovered for peptide separation by reverse phase HPLC. As the column, Magic C18 (0.2 mm I.D. × 5 cm; manufactured by Michrom BioResources) was used. The peptide was eluted on a linear acetonitrile gradient of 5% to 60%. The separated peptide was isolated into a tube for amino acid sequencing with a gas phase protein sequencer Precise 492cLC (manufactured by Applied Biosystems). The determined amino acid sequence was shown as SEQ ID NO:11.

A gel section containing the p31 protein after digestion with lysyl endopeptidase further reacted with 0.3 μg of trypsin (sequence grade; manufactured by Roche Diagnostics) in 100 mmol/l Tris-HCl buffer, pH 8.8 at 37°C for 13 hours. The peptide fragment obtained with trypsin digestion was separated by reverse phase HPLC by the same method as described above, for determination of the amino acid sequence. The determined amino acid sequence was shown as SEQ ID NO:12.

The amino acid sequences represented by SEQ ID NOs:11 and 12 were subjected to homology screening in the PIR protein database. Consequently, it was confirmed that these sequences coincided having a sequence consisting of the 199th amino acid residue to the 209th amino acid residue from the N terminus of the amino acid sequence represented by SEQ ID NO:2, which was derived from mouse NADPH-dependent retinol dehydrogenase/reductase, and a sequence consisting of the 34th amino acid residue to the 40th amino acid residue from the N terminus thereof.

Example 14

Preparation of p31 protein:

[1] Cloning of p31 cDNA from murine pancreatic β cell line and construction of plasmid expressing His-p31 protein

Using total RNA extracted from the murine pancreatic β cell line (MIN6) as template and oligodT as primer, a single-strand cDNA was synthesized. Using the single-stranded cDNA as template, a gene part encoding the full-length p31 protein was prepared by polymerase chain reaction (PCR), which was then integrated in an *Escherichia coli* expression vector pET-14b purchased from NOVAGEN to construct an expression plasmid of His-p31 protein with His tag added at the N terminus. The detail is now described below.

From 1 μg of the total RNA extracted from MIN6 cell according to the general method [*Biochemistry*, 18, 5294 (1977)], a single-stranded cDNA was synthesized, using a single-stranded cDNA synthetic kit SuperScript™ First-Strand Synthesis System for RT-PCR (manufactured by GIBCO BRL) and using oligo dT as primer. As PCR template, 0.4% of the single-stranded cDNA was used.

As a primer set for PCR, synthetic DNAs consisting of nucleotide sequences represented by SEQ ID NOs:7. and 8 as synthesized by GENSET were used.

The DNA consisting of the nucleotide sequence represented by SEQ ID NO:7 was designed to have an *Nde*I site, while the DNA consisting of the nucleotide sequence represented by SEQ ID NO:8 was designed to have a *Bam*HI site.

PCR was carried out by using Pyrobest DNA polymerase (manufactured by Takara Shuzo). The reaction solution was prepared according to the instruction for the polymerase. Using GeneAmp PCR system 9700 (Applied Biosystems), the reaction solution was incubated at 95°C for 5 minutes, then subjected to 30 cycles of 94°C for 30 seconds, 65°C for one minute and 72°C for 2 minutes, and incubated at 72°C for 7 minutes. In such manner, the reaction was progressed. After the completion of the reaction, DNA was purified by using a DNA purification kit Prep-A-Gene DNA Purification Systems (manufactured by BIO-RAD LABORATORIES, INC.).

The DNA was dissolved in 10 μl of NEB2 buffer (manufactured by New England Biolab), and 10 units of *Nde*I and 10 units of *Pst*I were added thereto for digestion at 37°C for 2 hours. The reaction solution was subjected to electrophoresis on agarose gel, to recover a DNA fragment of about 0.8 kb.

In 20 μl of the NEB2 buffer, 1 μg of pET-14b was dissolved, and 20 units of *Nde*I and 20 units of *Pst*I were added thereto for digestion at 37°C for 2 hours. The reaction solution was subjected to electrophoresis on agarose gel to recover a DNA fragment of about 3.4 kb.

In 20 μl of the NEB2 buffer, 1 μg of pET-14b was dissolved, and 20 units of *Pst*I and 20 units of *Bam*HI were

added thereto for digestion at 37°C for 2 hours. The reaction solution was subjected to electrophoresis on agarose gel, to recover a DNA fragment of about 13 kb.

In 10 μl of T4 DNA ligase buffer, 0.1 μg of the *Nde*I-*Bam*HI fragment (0.8 kb) derived from the PCR-amplified DNA, and 0.03 μg of the *Nde*I-*Pst*I fragment (3.4 kb) and 0.05 μg of the *Pst*I-*Bam*HI fragment as derived from pET-14b were dissolved, and 400 units of T4 DNA ligase were added thereto for ligation at 16°C for 16 hours.

Using the reaction solution, an *Escherichia coli* strain BL21 (manufactured by Stratagene) was transformed by the method of Cohen, *et al.* to recover a transformant. The plasmid harbored on the transformant strain was isolated according to a usual method to confirm the structure by digestion with restriction enzymes. The plasmid was designated as pET-14b-p31.

The nucleotide sequence of the plasmid pET-14b-p31 obtained above was determined by using a nucleotide sequencing kit of Applied Biosystems (BigDye Terminator Cycle Sequencing Ready Reaction Kit v2.0).

Consequently, the gene cloned in this Example encoded the protein consisting of 260 amino acids represented by SEQ ID NO:2. It was shown that the amino acid sequence was identical to the amino acid sequence of a murine-derived protein as described in GenBank Accession No. AB045132.1.

[2] Expression of murine His-p31 protein in *Escherichia coli*

The *Escherichia coli* strain BL21 was transformed with the plasmid pET-14b-p31 obtained above in the above [1] by the method of Cohen, *et al.* to obtain a transformant strain BL21/pET-14b-p31. BL21/pET-14b-p31 was cultured under shaking (220 rpm) in a 15-ml test tube containing 5 ml of ampicillin-containing L-broth [10 g/l Bacto-Tryptone (manufactured by DIFCO), 5 g/l yeast extract (manufactured by DIFCO), 5 g/l NaCl, 75 μg/ml ampicillin] at 37°C for 8 hours. Then, 2-5 ml of the culture was added to a 300-ml baffled Erlenmeyer flask containing 50 ml of the ampicillin-containing L-broth for shaking culture at 37°C until OD (590 nm) reached 1.0. Then, the resulting product was stored at 4°C. Subsequently, 20 ml each of the culture was added to two 2-L baffled Erlenmeyer flasks each containing 500 ml of ampicillin-containing L-broth, for aeration culture under shaking (220 rpm) at 37°C, until OD (590 nm) reached 0.9. Just then, IPTG (isopropyl-β-D-thiogalactopyranoside) as an expression inducer was added to 0.2 mmol/l to induce the expression of the His-p31 protein. Four hours after the expression induction, the culturing was terminated. The culture was centrifuged to obtain bacterial cells of about 6.6 g (wet weight).

[3] Purification of mouse His-p31

The mouse His-p31 was purified from the cultured bacterial cells obtained in Example 5 under conditions of 0 to 4°C without exception, by the following method.

After 6.6 g (wet weight) of the cultured bacterial cells obtained above in [2] was suspended in 20 ml of a homogenizing solution (20 mmol/l sodium phosphate buffer, 500 mmol/l NaCl, 5 mmol/l imidazole, 10 mmol/l dithiothreitol, pH 7.4), *Escherichia coli* was disrupted about 10 times. with an ultrasonicator (manufactured by Ieda-boeki; Vibra-Cell at an amplitude level of 70). for 5 seconds. The resulting disruption solution was centrifuged at 9,000 rpm for 15 minutes. The resulting supernatant was filtered through a filter (manufactured by Millipore; Millex-GV; 0.22 μm). The filtrate was purified by metal chelate affinity chromatography under the following conditions.
Conditions for metal chelate affinity chromatography:

Column: Chelating Sepharose Fast Flow with coordinated zinc, as prepared by elution with aqueous 200 mmol/l zinc sulfate solution at a flow of 24 ml/h for 20 minutes and then at a flow of 6 ml/h for 24 hours (manufactured by Amersham Pharmacia Biotech; 10 × 100 mm)
Eluent: 1000 ml of Solution A (20 mmol/l sodium phosphate buffer, 500 mmol/l NaCl, 5 mmol/l imidazole, pH 7.4) and 1000 ml of Solution B (20 mmol/l sodium phosphate buffer, 500 mmol/l NaCl, 500 mmol/l imidazole, pH 7.4)
Flow: 6 ml/h
Elution: eluted on a linear concentration gradient from solution A to solution B

The eluate was fractionated in 1.3-ml portions. Each fraction was analyzed by SDS-polyacrylamide electrophoresis under mercaptoethanol reduction, to collect a faction containing the mouse His-p31 band around a molecular weight of 31 kDa. The fraction was defined as zinc chelate chromatography mouse His-p31 fraction.

The p31 fraction via zinc chelate chromatography was treated with desalting. The desalting process was done under the following conditions.
Desalting process conditions:

Column: PD-10 (manufactured by Amersham Pharmacia Biotech)

Eluent: 400 mmol/l potassium phosphate buffer, pH 7.4

The eluate was fractionated at 0.5 ml. Each fraction was analyzed by SDS-polyacrylamide electrophoresis under mercaptoethanol reduction to collect a fraction containing the mouse His-p31 band around a molecular weight of 31 kDa. The resulting fraction was defined as the PD-10 mouse His-p31 fraction. The analytical results of the obtained His-p31 by SDS-PAGE are shown in Fig. 6.

[4] Confirmation of the structure of mouse His-p31

From the mouse His-p31 protein obtained above in the above item [3], the histidine tag at the N terminus was removed by thrombin digestion, for N-terminal amino acid sequencing. Specifically, 100 mU of biotinylated thrombin (biotinylated human thrombin; manufactured by Novagen) was added to about 10 μg of His-p31 for reaction in a reaction solution (20 mmol/l Tris-HCl buffer, pH 8.4, 1.5 mol/l NaCl, 2.5 mmol/l $CaCl_2$) at 20 °C for 15 hours. After the reaction solution was subjected to SDS-PAGE under 2-mercaptoethanol reduction, the gel was electrically transferred onto PDVF membrane (proBlott; manufactured by Applied Biosystems) according to the method described in *J. Biol. Chem.,* 262, 10035-10038 (1987). The membrane after transfer was stained with Coomassie-Blue, from which a band of a molecular weight of about 31 kDa was scissored out, to analyze the N terminal amino acid sequence using a gas phase protein sequencer (PPSQ-10; manufactured by Shimadzu Corporation) by the method recommended by the manufacturer. As shown in SEQ-ID NO:9, the resulting amino acid sequence was identical to an amino acid sequence, starting from the first residue from the N terminus of the amino acid sequence described as SEQ ID NO:2, as speculated on the basis of the gene sequence. It was confirmed that the first residue to the third residue from the N terminus of the amino acid sequence represented by SEQ ID NO:9 was derived from the pET-14b vector and the sequence from the fourth residue thereafter was identical to the amino acid sequence derived from mouse p31.

Example 15

Binding experiment using p31 protein:

Binding experiments using the His-p31 purified in Example 14 were carried out as follows.

A sensor chip CM5 (manufactured by BIAcore) was arranged in BIAcore2000 (manufactured by BIAcore) and was then equilibrated at 10°C, using HBS-EP buffer (100 mmol/l HEPES, 5 mmol/l NaCl, 500 mmol/l EDTA, 0.005% Polysorbate 20 v/v, pH 7.4; filtered through 0.22 μm filter) according to the attached manual.

After 50 μl of an EDC reagent for ligand immobilization (N-ethyl-N'-(3-dimethylaminapropyl)carbodiimide hydrochloride; manufactured by BIAcore) and 50 μl of an NHS reagent (H-hydroxylsuccinimide; manufactured by BIAcore) were mixed together, 70 μl of the resulting mixture flowed at a flow rate of 10 μl/minute on the sensor chip to activate the chip surface. The compound represented by Compound No. 155 in Table 1 above was dissolved in an immobilization buffer (20 mmol/l sodium carbonate buffer, pH 8.5) to give a concentration of 100 μg/ml. The resulting solution was injected onto the sensor chip at a flow rate of 10 μl/minute, for immobilization to about 50 RU.

Subsequently, 70 μl of 1 mol/l ethanolamine was injected at a flow rate of 10 μl/minute for reaction of the carboxyl group unreactive on the sensor chip. Then, the sensor chip was washed twice with 10 μl of 100 mmol/l hydrochloric acid

A solution prepared by dissolving the mouse His-p31 purified above in Example 12 in HBS-EP buffer to a concentration of 100 nmol/l was defined as His-p31 solution, while a tested substance solution added to the His-p31 solution to a tested substance concentration of 500 μmol/l was defined as His-p31/tested substance solution. These were used for binding experiments. Further, a solution prepared by dissolving a compound represented by Compound No. 16, 17, 36, 38, 103 or 144 in Table 1 above in dimethylsulfoxide (DMSO) to give a concentration of 50 mmol/l, and a solution prepared by dissolving a compound represented by Compound No. 94 or 183 in Table 1 in DMSO to give a concentration of 10 mmol/l were used as tested substance solutions.

The tested substance solutions were allowed to flow onto the sensor chip at a flow rate of 20 μl/minute for 300 seconds, and the binding amount (RU) was measured 15 seconds after the completion of the flowing. The surface of the sensor chip was regenerated by individually allowing 2 mmol/l guanidine-HCl and 10 mmol/l glycine-HCl to flow for 120 seconds.

By defining the binding amount (RU) before the flow of the p31 solution as 100% and defining the binding amount (RU) when the His-p31 solution with DMSO added therein instead of the tested substance solutions as 0%, the inhibition ratio of each tested substance on the binding of His-p31 to the immobilized Compound 155 was calculated and corrected on the basis of the binding amount (RU) when the His-p31/tested substance solution was allowed to flow.

Fig. 7 shows the correlation between the inhibition ratio of each tested substance on the binding of His-p31 to the immobilized Compound 155 and the activity of each tested substance for promoting insulin secretion as assayed

by the method described in Test Example 1. The correlation coefficient between the inhibition ratio of each tested substance on the binding of His-p31 to the immobilized Compound 155 and the activity of the substance for promoting insulin secretion was 0.78, indicating that the correlation was significant.

Herein, the tested substances were synthesized according to the methods described in WO00/01388 and WO01/47931.

Example 16

Screening of an insulin secretion-promoting agent by using a cell extract solution and a particle immobilizing compound represented by Compound No. 155 thereon:

To 1 ml of a 10-fold dilution of the solubilized membrane fraction of MIN6 as prepared in Example 12 with buffer B, 500 μmol/l tested substance (compound represented by Compound No. 16 or 17) was added and gently stirred at 4°C for 2 hours. As a control, 1 ml of a 10-fold dilution of the solubilized membrane fraction of MIN6 as prepared in Example 12 with buffer C [20 mmol/l HEPES, pH 7.4, 2 mmol/l EDTA, 150 mmol/l NaCl, 1% digitonin, 1 mmol/l PMSF, 1 μg/ml aprotinin], without any addition of any tested substance, was gently stirred at 4°C for 2 hours. Subsequently, a particle immobilizing the compound represented by Compound No. 15 thereon (hereinafter referred to as "Compound 155-immobilized particle") as prepared in Example 8 was added to the solutions of the solubilized membrane fraction with the tested substance added and the solubilized membrane fraction without the tested substance added and gently stirred at 4°C for 30 minutes. Subsequently, the particle was precipitated by centrifugation (12,000 rpm, one minute, 4°C) to remove the supernatant. Then, 750 μl of the buffer C was added and stirred for 3 seconds, followed by centrifugation (12,000 rpm, one minute, 4°C) to precipitate the particle to thereby remove the supernatant. Subsequently, 750 μl of buffer D [20 mmol/l HEPES, pH 7.4, 2 mmol/l EDTA] was added and stirred for 3 seconds. Then, the particle was precipitated by centrifugation (12,000 rpm, one minute, 4°C) to remove the supernatant. Then, 30 μl of an SDS-polyacrylamide gel electrophoresis sample buffer containing β-mercaptoethanol was added to solubilize the protein binding to the Compound 155-immobilized particle for SDS-polyacrylamide gel electrophoresis. The results are shown in Fig. 8.

At a control experiment without the test compound added, the band of the p31 protein can be confirmed. However, the band of the p31 protein cannot be confirmed at the experiment with the tested substance Compound 16 added. Thus, it is indicated that the Compound 16 inhibits the binding of the p31 protein to the Compound 155-immobilized particle. At an experiment using Compound 17 as a tested substance, alternatively, the band of the p31 protein cannot be confirmed. Thus, it is shown that Compound 17 cannot inhibit the binding of the p31 protein to the Compound 155-immobilized particle.

Example 17

Screening of insulin secretion-promoting agent using His-p31 protein (1):

Competitive experiment for binding by using 96-well plate:

To each well of a 96-well ELISA plate, 100 μl of a PBS solution containing 10 μg of the His-p31 protein prepared in Example 12 is added and allowed to stand overnight at 4°C for adsorption. Then, 300 μl of 1% BSA/PBS is added to each well and allowed to stand at 4°C for one hour for blocking.

A compound represented by Compound No. 155 labeled with fluorescein isothiocyanatc (FITC) (hereinafter referred to as "FITC-labeled Compound 155") can be prepared by mixing NHS-fluorescein commercially available with the compound represented by Compound No. 155 in Table 1 above in DMSO at room temperature for 3 hours.

In the presence or absence of 100 μmol/l tested substance, 100 μl of 5 μmol/l FITC-labeled Compound 155 is brought into contact with the His-p31 protein; after the supernatant is removed, the resulting pellet is washed with PBS containing 0.05% Tween 20; and the remaining FTTC-labeled Compound 155 is assayed with fluorescence plate reader to select a substance having activity of inhibiting the binding of the condensed purine derivative and the protein capable of binding to the condensed purine derivative.

The insulin secretion-promoting activity of the substance can be confirmed by the method descried above in Example 8.

Example 18

Screening of insulin secretion-promoting agent, using His-p31 protein (2):

Competitive experiment for binding by using chelate Sepharose bead:

400 µl of the supernatant of a disrupted product of the culture of a recombinant *Escherichia coli* expressing the His-p31 protein as prepared in Example 14 is mixed with 10 µl of $Zn^{2+}$ ion chelate Sepharose for binding the protein, followed by washing with PBS. The resulting bead is defined as affinity bead. The affinity bead is mixed with 1 ml of a solution containing 1 µmol/l FITC-labeled Compound 155 in the presence or absence of 500 µmol/l tested substance to remove the supernatant. After the affinity bead is washed, the remaining FITC-labeled Compound 155 is extracted into 100 µl of aqueous 1% SDS solution for assaying the fluorescence intensity of the extract solution with a fluorescence spectrometric system, so that a substance which inhibits the binding of the condensed purine derivative to the protein capable of binding to the condensed purine derivative can be selected.
The insulin secretion-promoting activity of the substance can be confirmed by the method of Example 8 above.

Example 19

Screening of insulin secretion-promoting agent using His-p31 protein (3)

Experiment about enzyme activity inhibition:

NADPH was added to 1 ml of a solution of 10 mmol/Tris-HCl, 100 mmol/l NaCl and 2 mmol/l EDTA, pH 7.4 containing the His-p31 protein prepared in Example 12 at 1 µg/ml and p-chlorobenzaldehyde at 250 µmol/l to give a final concentration of 5 µmol/l and incubated at 37°C. Then, the NADPH decrease following the decomposition of p-chlorobenzaldehyde in the presence or absence of a tested substance was detected at the absorbance at 340 nm.
The enzyme activity inhibition of the His-p31 protein was assayed when the substance represented by Compound No. 16 or 17 was used as the tested substance. The results show that the compound represented by Compound No. 16 strongly inhibited the activity of the protein, in comparison with the compound represented by Compound No. 17.

Example 20

Screening of insulin secretion-promoting agent using His-p31 protein (4):

Experiment about enzyme activity inhibition:

NADPH is added to 150 µl of a solution of 10 mmol/Tris-HCl, 100 mmol/l NaCl and 2 mmol/l EDTA, pH 7.4 containing the His-p31 protein prepared in Example 12 at 1 µg/ml and p-chlorobenzaldehyde at 250 µmol/l to give a final concentration of 5 µmol/l and incubated at 37°C. Then, 150 µl of a solution containing 0.2 mol/l potassium cyanide (KCN), 0.06 mol/l potassium hydroxide (KOH) and 1 mol/l bathophenanthroline disulfonic acid is added to the resulting reaction solution and allowed to stand at room temperature for 5 minutes. furthermore, 800 µl of chloroform is added for mixing. Subsequently, the resulting mixture is centrifuged at 150,000 rpm at 4°C for 5 minutes. The resulting supernatant is 4-fold diluted with 0.2 mol/l ammonium acetate, pH 6.0, and then 250 µl of the resulting dilution is injected for HPLC. The decrease of NADPH according to the decomposition of the substrate by p31 in the presence or absence of a tested substance is detected with a fluorescence detector (330/460 nm) [*Analytical Biochemistry,* 228, 312 (199: 5)] to select a substance inhibiting the activity of His-p31 from the tested substances.
The insulin secretion-promoting activity of the selected substance can be confirmed by the method in Example 8 above.

Example 21

Screening of insulin secretion-promoting agent using His-p31 protein (5):

Experiment about enzyme activity inhibition:

Solution A [20 mmol/l HEPES-NaOH, 50 mmol/l KCl and 3 mmol/l $MgCl_2$, pH 7.5 containing 1% bovine serum albumin, 20% glycerol, and 1% CHAPS] and Solution B [40 mmol/l HEPES NaOH, 100 mmol/l KCl and 6 mmol/l $MgCl_2$, pH 7.5 containing 40 µmol/l all-trans retinal, 25 µmol/l NADPH, 0.1% bovine serum albumin, 2% glycerol, and 0.1%

CHAPS] are prepared. The solution A contains the His-p31 protein prepared in Example 12 at 0.1 µg/ml. After 0.2 ml of the solution A and 0.2 ml of the solution B are mixed together and incubated at 32°C for 10 minutes, the decrease of NADPH according to the decomposition of the retinal in the presence or absence of a tested substance was detected at the absorbance at 340 nm [*Biochimica et Biophysica Acta,* 1338, 47 (1997)], so that a substance inhibiting the NADPH oxidation activity of the His-p31 protein can be selected from the tested substances.

The insulin secretion-promoting activity of the selected substance can be confirmed by the method of Example 8 above.

INDUSTRIAL APPLICABILITY

A therapeutic agent of diabetes mellitus without the onset of complications such as hypoglycemia, using the condensed purine derivative, as well as a screening method of an insulin secretion-promoting agent, can be provided in accordance with the present invention.

## Claims

**1.** A screening method of a substance having anti-diabetic activity, which comprises:

[1] bringing a compound selected from compounds represented by the following formula (1):

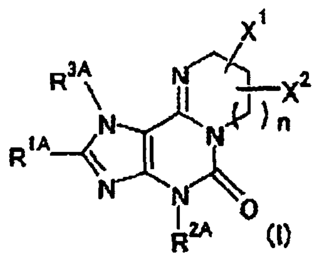
(I)

(wherein $R^{1A}$ represents hydrogen, lower alkyl, substituted or unsubstituted aryl, or a substituted or unsubstituted aromatic heterocyclic group; $R^{2A}$ represents hydrogen, lower alkyl, substituted or unsubstituted aralkyl, substituted or unsubstituted aryl, or a substituted or unsubstituted aromatic heterocyclic group; $R^{3A}$ represents hydrogen, lower alkyl, or substituted or unsubstituted aralkyl; $X^1$ and $X^2$ each independently represents hydrogen, lower alkyl, substituted or unsubstituted aralkyl, or substituted or unsubstituted aryl; and n is an integer of 0 to 3) or compounds represented by the following formula (II):

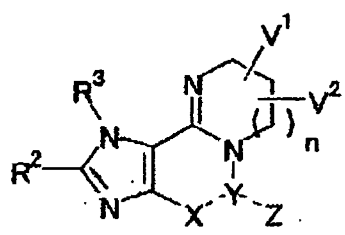
(II)

{wherein X--Y--Z represents $R^1N-C=O$ (wherein $R^1$ represents hydrogen, substituted or unsubstituted lower alkyl, substituted or unsubstituted aralkyl, substituted or unsubstituted aryl or a substituted or unsubstituted aromatic heterocyclic group) or N=C-W [wherein W represents halogen, a substituted or unsubstituted aromatic heterocyclic group, a substituted or unsubstituted alicyclic heterocyclic group, $-NR^4R^5$ (wherein $R^4$ and

$R^5$ are the same or different and each represents hydrogen, substituted or unsubstituted lower alkyl, substituted or unsubstituted aryl, or substituted or unsubstituted aralkyl or $R^4$ and $R^5$ together with adjacent nitrogen represent a heterocyclic group), $-OR^6$ (wherein $R^6$ represents substituted or unsubstituted lower alkyl, substituted or unsubstituted aryl, or substituted or unsubstituted aralkyl), $-SR^{6a}$ (wherein $R^{6a}$ has the same meaning as $R^6$), substituted or unsubstituted lower alkyl, or cyano]; $R^2$ represents hydrogen, substituted or unsubstituted lower alkyl, substituted or unsubstituted aralkyl, substituted or unsubstituted aryl, a substituted or unsubstituted aromatic heterocyclic group, a substituted or unsubstituted alicyclic heterocyclic group, halogen, lower alkylthio, $-NR^7R^8$ (wherein $R^7$ and $R^8$ have the same meanings as $R^4$ and $R^5$ as described above, respectively), $-CO_2H$, lower alkoxycarbonyl, -COHal (wherein Hal represents halogen), $-CONR^9R^{10}$ (wherein $R^9$ and $R^{10}$ have the same meanings as $R^4$ and $R^5$ as described above, respectively), or -CHO; $R^3$ represents hydrogen, lower alkyl, substituted or unsubstituted aralkyl, or lower alkoxyalkyl; n represents an integer of 0 to 3; $V^1$ represents hydrogen, substituted or unsubstituted lower alkyl, substituted or unsubstituted aralkyl, substituted or unsubstituted aryl, or a substituted or unsubstituted aromatic heterocyclic group; and $V^2$ represents substituted lower alkyl or a substituted or unsubstituted aromatic heterocyclic group, and when $V^1$ represents hydrogen, lower alkyl, substituted or unsubstituted aralkyl or substituted or unsubstituted aryl and (a) X--Y--Z represents $R^{1a}N$-C=O (wherein $R^{1a}$ represents the same groups defined for $R^1$ except the substituted lower alkyl), and $R^2$ represents substituted lower alkyl, substituted or unsubstituted aralkyl, a substituted or unsubstituted alicyclic heterocyclic group, halogen, lower alkylthio, $-NR^7R^8$ (wherein $R^7$ and $R^8$ have the same meanings as described above, respectively), $-CO_2H$, lower alkoxycarbonyl, -COHal (wherein Hal has the same meaning as described above), $-CONR^9R^{10}$ (wherein $R^9$ and $R^{10}$ have the same meanings as described above, respectively), or -CHO, (b) X--Y--Z represents $R^1N$-C=O (wherein $R^1$ has the same meaning as described above) and $R^3$ represents lower alkoxyalkyl, (c) X--Y--Z represents $R^{1b}N$-C=O (wherein $R^{1b}$ represents substituted lower alkyl), (d) X--Y--Z represents N=C-W (wherein W has the same meaning as described above), and $R^2$ represents substituted or unsubstituted lower alkyl. substituted or unsubstituted aralkyl, substituted or unsubstituted aryl, a substituted or unsubstituted aromatic heterocyclic group, a substituted or unsubstituted alicyclic heterocyclic group, halogen, lower alkylthio, $-NR^7R^8$ (wherein $R^7$ and $R^8$ have the same meanings as described above, respectively), $-CO_2H$, lower alkoxycarbonyl, -COHal (wherein Hal has the same meaning as described above), $-CONR^9R^{10}$ (wherein $R^9$ and $R^{10}$ have the same meanings as described above, respectively), or-CH, or (e) X--Y--Z represents N=-W (wherein W has the same meaning as described above), and $R^3$ represents lower alkyl, substituted or unsubstituted aralkyl, or lower alkoxyalkyl, $V^2$ may represent lower alkyl, substituted or unsubstituted aralkyl, or substituted or unsubstituted aryl] (hereinafter referred to as "condensed purine derivative") into contact with a pancreatic β cell or a treated product of the cell in the presence of a tested substance to assay the binding amount of the condensed purine derivative to the pancreatic β cell or the treated product of the cell,

[2] bringing the condensed purine derivative into contact with a pancreatic β cell or a treated product of the cell in the absence of the tested substance to assay the binding amount of the condensed purine derivative to the pancreatic β cell or the treated product of the cell,

[3] comparing the assayed amount in the presence of the tested substance with that in the absence of the tested substance, and

[4] selecting a substance which inhibits the binding of the condensed purine derivative to the pancreatic β cell or the treated product of the cell from the tested substances.

**2.** A screening method of a substance having anti-diabetic activity, which comprises:

[1] bringing the condensed purine derivative described in claim 1 into contact with a protein capable of binding to the condensed purine derivative in the presence of a tested substance to assay the binding amount of the condensed purine derivative to the protein,

[2] bringing the condensed purine derivative into contact with the protein in the absence of the tested substance to assay the binding amount of the condensed purine derivative to the protein,

[3] comparing the assayed amount in the presence of the tested substance with that in the absence of the tested substance, and

[4] selecting a substance which inhibits the binding of the condensed purine derivative to the protein from the tested substances.

**3.** A screening method of a substance which is capable of binding to a protein capable of binding to the condensed purine derivative described in claim 1, which comprises:

[1] bringing the condensed purine derivative described in claim 1 into contact with a pancreatic β cell or a

treated product of the cell in the presence of a tested substance to assay the binding amount of the condensed purine derivative to the pancreatic β cell or the treated product of the cell,
[2] bringing the condensed purine derivative into contact with a pancreatic β cell or a treated product of the cell in the absence of the tested substance to assay the binding amount of the condensed purine derivative to pancreatic β cell or the treated product of the cell,
[3] comparing the assayed amount in the presence of the tested substance with that in the absence of the tested substance,
[4] selecting a substance which inhibits the binding of the condensed purine derivative to the pancreatic β cell or the treated product of the cell from the tested substances, and
[5] selecting a substance binding to a protein capable of binding to the condensed purine derivative from the substances obtained in the step [4].

**4.** A screening method of a substance which is capable of binding to a protein capable of binding to the condensed purine derivative described in claim 1, which comprises:

[1] bringing the condensed purine derivative described in claim 1 into contact with a protein capable of binding to the condensed purine derivative in the presence of a tested substance to assay the binding amount of the condensed purine derivative to the protein,
[2] bringing the condensed purine derivative into contact with the protein in the absence of the tested substance to assay the binding amount of the condensed purine derivative to the protein,
[3] comparing the assayed amount in the presence of the tested substance with that in the absence of the tested substance, and
[4] selecting a substance which inhibits the binding of the condensed purine derivative to the protein from the tested substances.

**5.** A screening method of a substance which is capable of changing the activity of a protein capable of binding to the condensed purine derivative described in claim 1, which comprises:

[1] bringing a tested substance into contact with a protein capable of binding to the condensed purine derivative described in claim 1 to assay the activity of the protein,
[2] assaying the activity of the protein which is out of contact with the tested substance,
[3] comparing the assayed amount in the presence of the tested substance with that in the absence of the tested substance, and
[4] selecting a substance which is capable of changing the activity of the protein from the tested substances.

**6.** A screening method of a substance which is capable of suppressing the activity of a protein capable of binding to the condensed purine derivative described in claim 1, which comprises:

[1] bringing a tested substance into contact with a protein capable of binding to the condensed purine derivative described in claim 1 to assay the activity of the protein,
[2] assaying the activity of the protein which is out of contact with the tested substance,
[3] comparing the assayed amount in the presence of the tested substance with that in the absence of the tested substance, and
[4] selecting a substance which is capable of suppressing the activity of the protein from the tested substances.

**7.** A screening method of a substance which is capable of changing the function of a protein capable of binding to the condensed purine derivative described in claim 1, which comprises:

[1] bringing a tested substance into contact with a transformant which expresses a protein capable of binding to the condensed purine derivative described in claim 1 to assay the cell response of the transformant,
[2] assaying the cell response of the transformant in the absence of the tested substance,
[3] comparing the assayed amount in the presence of the tested substance with that in the absence of the tested substance, and
[4] selecting a substance which is capable of changing the cell response of the transformant from the tested substances.

**8.** A screening method of a substance which is capable of changing the expression level of a gene encoding a protein capable of binding to the condensed purine derivative described in claim 1, which comprises:

[1] bringing a tested substance into contact with a transformant which expresses a protein capable of binding to the condensed purine derivative described in claim 1 to assay the expression level of the gene encoding the protein in the transformant,
[2] assaying the expression level of the gene encoding the protein in the transformant in the absence of the tested substance,
[3] comparing the assayed amount in the presence of the tested substance with that in the absence of the tested substance, and
[4] selecting a substance which is capable of changing the expression level of the gene encoding the protein from the tested substances.

**9.** A screening method of a substance which is capable of changing the expression level of a protein capable of binding to the condensed purine derivative described in claim 1, which comprises:

[1] bringing a tested substance into contact with a transformant which expresses a protein capable of binding to the condensed purine derivative described in claim 1 to assay the expression level of the protein in the transformant,
[2] assaying the expression level of the protein in the transformant in the absence of the tested substance,
[3] comparing the assayed amount in the presence of the tested substance with that in the absence of the tested substance, and
[4] selecting a substance which is capable of changing the expression level of the protein from the tested substances.

**10.** The screening method according to any one of claims 1 to 9, wherein the condensed purine derivative is a compound represented by the following formula (III):

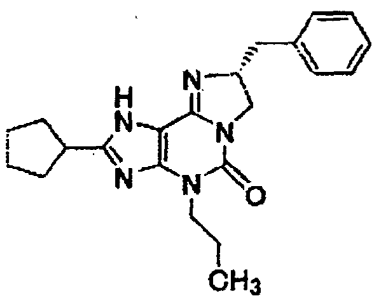

(III)

**11.** The screening method according to any one of claims 2 and 4 to 9, wherein the purine derivative described in claim 1 is immobilized on a carrier.

**12.** The screening method according to claim 11, wherein the carrier is a Sepharose particle.

**13.** The screening method according to claim 11 or 12, wherein the condensed purine derivative is a compound represented by formula (IV):

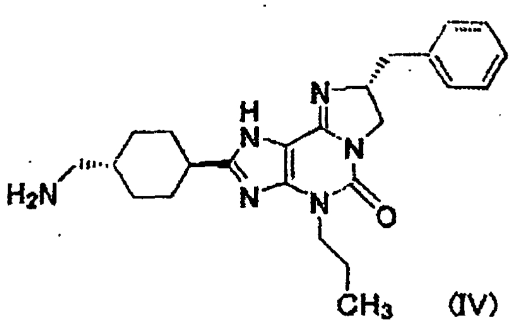

(IV)

**14.** A carrier which is immobilized with the condensed purine derivative described in claim 1.

**15.** The carrier according to claim 14, wherein the condensed purine derivative is the condensed purine derivative represented by formula (IV) described in claim 13.

**16.** The carrier according to claim 14 or 15, wherein the carrier is a Sepharose particle.

**17.** The screening method according to any one of claims 2 and 4 to 13, wherein the protein capable of binding to the condensed purine derivative described in claim 1 is a protein selected from the following [i] to [iii]:

[i] a protein which comprises the amino acid sequence represented by any one of SEQ ID NOs:1 to 3;
[ii] a protein which consists of an amino acid sequence in which at least one amino acid is deleted, substituted, inserted or added in the amino acid sequence represented by any one of SEQ ID NOs:1 to 3, and is capable of binding to the condensed purine derivative described in claim 1; and
[iii] a protein which consists of an amino acid sequence having a homology of 60% or more with the amino acid sequence represented by any one of SEQ ID NOs:1 to 3, and is capable of binding to the condensed purine derivative described in claim 1.

**18.** A substance obtainable by the screening method according to any one of claims 1 to 13 and 17 or a pharmaceutically acceptable salt of the substance.

**19.** A medicament which comprises the substance according to claim 18 or a pharmaceutically acceptable salt of the substance as an active ingredient.

**20.** A therapeutic agent of diabetes mellitus, which comprises the substance according to claim 18 or a pharmaceutically acceptable salt of the substance as an active ingredient.

**21.** An insulin secretion-promoting agent, which comprises the substance according to claim 18 or a pharmaceutically acceptable salt of the substance as an active ingredient.

**22.** An insulin secretion-promoting agent, which comprises a compound represented by the following formula (V):

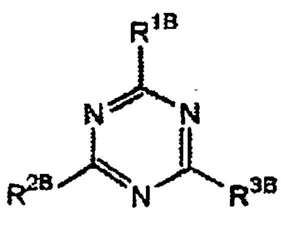

(V)

[wherein $R^{1B}$, $R^{2B}$ and $R^{3B}$ are the same or different and each represents amino, mono- or di-substituted amino, or a substituted or unsubstituted N-containing heterocyclic group (wherein the heterocyclic group binds to the triazine ring at the N atom)] or a pharmaceutically acceptable salt thereof as an active ingredient.

**23.** A method for promoting insulin secretion, which comprises administering an effective amount of the compound represented by formula (V) described in claim 22 or a pharmaceutically acceptable salt thereof.

**24.** Use of the compound represented by formula (V) described in claim 22 or a pharmaceutically acceptable salt thereof for the manufacture of an insulin secretion-promoting agent.

FIG. 1

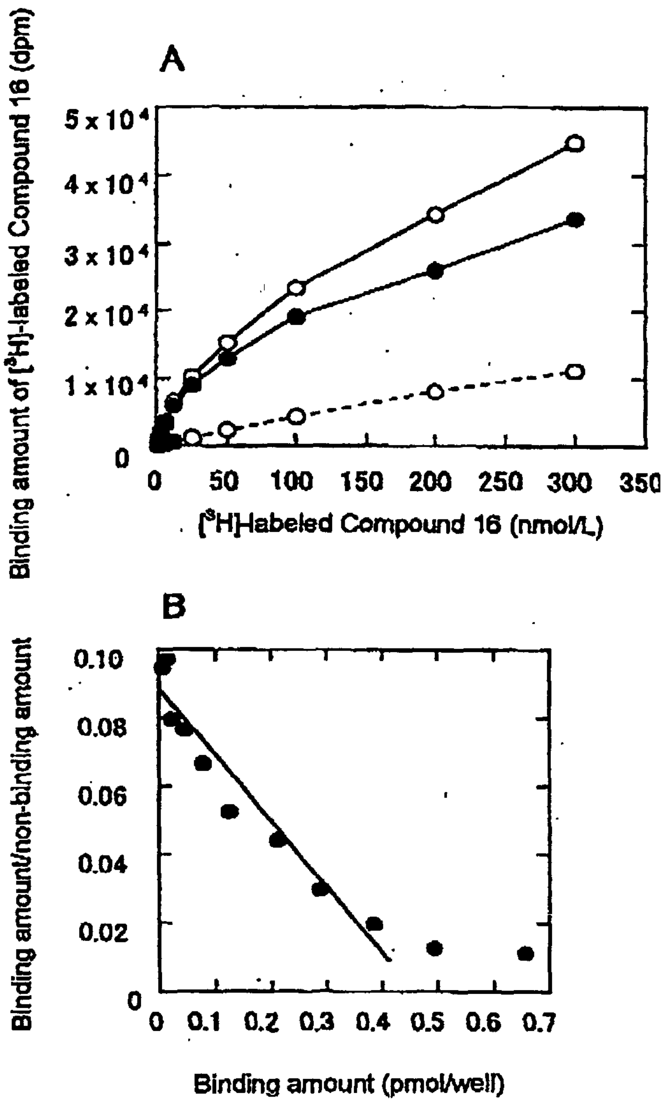
A
Binding amount of [³H]-labeled Compound 16 (dpm)
5 x 10⁴
4 x 10⁴
3 x 10⁴
2 x 10⁴
1 x 10⁴
0
0 50 100 150 200 250 300 350
[³H]-labeled Compound 16 (nmol/L)
B
Binding amount/non-binding amount
0.10
0.08
0.06
0.04
0.02
0
0 0.1 0.2 0.3 0.4 0.5 0.6 0.7
Binding amount (pmol/well)

FIG. 2

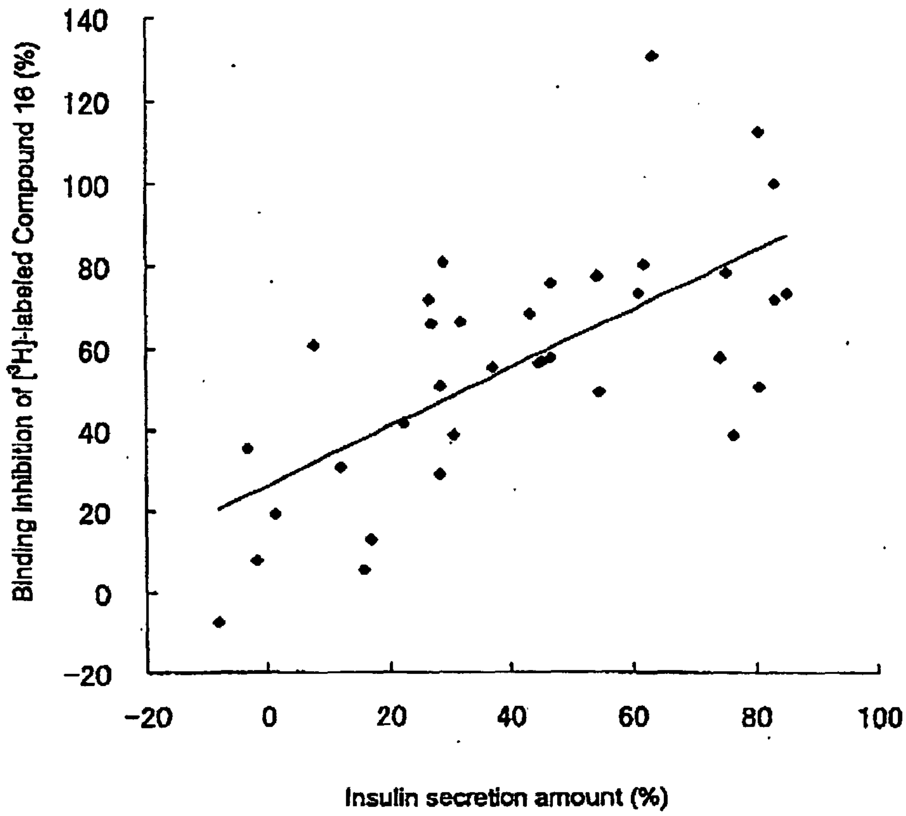
Binding Inhibition of [³H]-labeled Compound 16 (%)
140
120
100
80
60
40
20
0
−20
−20
0
20
40
60
80
100
Insulin secretion amount (%)

FIG. 3

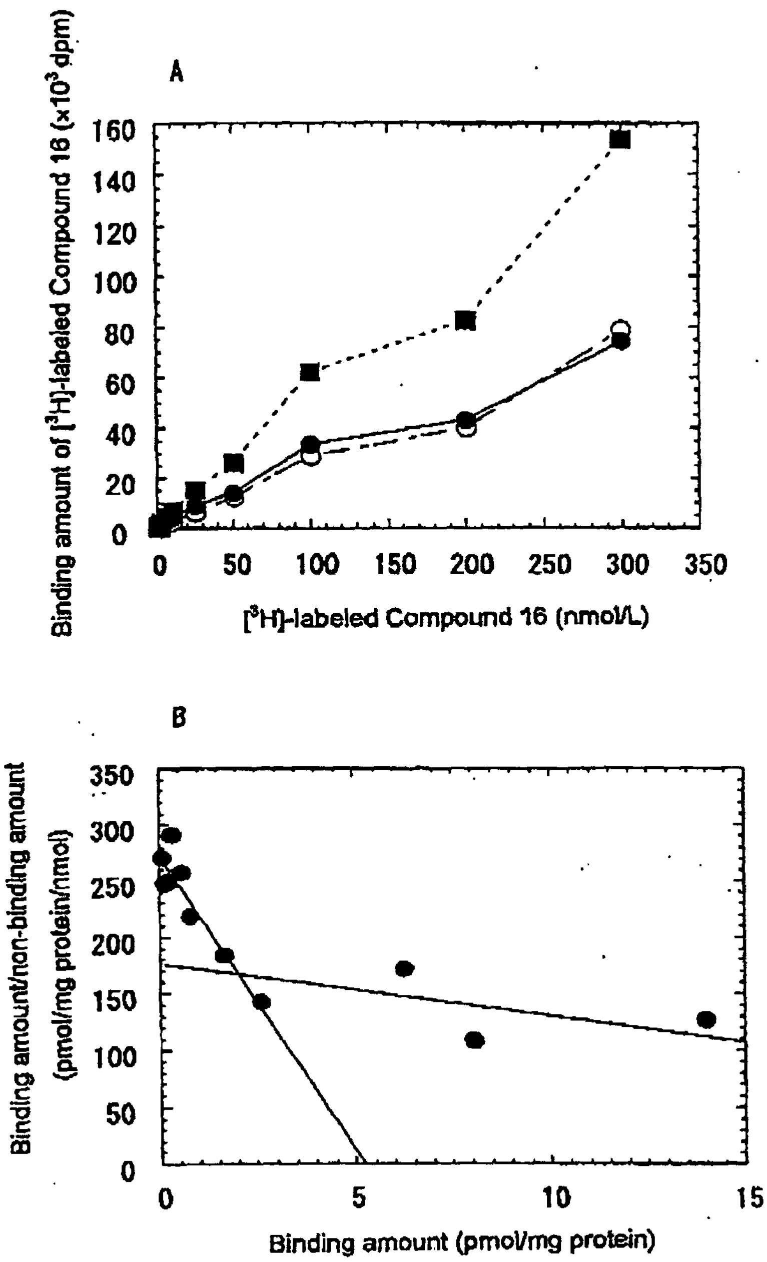
A
Binding amount of [3H]-labeled Compound 16 (×10³ dpm)
160
140
120
100
80
60
40
20
0
0 50 100 150 200 250 300 350
[3H]-labeled Compound 16 (nmol/L)
B
Binding amount/non-binding amount (pmol/mg protein/nmol)
350
300
250
200
150
100
50
0
0 5 10 15
Binding amount (pmol/mg protein)

FIG. 4

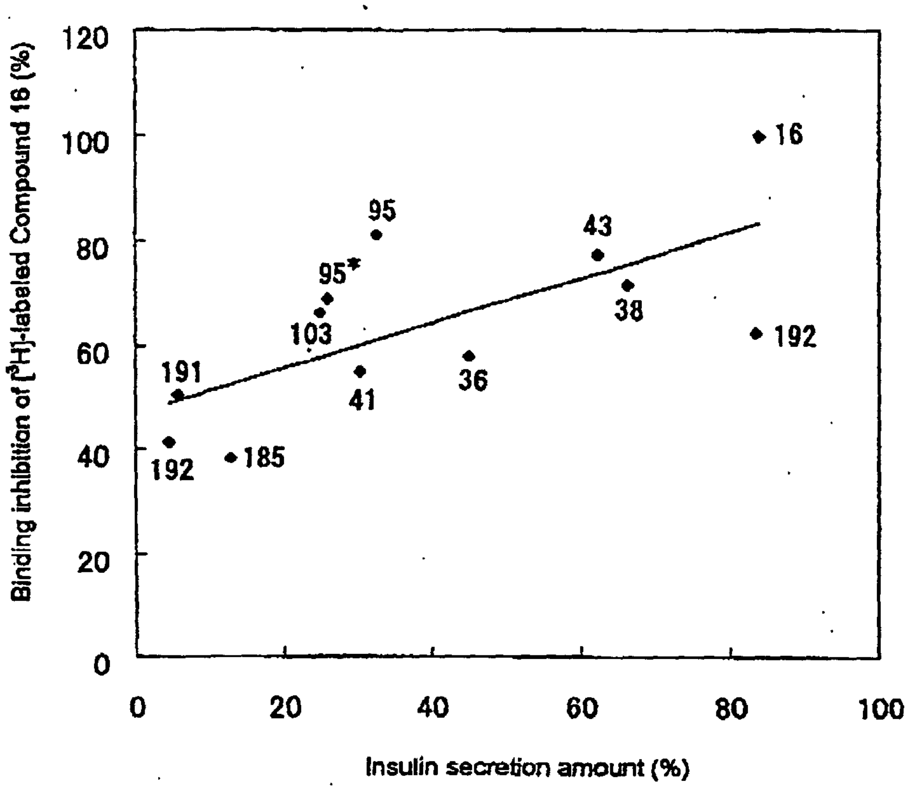
Binding inhibition of [3H]-labeled Compound 16 (%)
120
100
80
60
40
20
0
16
95
43
95*
38
103
192
191
36
41
192
185
0
20
40
60
80
100
Insulin secretion amount (%)

FIG. 5

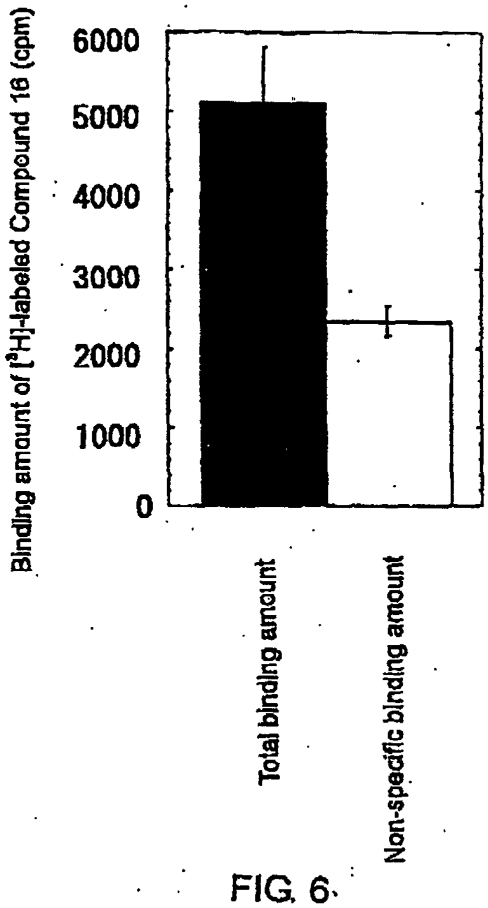
Binding amount of [3H]-labeled Compound 16 (cpm)
6000
5000
4000
3000
2000
1000
0
Total binding amount
Non-specific binding amount

FIG. 6

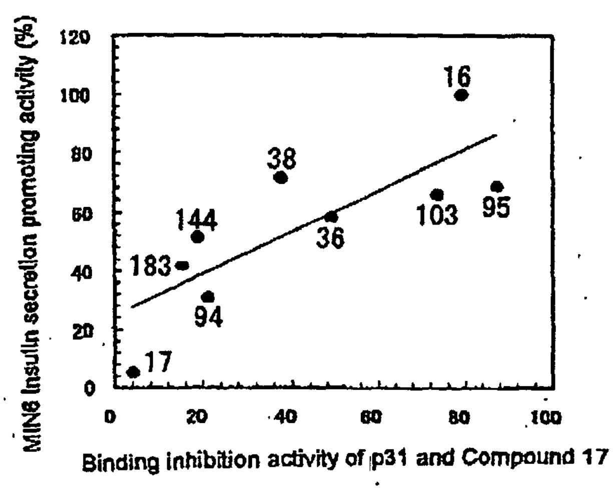
MIN6 Insulin secretion promoting activity (%)
120
100
80
60
40
20
0
16
38
144
103
95
36
183
94
17
0
20
40
60
80
100
Binding inhibition activity of p31 and Compound 17

FIG. 7

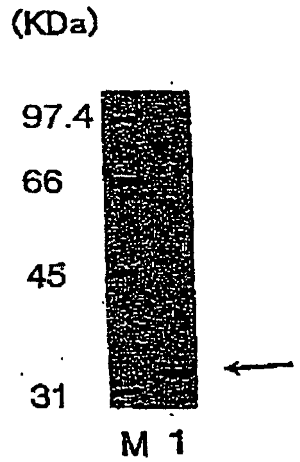


FIG. 8

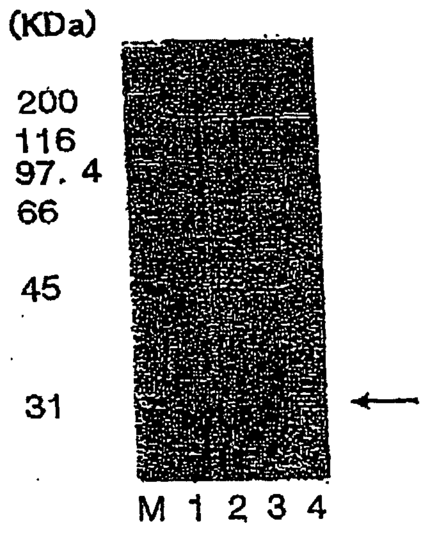

## INTERNATIONAL SEARCH REPORT

International application No.
PCT/JP03/06137

**A. CLASSIFICATION OF SUBJECT MATTER**

Int.Cl[7] C12Q1/04, C07K14/47, C12N9/02, A61K31/53, 31/5377, 31/55, 45/00, A61P3/10, 5/50, 43/00, C07D251/70, 403/14, 405/14, 487/14, G01N33/15, 33/50

According to International Patent Classification (IPC) or to both national classification and IPC

**B. FIELDS SEARCHED**

Minimum documentation searched (classification system followed by classification symbols)

Int.Cl[7] C12Q1/04, C07K14/47, C12N9/02, A61K31/53, 31/5377, 31/55, 45/00, A61P3/10, 5/50, 43/00, C07D251/70, 403/14, 405/14, 487/14, G01N33/15, 33/50

Documentation searched other than minimum documentation to the extent that such documents are included in the fields searched

Electronic data base consulted during the international search (name of data base and, where practicable, search terms used)

CA/BIOSIS/WPIDS/MEDLINE(STN), SwissProt/PIR/GeneSeq

**C. DOCUMENTS CONSIDERED TO BE RELEVANT**

| Category* | Citation of document, with indication, where appropriate, of the relevant passages | Relevant to claim No. |
|---|---|---|
| X,Y<br>A | WO 00/01388 A1 (Kyowa Hakko Kogyo Co., Ltd.), 13 January, 2000 (13.01.00), Claims; examples & US 6489331 B1 & EP 1092435 A1 & JP 2000-557834 A | 1,10,14-16<br>2-9,11-13 |
| X,Y<br>A | WO 01/47931 A1 (Kyowa Hakko Kogyo Co., Ltd.), 05 July, 2001 (05.07.01), Claims; examples & EP 1251130 A1 & JP 2001-549401 A | 1,14-16<br>2-13 |
| Y | WO 00/09464 A1 (PHYLOS INC.), 24 February, 2000 (24.02.00), Claims; examples & EP 1105360 A1 & JP 2002-522057 A | 14-16 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>13 August, 2003 (13.08.03) | Date of mailing of the international search report<br>26 August, 2003 (26.08.03) |
| Name and mailing address of the ISA/<br>Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/06137

C (Continuation). DOCUMENTS CONSIDERED TO BE RELEVANT

| Category* | Citation of document, with indication, where appropriate, of the relevant passages | Relevant to claim No. |
|---|---|---|
| X | J.A. Molello et al., "Pancreatic Beta Cell Vacuolation in Rats after Oral Administration of Hexamethylmelamine", Toxicology and Applied Pharmacology, 1984, Vol.72, pages 255 to 261 | 22-24 |
| A | WO 00/47219 A2 (ONTOGENY INC.), 17 August, 2000 (17.08.00), Claims; examples & EP 1189629 A2 & JP 2003-518914 A | 1-13,17 |
| A | WO 00/36102 A2 (GENENTECH INC.), 22 June, 2000 (22.06.00), Claims; examples & EP 1141285 A2 & JP 2003-521229 A | 2-13,17 |
| A | JP 54-14986 A (Taiho Pharmaceutical Co., Ltd.), 03 February, 1979 (03.02.79), Claims; examples (Family: none) | 22-24 |

Form PCT/ISA/210 (continuation of second sheet) (July 1998)

## INTERNATIONAL SEARCH REPORT

International application No.
PCT/JP03/06137

**Box I Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☒ Claims Nos.: 18-21
   because they relate to parts of the international application that do not comply with the prescribed requirements to such an extent that no meaningful international search can be carried out, specifically:
   With respect to "substance obtained by the searching method or pharmaceutically acceptable salt thereof" and "drug comprising the substance or pharmaceutically acceptable salt thereof as an active ingredient" claimed (continued to extra sheet)

3. ☐ Claims Nos.:
   because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1998)

**INTERNATIONAL SEARCH REPORT**

International application No.
PCT/JP03/06137

Continuation of Box No. I-2 of continuation of first sheet(1)

in the claims, what compound is comprehended and what compound is not comprehended apart from specifically obtained compounds are entirely unclear even if the contents of the description are taken into account, so that the inventions of the claims are extremely unclear. Therefore, no meaningful international search can be carried out on the claims.

Form PCT/ISA/210 (extra sheet) (July 1998)